# EUROPEAN PATENT APPLICATION

(11) **EP 2 848 702 A1**
(43) Date of publication of application: **18.03.2015**
(21) Application number: 14191479.6
(22) Date of filing: 06.02.2009
(51) Int. Cl.: C12Q 1/68

(54) **Disease markers and uses thereof**

(30) Priority: 08.02.2008 US 6963 P
(62) Divisional of application: 09709316.5
(71) Applicant: MedImmune, LLC, Gaithersburg, MD 20878 (US); Brigham and Women's Hospital, Inc., Boston, MA 02115 (US)
(72) Inventor: Yao, Yihong, Boyds, MD 20841 (US); Morehouse, Chris, Middletown, MD 21769 (US); Higgs, Brandon, Gaithersburg, MD 20878 (US); Jallal, Bahija, Potomac, MD 20854 (US); Greenberg, Steven, Newton, MA 02458 (US)
(74) Representative: Ttofi, Evangelia

(57) **Abstract**

A method of monitoring myositis disease progression of a patient receiving treatment with a therapeutic agent comprising (i) obtaining a first IFNα-inducible PD marker expression profile in a first sample from the patient; (ii) obtaining a second IFNα-inducible PD marker expression profile in a second sample from the patient after a therapeutic agent has been administered; and (iii) comparing the first and the second IFNα-inducible PD marker expression profiles, wherein the IFNα-inducible PD marker expression profile is based upon up-regulation of the expression of the following genes EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, and USP18; and wherein a variance in the first and the second IFNα-inducible PD marker expression profiles indicates a level of efficacy of the therapeutic agent, wherein the variance is a decrease in up-regulated expression of the gene.

SEQUENCE LISTING

<110>  MedImmune LLC
       Yao, Yihong
       Morehouse, Chris
       Higgs, Brandon
       Jallal, Bahija

<120>  DISEASE MARKERS AND USES THEREOF

<130>  IFNMY-400EP2

<150>  PCT/US2009/033407eq
<151>  2009-02-06

<150>  US61/006,963
<151>  2008-02-08

<160>  79

<170>  PatentIn version 3.3

<210>  1
<211>  22
<212>  RNA
<213>  Homo sapiens

<400>  1
uagcuuauca gacugauguu ga                                                22


<210>  2
<211>  23
<212>  RNA
<213>  Homo sapiens

<400>  2
uaggcagugu cauuagcuga uug                                               23


<210>  3
<211>  22
<212>  RNA
<213>  Homo sapiens

<400>  3
gaaguuguuc gugguggauu cg                                                22


<210>  4
<211>  21
<212>  RNA
<213>  Homo sapiens

<400>  4
uggaauguaa agaaguaugu a                                                 21


<210>  5
<211>  22
<212>  RNA
<213>  Homo sapiens

<400>  5
aacccguaga uccgaacuug ug                                                22

<210>  6
<211>  22
<212>  RNA
<213>  Homo sapiens

<400>  6
uacaguacug ugauaacuga ag                                                22

<210>  7
<211>  22
<212>  RNA
<213>  Homo sapiens

<400>  7
ucacagugaa ccggucucuu uc                                                22

<210>  8
<211>  22
<212>  RNA
<213>  Homo sapiens

<400>  8
uugguccccu ucaaccagcu gu                                                22

<210>  9
<211>  21
<212>  RNA
<213>  Homo sapiens

<400>  9
uugguccccu ucaaccagcu a                                                 21

<210>  10
<211>  22
<212>  RNA
<213>  Homo sapiens

<400>  10
aacauucaac cugucgguga gu                                                22

<210>  11
<211>  23
<212>  RNA
<213>  Homo sapiens

<400>  11
caaagaauuc uccuuuuggg cuu                                               23

<210>  12
<211>  24
<212>  RNA
<213>  Homo sapiens

<400>  12
aacuggcccu caaagucccg cuuu                                              24


<210>  13
<211>  22
<212>  RNA
<213>  Homo sapiens

<400>  13
aagcugccag uugaagaacu gu                                                22


<210>  14
<211>  21
<212>  RNA
<213>  Homo sapiens

<400>  14
aucacauugc cagggauuuc c                                                 21


<210>  15
<211>  21
<212>  RNA
<213>  Homo sapiens

<400>  15
aucacauugc cagggauuac c                                                 21


<210>  16
<211>  22
<212>  RNA
<213>  Homo sapiens

<400>  16
uucaaguaau ucaggauagg uu                                                22


<210>  17
<211>  21
<212>  RNA
<213>  Homo sapiens

<400>  17
uucacagugg cuaaguuccg c                                                 21


<210>  18
<211>  21
<212>  RNA
<213>  Homo sapiens

<400>  18
uucacagugg cuaaguucug c                                                 21


<210>  19
<211>  21
<212>  RNA
<213>  Homo sapiens

<400>  19
agggcccccc cucaauccug u                                                21

<210>  20
<211>  21
<212>  RNA
<213>  Homo sapiens

<400>  20
uagcaccauc ugaaaucggu u                                                21

<210>  21
<211>  20
<212>  RNA
<213>  Homo sapiens

<400>  21
uagcaccauu ugaaaucggu                                                  20

<210>  22
<211>  22
<212>  RNA
<213>  Homo sapiens

<400>  22
cuuucagucg gauguuugca gc                                               22

<210>  23
<211>  22
<212>  RNA
<213>  Homo sapiens

<400>  23
uguaaacauc cucgacugga ag                                               22

<210>  24
<211>  22
<212>  RNA
<213>  Homo sapiens

<400>  24
uguaaacauc cuacacucag cu                                               22

<210>  25
<211>  23
<212>  RNA
<213>  Homo sapiens

<400>  25
uguaaacauc cuacacucuc agc                                              23

<210>  26
<211>  22
<212>  RNA
<213>  Homo sapiens

<400>  26
uguaaacauc cccgacugga ag                                              22


<210>  27
<211>  22
<212>  RNA
<213>  Homo sapiens

<400>  27
cuuucagucg gauguuuaca gc                                              22


<210>  28
<211>  20
<212>  RNA
<213>  Homo sapiens

<400>  28
uguaaacauc cuugacugga                                                 20


<210>  29
<211>  23
<212>  RNA
<213>  Homo sapiens

<400>  29
   cgcauccccu agggcauugg ugu                                              23


<210>  30
<211>  22
<212>  RNA
<213>  Homo sapiens

<400>  30
cuggcccucu cugcccuucc gu                                              22


<210>  31
<211>  21
<212>  RNA
<213>  Homo sapiens

<400>  31
gccccugggc cuauccuaga a                                               21


<210>  32
<211>  23
<212>  RNA
<213>  Homo sapiens

<400>  32
uccgucucag uuacuuuaua gcc                                             23


<210>  33
<211>  22
<212>  RNA
<213>  Homo sapiens

<400>  33
uuaucagaau cuccaggggu ac                                                22


<210>  34
<211>  22
<212>  RNA
<213>  Homo sapiens

<400>  34
uaaugccccu aaaaauccuu au                                                22


<210>  35
<211>  22
<212>  RNA
<213>  Homo sapiens

<400>  35
cuccugacuc cagguccugu gu                                                22


<210>  36
<211>  22
<212>  RNA
<213>  Homo sapiens

<400>  36
cuggacuuag ggucagaagg cc                                                22


<210>  37
<211>  22
<212>  RNA
<213>  Homo sapiens

<400>  37
agcucggucu gaggccccuc ag                                                22


<210>  38
<211>  23
<212>  RNA
<213>  Homo sapiens

<400>  38
agugacauca cauauacggc agc                                               23


<210>  39
<211>  23
<212>  RNA
<213>  Homo sapiens

<400>  39
aguggggaac ccuuccauga gga                                               23


<210>  40
<211>  21
<212>  RNA
<213>  Homo sapiens

<400>  40
agacccuggu cugcacucua u                                                21

<210>  41
<211>  22
<212>  RNA
<213>  Homo sapiens

<400>  41
aaagcgcuuc ccuucagagu gu                                               22

<210>  42
<211>  25
<212>  RNA
<213>  Homo sapiens

<400>  42
cccaucuggg guggccugug acuuu                                            25

<210>  43
<211>  22
<212>  RNA
<213>  Homo sapiens

<400>  43
aacccguaga uccgaucuug ug                                               22

<210>  44
<211>  22
<212>  RNA
<213>  Homo sapiens

<400>  44
ucggauccgu cugagcuugg cu                                               22

<210>  45
<211>  22
<212>  RNA
<213>  Homo sapiens

<400>  45
uaacagucua cagccauggu cg                                               22

<210>  46
<211>  22
<212>  RNA
<213>  Homo sapiens

<400>  46
ugagaacuga auuccauggg uu                                               22

<210>  47
<211>  22
<212>  RNA
<213>  Homo sapiens

<400>  47
uuaaugcuaa ucgugauagg gg                                              22


<210>  48
<211>  22
<212>  RNA
<213>  Homo sapiens

<400>  48
uagcuuauca gacugauguu ga                                              22


<210>  49
<211>  21
<212>  RNA
<213>  Homo sapiens

<400>  49
acagcaggca cagacaggca g                                               21


<210>  50
<211>  23
<212>  RNA
<213>  Homo sapiens

<400>  50
agcuacauug ucugcugggu uuc                                             23


<210>  51
<211>  24
<212>  RNA
<213>  Homo sapiens

<400>  51
agcuacaucu ggcuacuggg ucuc                                            24


<210>  52
<211>  22
<212>  RNA
<213>  Homo sapiens

<400>  52
ugguuuaccg ucccacauac au                                              22


<210>  53
<211>  21
<212>  RNA
<213>  Homo sapiens

<400>  53
ggcaagaugc uggcauagcu g                                               21


<210>  54
<211>  22
<212>  RNA
<213>  Homo sapiens

<400>  54
gcacauuaca cggucgaccu cu                                                22

<210>  55
<211>  23
<212>  RNA
<213>  Homo sapiens

<400>  55
uggcaguguc uuagcugguu guu                                               23

<210>  56
<211>  21
<212>  RNA
<213>  Homo sapiens

<400>  56
aucauagagg aaaauccacg u                                                 21

<210>  57
<211>  21
<212>  RNA
<213>  Homo sapiens

<400>  57
uaguagaccg uauagcguac g                                                 21

<210>  58
<211>  23
<212>  RNA
<213>  Homo sapiens

<400>  58
ucuuggagua ggucauuggg ugg                                               23

<210>  59
<211>  22
<212>  RNA
<213>  Homo sapiens

<400>  59
aucaugaugg gcuccucggu gu                                                22

<210>  60
<211>  21
<212>  RNA
<213>  Homo sapiens

<400>  60
gugucuuuug cucugcaguc a                                                 21

<210>  61
<211>  22
<212>  RNA
<213>  Homo sapiens

<400>  61
ugagguagua gguuguauag uu                                              22


<210>  62
<211>  21
<212>  RNA
<213>  Homo sapiens

<400>  62
agagguagua gguugcauag u                                               21


<210>  63
<211>  22
<212>  RNA
<213>  Homo sapiens

<400>  63
ugagguagua gauuguauag uu                                              22


<210>  64
<211>  21
<212>  RNA
<213>  Homo sapiens

<400>  64
ugagguagua guuuguacag u                                               21


<210>  65
<211>  21
<212>  RNA
<213>  Homo sapiens

<400>  65
ucguaccgug aguaauaaug c                                               21


<210>  66
<211>  22
<212>  RNA
<213>  Homo sapiens

<400>  66
ugauauguuu gauauauuag gu                                              22


<210>  67
<211>  21
<212>  RNA
<213>  Homo sapiens

<400>  67
uagguaguuu ccuguuguug g                                               21


<210>  68
<211>  22
<212>  RNA
<213>  Homo sapiens

<400>  68
uucaccaccu ucuccaccca gc                                              22


<210>  69
<211>  23
<212>  RNA
<213>  Homo sapiens

<400>  69
ugugcaaauc uaugcaaaac uga                                             23


<210>  70
<211>  23
<212>  RNA
<213>  Homo sapiens

<400>  70
uaaagugcuu auagugcagg uag                                             23


<210>  71
<211>  21
<212>  RNA
<213>  Homo sapiens

<400>  71
uucaaguaau ccaggauagg c                                               21


<210>  72
<211>  21
<212>  RNA
<213>  Homo sapiens

<400>  72
uauugcacau uacuaaguug c                                               21


<210>  73
<211>  21
<212>  RNA
<213>  Homo sapiens

<400>  73
ugcugacucc uaguccaggg c                                               21


<210>  74
<211>  22
<212>  RNA
<213>  Homo sapiens

<400>  74
uuauaauaca accugauaag ug                                              22


<210>  75
<211>  22
<212>  RNA
<213>  Homo sapiens

<400>  75
uccuguacug agcugccccg ag                                                22

<210>  76
<211>  22
<212>  RNA
<213>  Homo sapiens

<400>  76
acucaaaacc cuucagugac uu                                                22

<210>  77
<211>  21
<212>  RNA
<213>  Homo sapiens

<400>  77
uauugcacuu gucccggccu g                                                 21

<210>  78
<211>  22
<212>  RNA
<213>  Homo sapiens

<400>  78
uucaacgggu auuuauugag ca                                                22

<210>  79
<211>  22
<212>  RNA
<213>  Homo sapiens

<400>  79
ugagguagua aguuguauug uu                                                22

## Description

### FIELD OF THE INVENTION

The present invention relates to miRNA markers and interferon (IFN) alpha/type I IFN-inducible pharmacodynamic (PD) markers and methods employing the same.

### BACKGROUND OF THE INVENTION

The present invention encompasses miRNA markers and IFNα/type I IFN-inducible PD markers. The present invention further encompasses methods that employ these markers in methods of, *e.g.,* treating patients and prognosing or monitoring disease.

### SUMMARY OF THE INVENTION

One embodiment of the invention encompasses a method of treating a patient having a type I IFN or IFNα-mediated disease or disorder. An agent that binds to and modulates type I IFN or IFNα activity is administered to the patient. The patient comprises a differentially regulated miRNA marker profile. The agent neutralizes the differentially regulated miRNA marker profile.

Another embodiment of the invention is a method of neutralizing a differentially regulated miRNA marker profile in a patient in need thereof. An agent that binds to and modulates type I IFN or IFNα activity is administered to the patient. The agent neutralizes the differentially regulated miRNA marker profile of the patient.

A further embodiment of the invention is a method of monitoring or prognosing an autoimmune or inflammatory disease progression of a patient. A first differentially regulated miRNA marker profile is obtained in a first sample from a patient.

In yet another embodiment of the invention is a method of monitoring autoimmune or inflammatory disease progression of a patient receiving treatment with a therapeutic agent. A first miRNA profile is obtained in a first sample from the patient. A therapeutic agent is administered. A second miRNA profile is obtained in a second sample from the patient. The first and the second miRNA profiles are compared. A variance in the first and the second miRNA profiles indicates a level of efficacy of the therapeutic agent.

In another embodiment of the invention is a method of treating a myositis patient. An agent that binds to and modulates type I IFN or IFNα activity is administered to the patient. The agent neutralizes the type I IFN or IFNα-inducible PD marker expression profile of the patient.

A further embodiment of the invention encompasses a method of monitoring or prognosing myositis disease progression of a patient. A first IFNα-inducible PD marker expression profile is obtained in a first sample from the patient.

In yet a further embodiment of the invention is a method of monitoring myositis disease progression of a patient receiving treatment with a therapeutic agent. A first IFNα-inducible PD marker expression profile is obtained in a first sample from the patient. A therapeutic agent is administered to the patient. A second IFNα-inducible PD marker expression profile is obtained from a second sample from the patient. The first and the second IFNα-inducible PD marker expression profiles are compared.

### BRIEF DESCRIPTION OF THE FIGURES

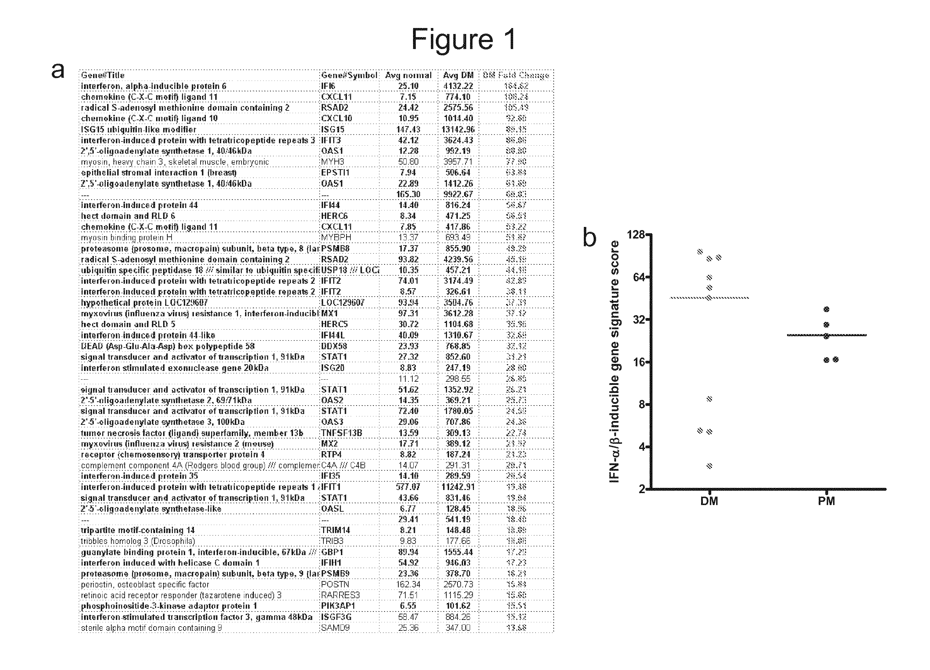

Figure 1a and 1b: The majority of the most upregulated genes in muscle specimens of DM and PM patients are IFN-α/β-inducible. (a) table showing genes up-regulated in DM patient muscle specimens; (b) IFN-α/β-inducible gene signature score in muscle of DM and PM patients.

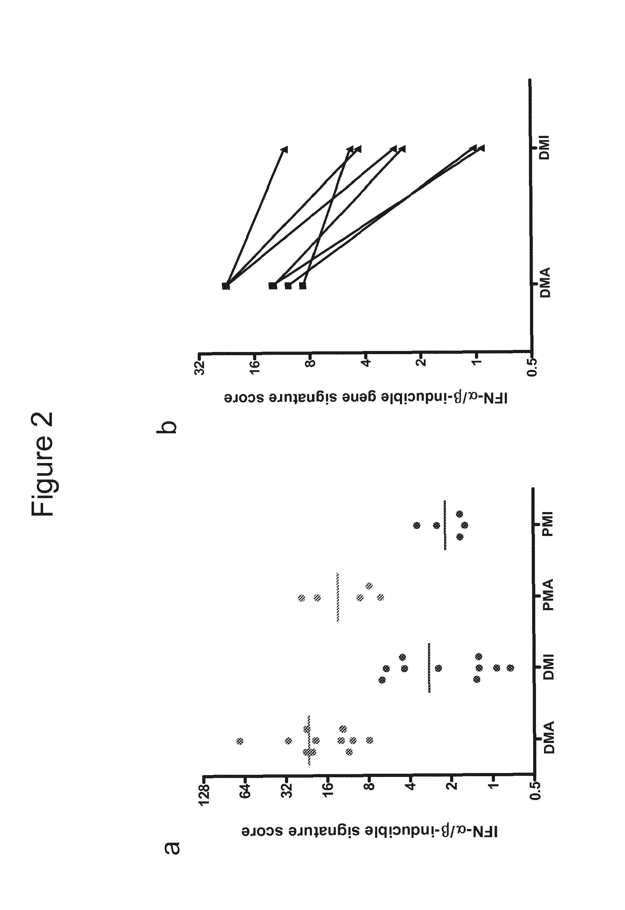

Figure 2a and 2b: IFN-α/β-inducible gene signature scores in DM and PM patients decreases as patients disease condition improves. (a) provides IFN-α/β-inducible gene signature scores of DM active patients (DMA), DM improving patients (DMI), PM active patients (PMA), and PM improving patients (PMI). (b) shows that IFN-α/β-inducible gene signature scores decrease in DM patients as they go from active disease to improvement.

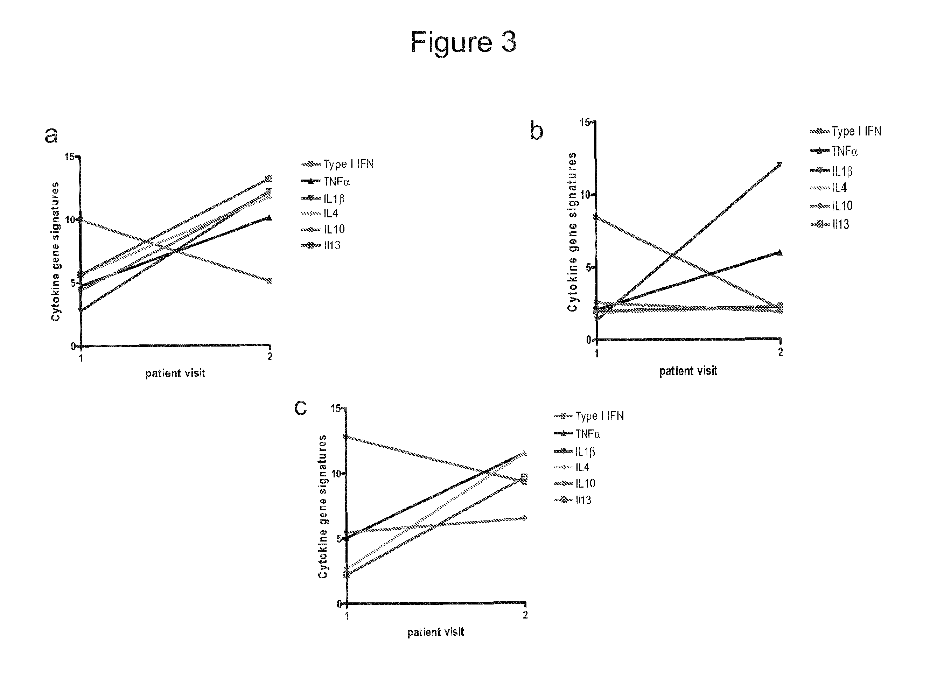

Figure 3a - 3c: IFN-α/β-inducible gene signature scores in DM patient whole blood samples correlate with clinical improvement, but not gene signature scores of other cytokines (TNFα, IL1β, IL4, IL10, and IL13) as shown for three, (a) - (c), patients.

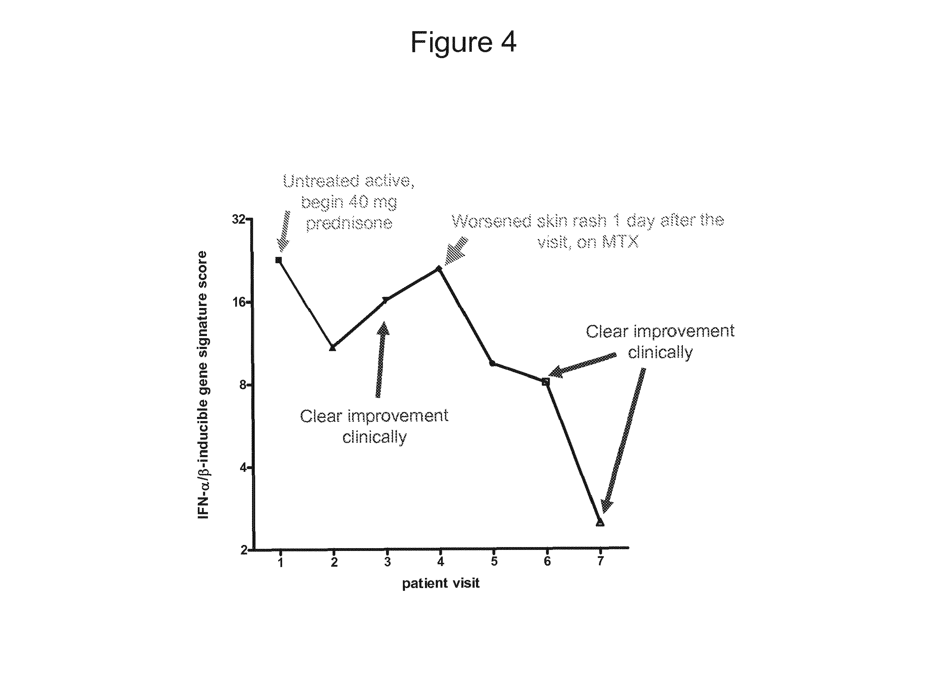

Figure 4: IFN-α/β-inducible gene signature scores of a DM patient predicted clinical improvement as well as relapse.

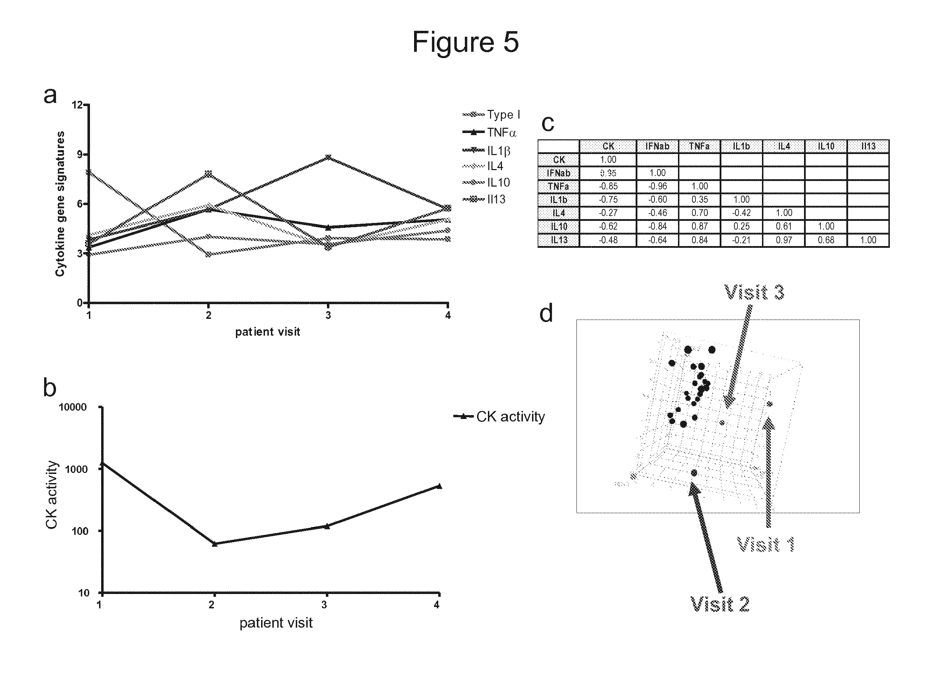

Figure 5a - 5d: Change in IFN-α/β-inducible gene signature score in a PM patient correlates with serum CK level. (a) shows the IFN-α/β-, TNFα-, IL1β-, IL4-, IL10-, IL13-inducible gene signature scores of the PM patient, (b) provides the serum CK activity of the patient, which follows the curve of the IFN-α/β-inducible gene signature; (c) provides the correlation between the various cytokine gene signature scores and serum CK level; (d) is a PCA plot of the PM patient's IFN-α/β-inducible gene signature score relative to that of healthy donors (black circles).

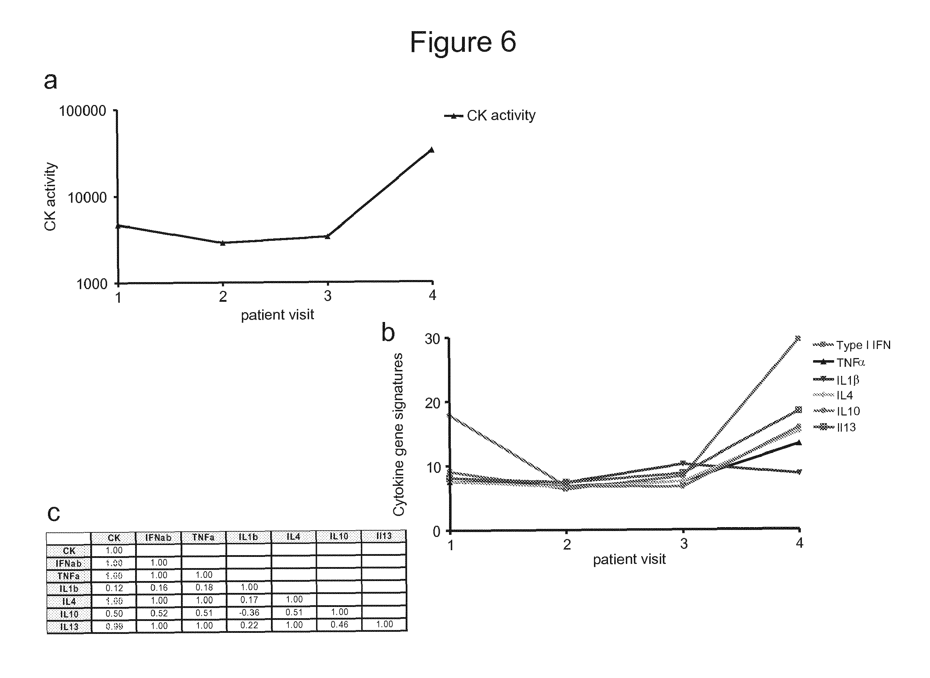

Figure 6: Change in IFN-α/β-inducible gene signature score in a DM patient correlates with serum CK level. (a) shows the patient's serum CK activity over four visits with a physician; (b) shows the IFN-α/β-, TNFα-, IL1β-, IL4-, IL10-, IL13-inducible gene signature scores of the patient over the same four visits; (c) provides the correlation between the various cytokine gene signature scores and serum CK level. The type I IFN gene signature (shown in (b)), as monitored by the neutralization of top 25 inducible genes in the whole blood of the patient, tracked the clinical activity. Visit 1, pretreatment; Visit 2, followed treatment with Rituximab and steroids (marginal improvement); Visit 3, patient remained stable; Visit 4, patient crashed (sharp increase in serum CK level).

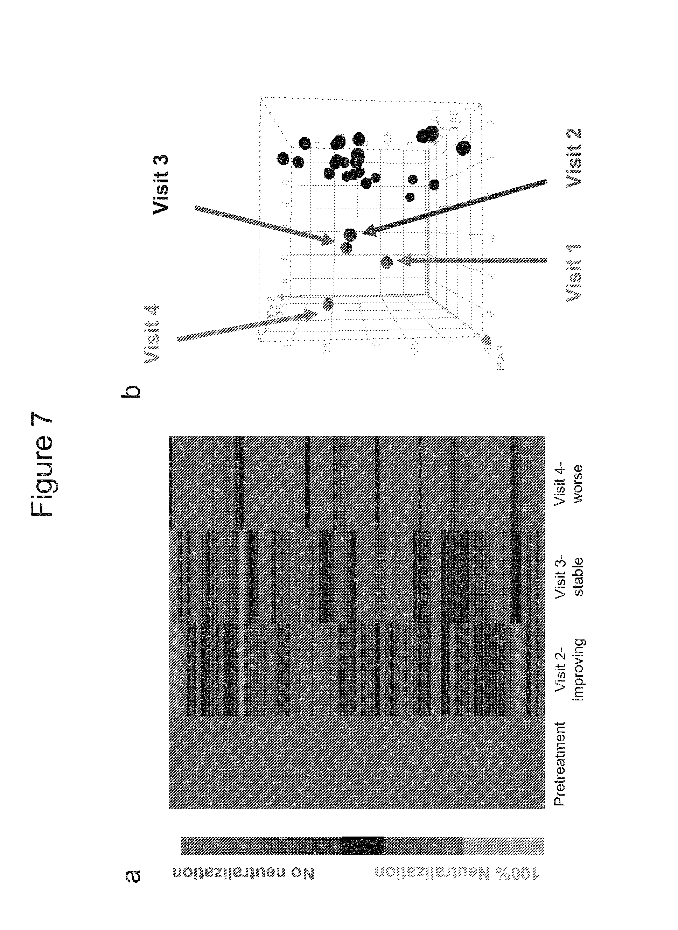

Figure 7a and 7b: The IFN-α/β-inducible gene signature of the DM patient discussed in figure 6 tracks disease progression/regression. (a) heatmap showing neutralization and de-neutralization of genes in the IFN-α/β-inducible signature as patient progresses/regresses; (b) PCA plot tracking the DM patient's IFN-α/β-inducible gene signature score relative to that of healthy donors (black circles).

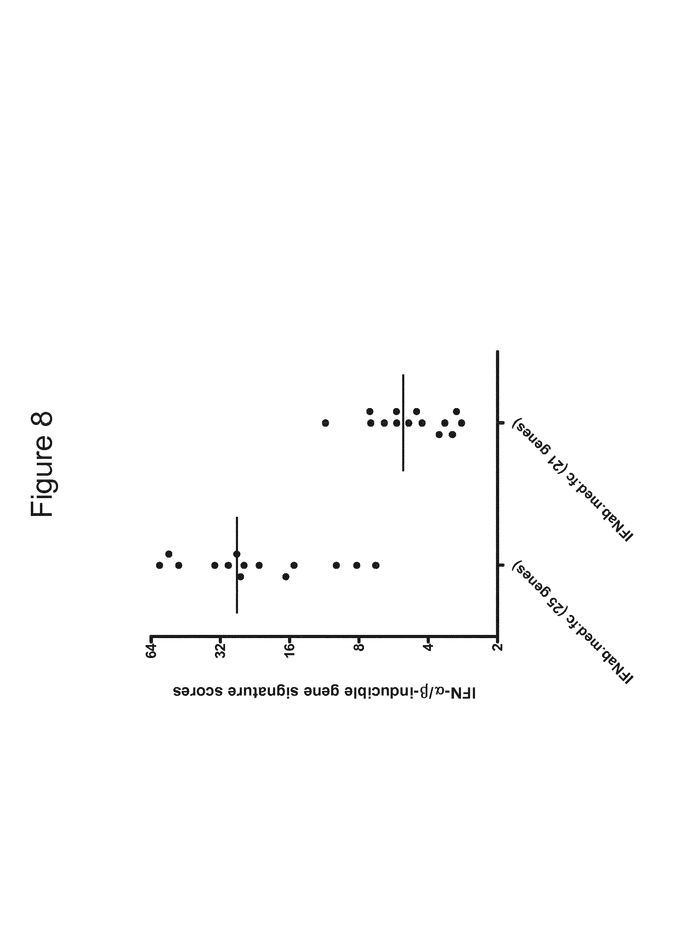

Figure 8: Scatter plot showing that muscle specimens of IBM patients exhibit IFN-α/β-inducible gene signature overexpression. Scatter plot is of IFN-α/β-gene signature scores for muscle specimens from 14 patients with IBM using two methods: a dynamic list of 25 genes and a static list of 21 genes. Each point represents a patient.

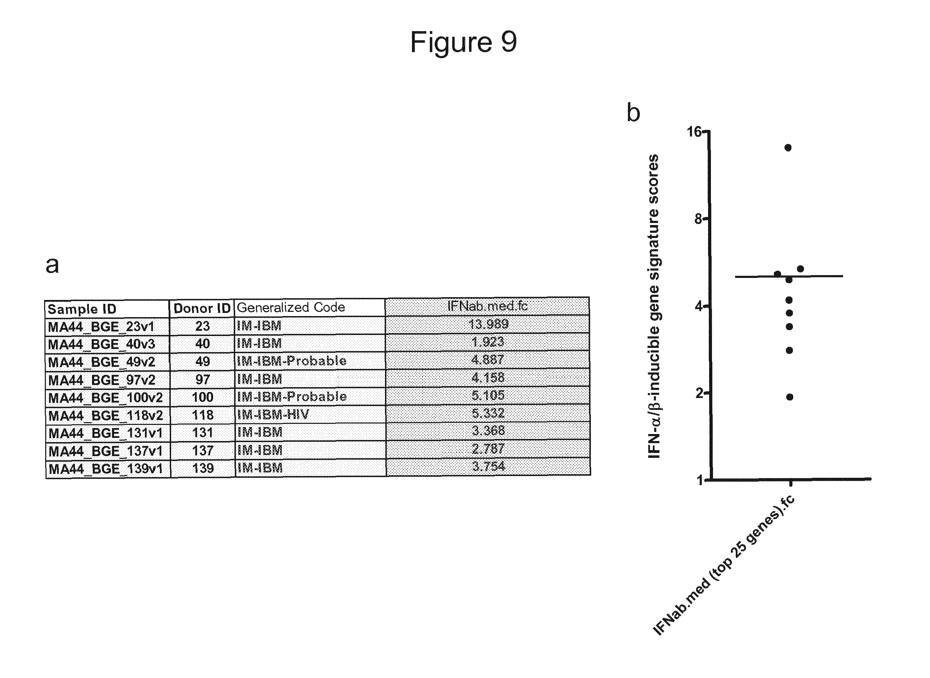

Figure 9a and 9b: IBM patient serum samples show distinct IFN-α/β-gene signature overexpression. (a) IFN-α/β-gene signature scores for whole blood from 9 IBM patients using two methods: a dynamic list of 25 genes and a static list of 21 genes; (b) scatter plot of IFN-α/β-gene signature scores for whole blood from 9 IBM patients using a dynamic list of 25 genes. Each point represents a patient.

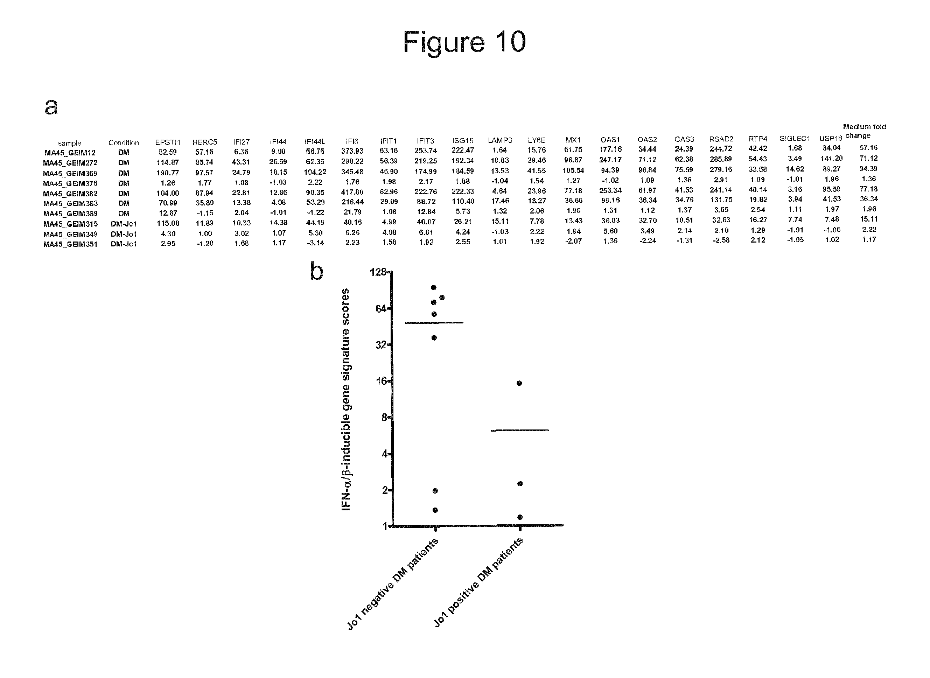

Figure 10a and 10b: Jo1 negative DM patients have higher IFN-α/β-gene signature scores than Jo1 positive DM patients. (a) overexpression of 19 IFN-α/β-inducible genes in DM Jo1 positive and DM Jo1 negative patients; (b) scatter plot of IFN-α/β-gene signature scores for muscle from 3 Jo1 positive and 7 Jo1 negative DM patients using the 19 genes. Each point represents a patient.
Figures 11-104: Probe list with annotation of genes in IL4 signature.
Figures 105-158: Probe list with annotation of genes in IL10 signature.
Figures 159-234: Probe list with annotation of genes in IL13 signature.
Figures 235-381: Probe list with annotation of 807 IFNα-inducible genes.

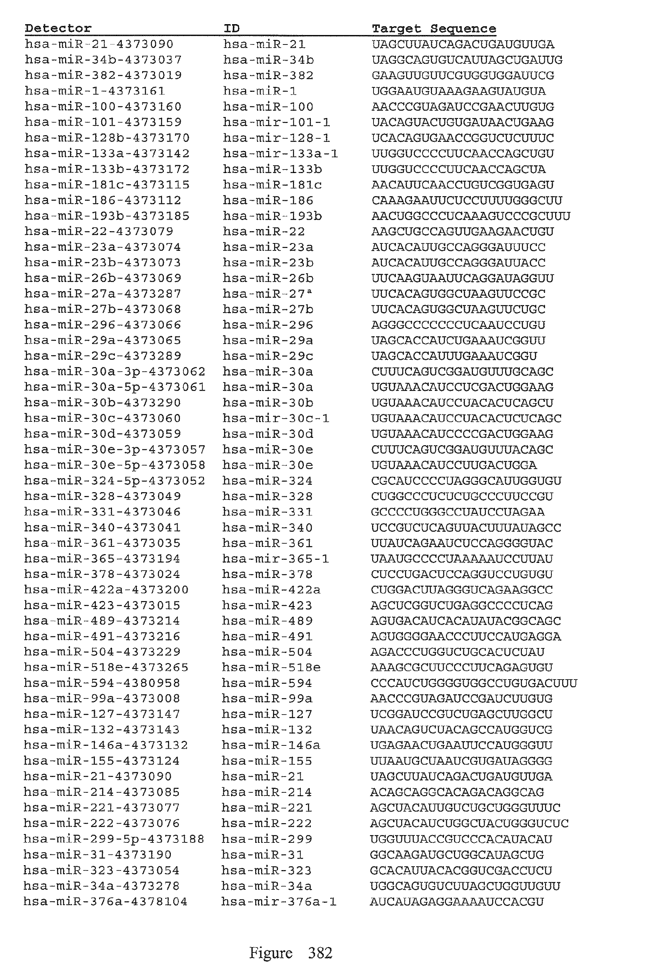

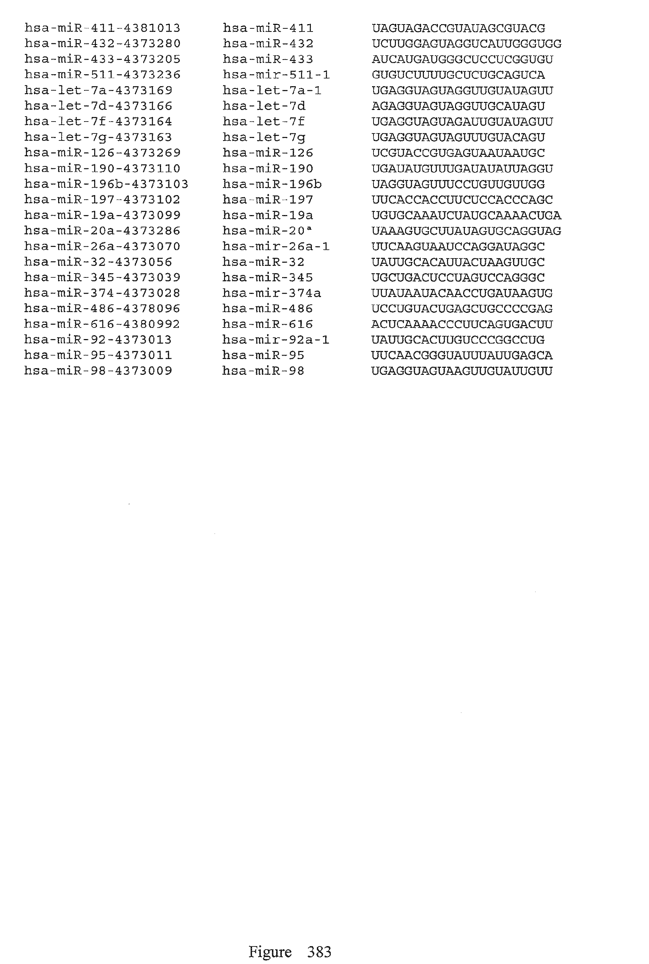

Figures 382-383: List with unique detector ids, miRNA ids, and sequences.

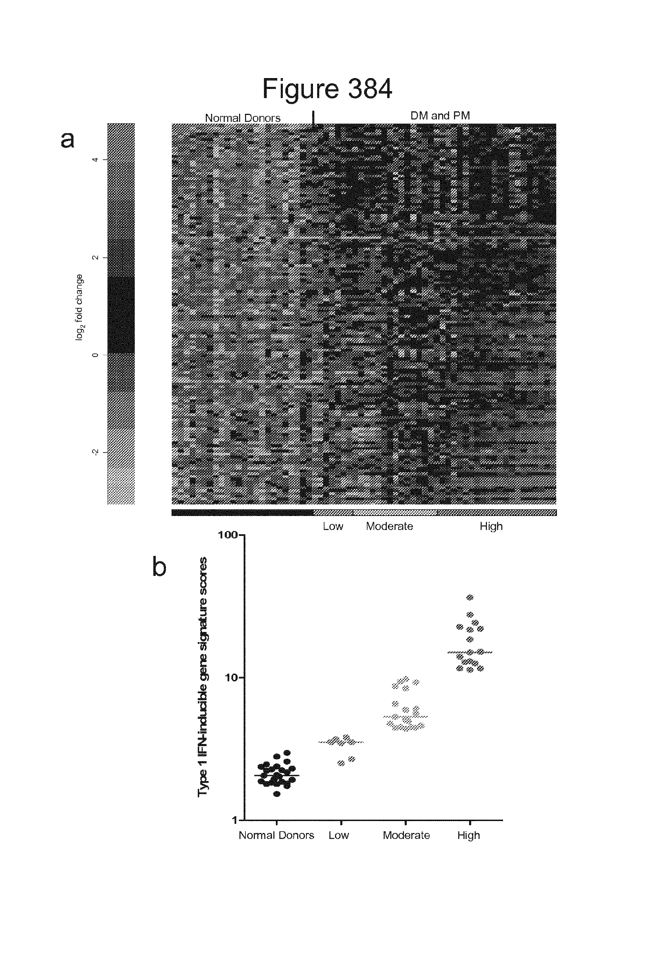

Figure 384a and 384b: The prevalence of type 1 IFN-inducible genes among those most overexpressed in DM and PM patients can be used to identify type 1 IFN-inducible gene signature score-positive patients. (a) Heatmap representing 24 normal healthy donors, 20 PM patients, and 22 DM patients and 136 probe sets identified as both type 1 IFN-inducible and significantly over-expressed (q-value < 0.05 and fold change > 2), comparing the 42 DM and PM patients to 24 normal healthy donors. The first, black, horizontal bar represents the normal healthy donors; the second horizontal bar, labeled low, represents patients with a weak type 1 IFN-inducible gene signature score (fold change < 4); the third horizontal bar, labeled moderate, represents patients with a moderate type 1 IFN-inducible gene signature score (4 ≤ fold change < 10), and the fourth horizontal bar, labeled high, represents patients with a high type 1 IFN-inducible gene signature score (i.e., signature score-positive). (b) Scatter plot of the type 1 IFN-inducible gene signature scores for the same subjects in (a) stratified by normal healthy donors, weak signature score, moderate signature score, and high signature score. IFN=interferon; DM=dermatomyositis; PM=polymyositis.

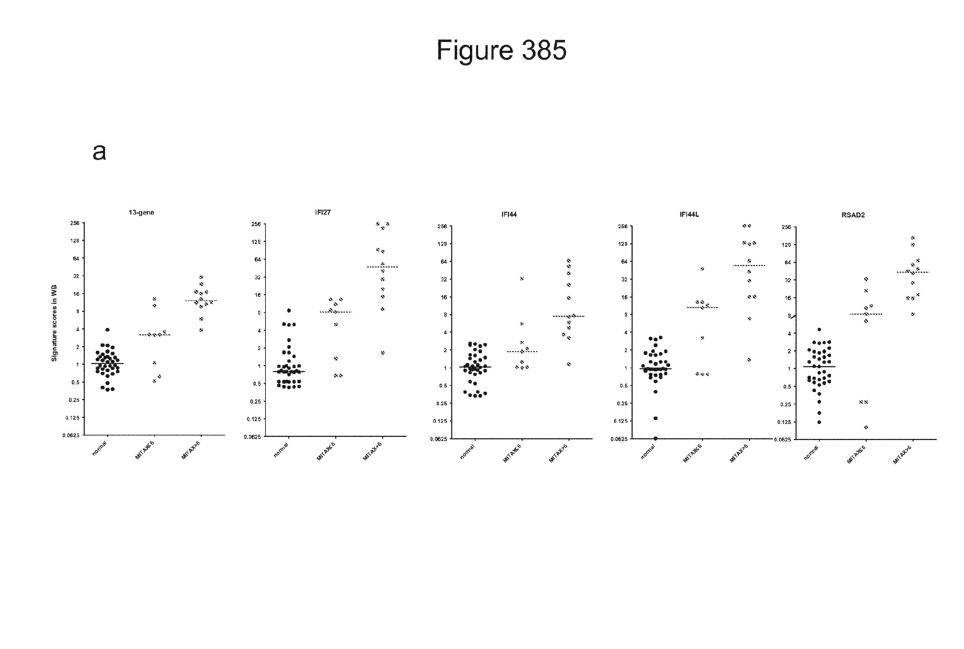

Figure 385a - 385c: Patient-specific longitudinal type 1 IFN-inducible transcript measurements have utility as biomarkers to measure DM and PM disease activity. (a) Individual gene composite scores and fold-change values using a 13-gene composite score and individual transcript measurements for IFI27, IFI44, IFI44L and RSAD2 for 21 patients at visit 1 and for 36 normal healthy donors. Patients are grouped by relatively low or moderate clinically measured disease activity (MITAX ≤ 6) and high disease activity (MITAX > 6). After multiple testing adjustment, there is no significant difference between normal healthy donors and patients with MITAX ≤ 6 for any of the 4 genes or the 13-gene composite scores. There are significant differences in comparing normal healthy donors vs. patients with MITAX > 6, and comparing patients with MITAX ≤ 6 vs. MITAX > 6, for all genes and gene composite scores (see Table 8). (b) Patient-specific analysis for 18 patients with some variation in MITAX-measured disease activity and (c) 3 patients with no change in MITAX across the study. Gene composite scores, fold-change values, and raw p-values are provided for each of the 18 patients at their visits, with lowest to highest MITAX values over the course of the study. Patients with MITAX variation showed highly significant concordant gene expression changes for all genes/gene composite scores except IFI44 after adjustment; patients without MITAX variation showed essentially no gene expression changes, though the sample size was too small to carry statistical significance. All gene composite scores and fold change values were calculated relative to the median of the 36 normal healthy donors.

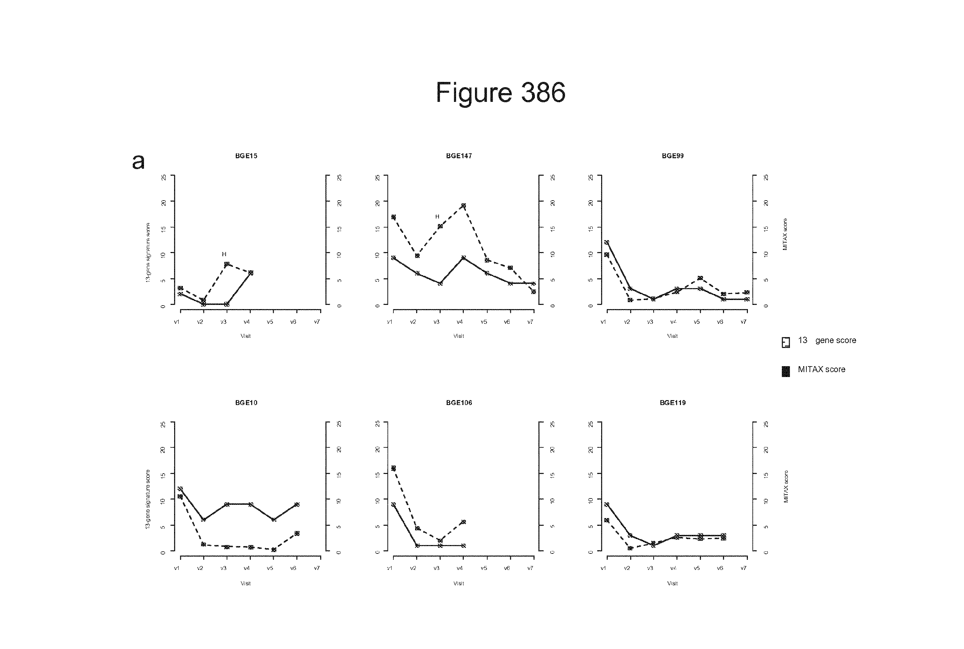

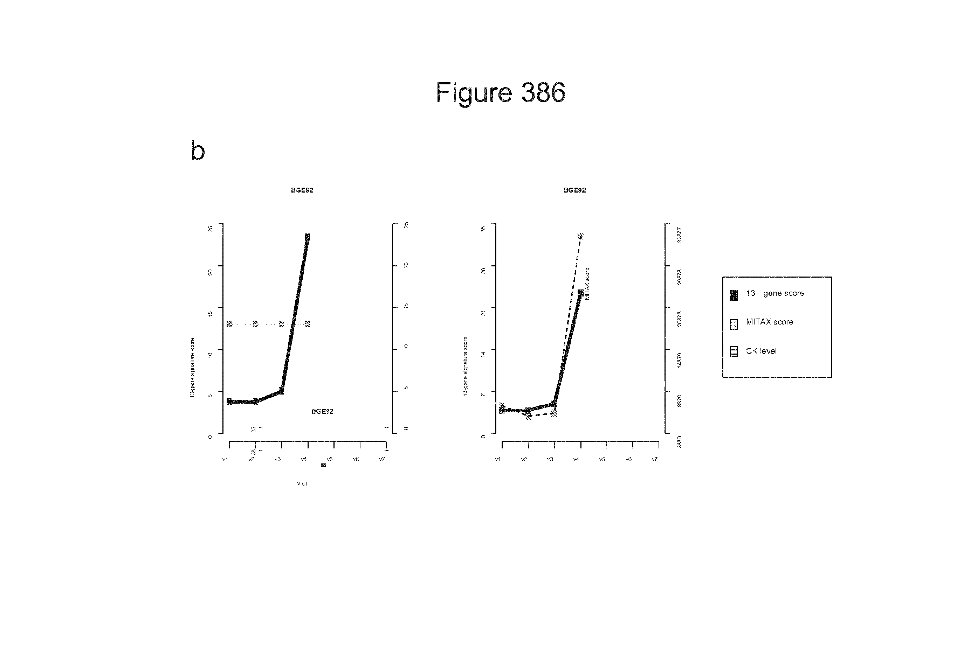

Figure 386a and 386b. DM and PM patients demonstrate a correlation between MITAX score and type 1 IFN 13-gene composite score from peripheral blood. (a) Comparisons between MITAX score and the 13-gene composite score for 6 patients (BGE15, BGE99, BGE10, BGE106, BGE119, and BGE147) with at least 4 total visits. The solid black line and rightmost y-axis represent MITAX scores, while the dashed black line and leftmost y-axis represent 13-gene composite scores. The 'H' designation in the plots for BGE15 and BGE147 represent the herald visits. (b) An additional correlation is seen for patient BGE92 between the 13-gene composite score and CK levels. The dashed black line and rightmost y-axis in the leftmost plot represents the CK levels for this patient. DM=dermatomyositis; PM=polymyositis; CK=creatine kinase.

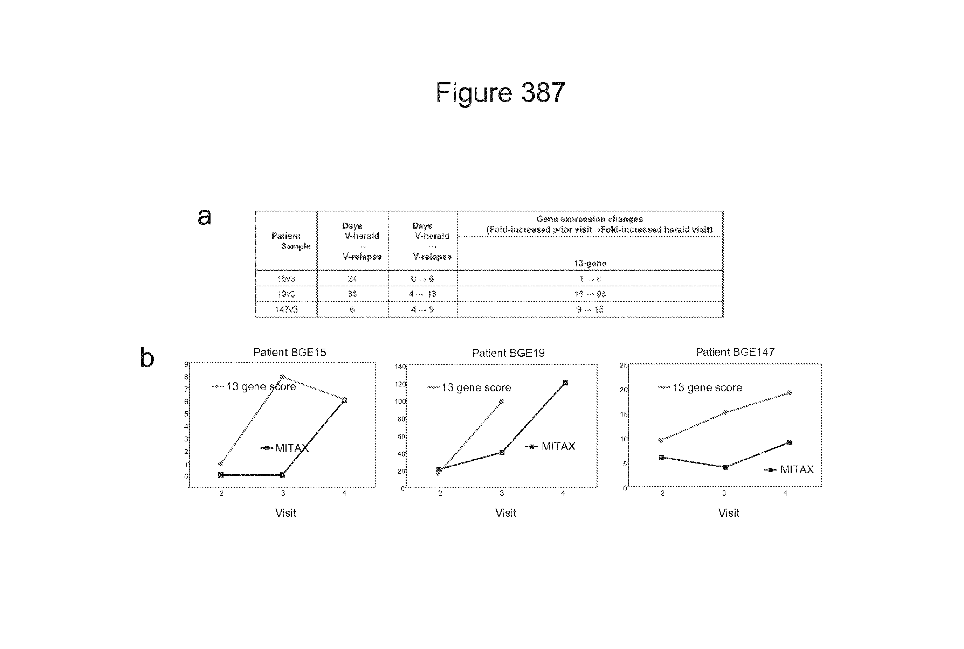

Figure 387a and 387b: Strong elevation of type 1 IFN-inducible gene expression in WB heralding disease relapse in several patients with DM or PM. Three visits occurred in which disease was believed to be clinically stable or improving, yet the patient developed a clinical relapse 6-35 days later. The significant IFN-inducible gene expression increases in each of these 3 visits "heralded" that relapses would soon occur. (a) Tabular listing of expression changes for the 13-gene composite score. Columns show: patient samples; days between herald (V-herald) and relapse (V-relapse) visits; MITAX score changes between V-herald and V-relapse; fold-increases in gene expression from the prior visit to V-herald. (b) Graphical representation of the 13-gene composite scores. Each of the middle visits (#3) is the herald visit; MITAX score was stable or improved compared to the prior visit (#2), but the 13-gene composite scores had significantly increased, possibly heralding the relapses confirmed by increased MITAX scores at the next visit (#4). IFN=interferon; DM=dermatomyositis; PM=polymyositis.

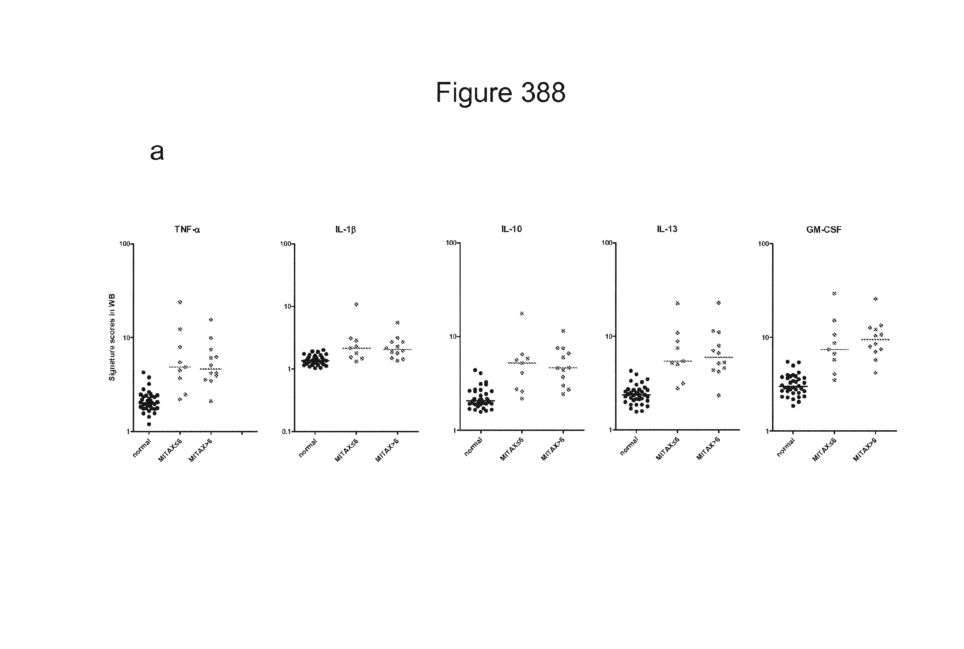

Figure 388a - 388c: Other cytokine-inducible signature scores do not differ between moderate and severe patients with DM or PM and are not correlated with disease activity. (a) Cytokine-inducible gene signatures for TNF-α, IL-1β, IL-10, IL-13, and GM-CSF for 21 patients at visit 1 and for 36 normal healthy donors. Patients are grouped by relatively low or moderate clinically measured disease activity (MITAX ≤ 6) and high disease activity (MITAX > 6). After multiple testing adjustment, there is a significant difference between normal healthy donors and patients with a MITAX ≤ 6 for all 5 cytokine-inducible gene signatures. There is also a significant difference between normal healthy donors and patients with a MITAX > 6 for all 5 cytokine-inducible gene signatures. However, there is no significant difference between patients with a MITAX ≤ 6 vs. MITAX > 6 for any of the 5 cytokine-inducible gene signatures (see Table 8). (b) Patient-specific analysis for 18 patients with some variation in MITAX-measured disease activity and (c) 3 patients with no change in MITAX activity across the study. Cytokine-inducible gene signatures and raw p-values are provided for each of the 18 patients at their visits with lowest to highest MITAX values over the course of the study. Patients with MITAX variation showed no significant concordant gene expression changes for any cytokine-inducible gene signature; patients without MITAX variation showed essentially no gene expression changes either, although the sample size was too small to calculate statistical significance. All cytokine-inducible gene signatures are calculated relative to the median of the 36 normal healthy donors. DM=dermatomyositis; PM=polymyositis.

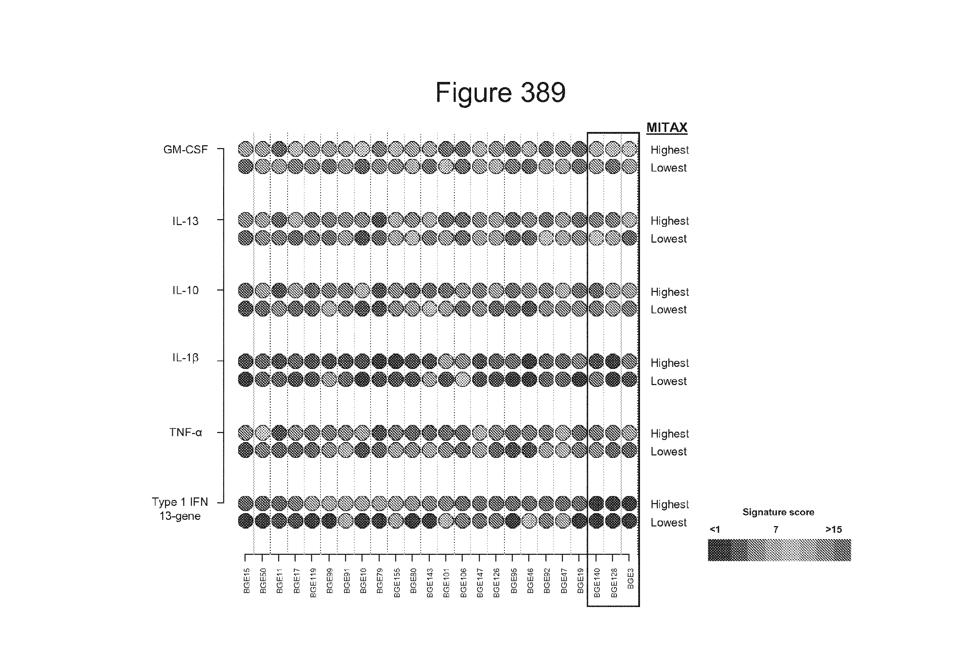

Figure 389: Comparison of different cytokine-inducible gene signatures for 24 DM or PM patients at the visits with the highest (upper panel) and lowest (lower panel) MITAX scores. Each row indicates the overexpression of a different cytokine-inducible gene signature. The 3 patients outlined in the box are type 1 IFN-inducible gene signature score-negative. Elevated cytokine-induced gene signature levels for each patient are represented by colors approaching red, while colors approaching blue represent low cytokine-induced gene signature values. IFN=interferon; DM=dermatomyositis; PM=polymyositis.

### DETAILED DESCRIPTION

The invention encompasses methods of identifying, diagnosing, treating, and monitoring disease progression in patients. Patients include any animal having a type I IFN or an IFNα-inducible disease, disorder, or condition; or any animal having an inflammatory or autoimmune disease, disorder, or condition. The patient may have the disease, disorder, or condition as a result of experimental research, e.g., it may be an experimental model developed for the disease, disorder, or condition. Alternatively, the patient may have the disease, disorder, or condition in the absence of experimental manipulation. Patients include humans, mice, rats, horses, pigs, cats, dogs, and any animal used for research.

A type I IFN or an IFNα-inducible disease, disorder, or condition is any that exhibits a type I IFN or an IFNα PD marker expression profile or gene signature. A PD marker expression profile and a gene signature will be understood to be equivalent. These diseases, disorders, or conditions include those with an autoimmune component such as systemic lupus erythematosus, insulin dependent diabetes mellitus, inflammatory bowel disease (including Crohn's disease, ulcerative colitis, and Celiac's disease), multiple sclerosis, psoriasis, autoimmune thyroiditis, rheumatoid arthritis, glomerulonephritis, idiopathic inflammatory myositis such as inclusion body myositis or dermatomyositis or polymyositis or nonspecific myositis, or necrotizing myopathy, Sjogren's syndrome, vasculitis, and sarcoidosis. Other diseases, disorders, or conditions include graft versus host disease and transplant rejection.

If the patient is a myositis patient, the patient may have inclusion body myositis or dermatomyositis or polymyositis. The myositis patient may have a high or a low Myositis Intention to Treat scale (MITAX) score. One of skill in the art would readily be able to determine the MITAX score of a myositis patient. See, for example, Walsh RJ, Pinkus JL, et al. Type I interferon-inducible gene expression in blood is present and reflects disease activity in dermatomyositis and polymyositis. Arthritis Rheum. 2007; 56(11):3784-92, incorporated by reference. A high MITAX score may be about greater than 6. A low MITAX score may be about less than or equal to 6. The myositis patient may alternatively or further have a moderate or strong type I IFN- or IFNα-inducible gene signature score. A type 1 IFN- or IFNα-inducible gene signature score may be weak if assigned a score of about less than 4, moderate if assigned a score around or about greater than 4 but less than 10, and high if assigned a score of about 10 or greater than 10. Those of skill in the art would readily understand and be able to determine type I IFN or IFNα-inducible gene signature score. See, for example, Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312, incorporated by reference.

An autoimmune disease, disorder, or condition may be any disease, disorder, or condition in which the immune system triggers an immune response when there are no foreign substances to fight and the body's normally protective immune system causes damage to its own tissues by mistakenly attacking self. These diseases, disorders, or conditions include multiple sclerosis, Crohn's disease, rheumatoid arthritis, alopecia greata, ankylosing spondylitis, antiphospholipid syndrome, autoimmune Addison's disease, autoimmune diseases of the adrenal gland, allergic encephalomyelitis, autoimmune hemolytic anemia, autoimmune hepatitis, autoimmune inflammatory eye disease, autoimmune neonatal thrombocytopenia, autoimmune neutropenia, autoimmune oophoritis and orchitis, autoimmune thrombocytopenia, autoimmune thyroiditis, Behcet's disease, bullous pemphigoid, cardiomyopathy, cardiotomy syndrome, celiac spruedermatitis, chronic active hepatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, dense deposit disease, discoid lupus, essential mixed cryoglobulinemia, fibromyalgiafibromyositis, glomerulonephritis (e.g., IgA nephrophathy), gluten-sensitive enteropathy, Goodpasture 's syndrome, Graves' disease, Guillain-Barre, hyperthyroidism (*i.e*., Hashimoto's thyroiditis), idiopathic pulmonary fibrosis, idiopathic Addison's disease, idiopathic thrombocytopenia purpura (ITP), IgA neuropathy, juvenile arthritis, lichen planus, lupus erthematosus, Meniere's disease, mixed connective tissue disease, multiple sclerosis, Myasthenia Gravis, myocarditis, type 1 or immune-mediated diabetes mellitus, myasthenia gravis, myocarditis, neuritis, other endocrine gland failure, pemphigus vulgaris, pernicious anemia, polyarteritis nodosa, polychrondritis, Polyendocrinopathies, polyglandular syndromes, polymyalgia rheumatica, polymyositis and dermatomyositis, post-MI, primary agammaglobulinemia, primary biliary cirrhosis, psoriasis, psoriatic arthritis, Raynauld's phenomenon, relapsing polychondritis, Reiter's syndrome, rheumatic heart disease, rheumatoid arthritis, sarcoidosis, scleroderma, Sjogren's syndrome, stiffman syndrome, systemic lupus erythematosus, lupus erythematosus, takayasu arteritis, temporal arteristis/giant cell arteritis, ulcerative colitis, urticaria, uveitis, Uveitis Opthalmia, vasculitides such as dermatitis herpetiformis vasculitis, vitiligo, and Wegener's granulomatosis.

An inflammatory disorder, disease, or condition may include asthma, allergic disorders, inflammatory disorders characterized by type-2 mediated inflammation, pulmonary fibrosis, chronic obstructive pulmonary disease (COPD), encephilitis, inflammatory bowel disease, septic shock, undifferentiated spondyloarthropathy, undifferentiated arthropathy, arthritis, inflammatory osteolysis, and chronic inflammation resulting from chronic viral or bacteria infections.

The patient may comprise a differentially regulated miRNA profile. A differentially regulated miRNA profile may be one in which a tissue sample of the patient exhibits increased expression of one or more miRNAs relative to a control tissue sample of the patient or relative to a healthy control individual. A differentially regulated miRNA profile may be one in which a tissue sample of the patient exhibits decreased expression of one or more miRNAs relative to a control sample of the patient or relative to a healthy control individual. A differentially regulated miRNA profile may be one in which a tissue sample of the patient exhibits both increased expression of one or more miRNAs relative to a control sample and decreased expression of one or more miRNAs relative to a control sample. The number of miRNAs exhibiting increased expression may be 1, at least 1, 2, at least 2, 3, at least 3, 4, at least 4, 4, at least 5, at least 6, at least 7, at least 8, at least 9, at least 10, at least 15, at least 20, at least 25, at least 30, or at least 50. The number of miRNAs exhibiting decreased expression may be 1, at least 1, 2, at least 2, 3, at least 3, 4, at least 4, 4, at least 5, at least 6, at least 7, at least 8, at least 9, at least 10, at least 15, at least 20, at least 25, at least 30, or at least 50. The differential increase or decrease in expression may be approximately 10% - 500% of the control sample, approximately 10% - 400% of the control sample, approximately 10% - 300% of the control sample, approximately 10% - 250% of the control sample, approximately 10% - 200% of the control sample, approximately 10% - 150% of the control sample, approximately 10% - 100% of the control sample, approximately 10% - 50% of the control sample, approximately 100% - 500% of the control sample, approximately 200% - 500% of the control sample, approximately 300% - 500% of the control sample, approximately 400% - 500% of the control sample, approximately 50% - 100% of the control sample, approximately 100% - 200% of the control sample, approximately 100% - 400% of the control sample, approximately 200% - 400% of the control sample, approximately 10% - 50% of the control sample, approximately 20% - 100% of the control sample, approximately 25% - 75% of the control sample, or approximately 50% - 100% of the control sample. It may be 10, 20, 25, 30, 40, 50, 75, 100, 125, 150, 175, 200, 250, 300, 400, or 500 percent of the control sample.

The any 1, at least 1, 2, at least 2, 3, at least 3, 4, at least 4, 4, at least 5, at least 6, at least 7, at least 8, at least 9, at least 10, at least 15, at least 20, at least 25, at least 30, or at least 50 miRNAs differentially expressed may include any of the miRNAs discussed in Tables 2-5 or figures 382-383. The miRNA may be detected by the detector hsa-miR-34b-4373037 or hsa-miR-1-4373161.

Administration of an agent which binds to and modulates type I IFN or IFNα activity may be administration of a small molecule or a biological agent. If the therapeutic agent is a small molecule it may be synthesized or identified and isolated from a natural source.

If the therapeutic agent is a biological agent, it may be an antibody specific for any subtype(s) of type I IFN or IFNα. For instance, the antibody may be specific for any one of IFNα1, IFNα2, IFNα4, IFNα5, IFNα6, IFNα7, IFNα8, IFNα10, IFNα14, IFNα17, IFNα21, IFNβ, or IFNω. Alternatively, the antibody may be specific for any two, any three, any four, any five, any six, any seven, any eight, any nine, any ten, any eleven, any twelve type I IFN of IFNα subtypes. If the antibody is specific for more than one type I IFN subtype, the antibody may be specific for IFNα1, IFNα2, IFNα4, IFNα5, IFNα8, IFNα10, and IFNα21; or it may be specific for IFNα1, IFNα2, IFNα4, IFNα5, IFNα8, and IFNα10; or it may be specific for IFNα1, IFNα2, IFNα4, IFNα5, IFNα8, and IFNα21; or it may be specific for IFNα1, IFNα2, IFNα4, IFNα5, IFNα10, and IFNα21. Antibodies specific for type I IFN or IFNα include MEDI-545, any biologic or antibody other than MEDI-545, antibodies described in U.S. patent applications 11/009,410 filed December 10, 2004 and 11/157,494 filed June 20, 2005, 9F3 and other antibodies described in U.S. Patent No. 7,087,726 (Example 1 and Example 2, those disclosed in Table 3 and Table 4, and/or those disclosed in the table entitled "Deposit of Material" on lines 25-54, column 56), NK-2 and YOK5/19 (WO 84/03105), LO-22 (U.S. Patent 4,902,618), 144 BS (U.S. Patent 4,885,166), and EBI-1, EBI-2, and EBI-3 (EP 119476). A therapeutic agent that modulates IFNα activity may neutralize IFNα activity. One of skill in the art is well aware of preparation and formulation of such biological agents and methods of their administration.

The antibody may be a synthetic antibody, a monoclonal antibody, polyclonal antibodies, a recombinantly produced antibody, an intrabody, a multispecific antibody (including bi-specific antibodies), a human antibody, a humanized antibody, a chimeric antibody, a single-chain Fv (scFv) (including bi-specific scFv), a BiTE molecule, a single chain antibody, a Fab fragments, a F(ab') fragment, a disulfide-linked Fv (sdFv), or an epitope-binding fragment of any of the above. The antibody may be any of an immunoglobulin molecule or immunologically active portion of an immunoglobulin molecule. Furthermore, the antibody may be of any isotype. For example, it may be any of isotypes IgG1, IgG2, IgG3 or IgG4. The antibody may be a full-length antibody comprising variable and constant regions, or an antigen-binding fragment thereof, such as a single chain antibody, or a Fab or Fab'2 fragment. The antibody may also be conjugated or linked to a therapeutic agent, such as a cytotoxin or a radioactive isotope.

A second agent other than the agent that binds to modulates IFNα activity may be administered to the patient. Second agents include, but are not limited to non-steroidal anti-inflammatory drugs such as ibuprofen, naproxen, sulindac, diclofenac, piroxicam, ketoprofen, diflunisal, nabumetone, etodolac, and oxaprozin, indomethacin; anti-malarial drugs such as hydroxychloroquine; corticosteroid hormones, such as prednisone, hydrocortisone, methylprednisolone, and dexamethasone; methotrexate; immunosuppressive agents, such as azathioprine and cyclophosphamide; and biologic agents that, *e.g.,* target T cells such as Alefacept and Efalizumab, or target TNFα, such as, Enbrel, Remicade, and Humira.

Treatment with the agent may result in neutralization of the differentially regulated miRNA profile. Treatment with the agent may result in a decrease in one or more symptoms of the type I IFN or an IFNα-mediated disease or disorder. Treatment with the agent may result in fewer flare-ups related to the type I IFN or an IFNα-mediated disease or disorder. Treatment with the agent may result in improved prognosis for the patient having the type I IFN or an IFNα-mediated disease or disorder. Treatment with the agent may result in a higher quality of life for the patient. Treatment with the agent may alleviate the need to co-administer second agents or may lessen the dosage of administration of the second agent to the patient. Treatment with the agent may reduce the number of hospitalizations of the patient that are related to the type I IFN or an IFNα-mediated disease or disorder.

The agent that binds to and modulates type I IFN or IFNα activity may neutralize a differentially regulated miRNA profile. Neutralization of the differentially regulated miRNA profile may be a reduction in at least one, at least two, at least three, at least five, at least seven, at least eight, at least ten, at least twelve, at least fifteen, at least twenty, at least twenty five, at least thirty, at least thirty five, at least forty, at least forty five, or at least fifty up-regulated miRNAs. The upregulated miRNAs may include any of those detected by or included in Tables 2 or 4 or as shown in figure sheets 382-383. Neutralization of the differentially regulated miRNA profile may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, at least ten, at least twelve, at least fifteen, at least twenty, at least twenty five, at least thirty, at least thirty five, at least forty, at least forty five, or at least fifty genes up-regulated in any differentially regulated miRNA profile. Alternatively, neutralization of the differentially regulated miRNA profile refers to a reduction of expression of up-regulated miRNAs that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those miRNAs in a control sample. If the agent that binds to and modulates type I IFN or IFNα activity is a biologic agent, such as an antibody, the agent may neutralize the type I IFN or IFNα profile at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

Neutralization of the differentially regulated miRNA profile may be an increase in down-regulated expression of at least one, at least two, at least three, at least five, at least seven, at least eight, at least ten, at least twelve, at least fifteen, at least twenty, at least twenty five, at least thirty, at least thirty five, at least forty, at least forty five, or at least fifty miRNAs. The downregulated miRNAs may include any of these detected by or included in Tables 3 or 5 or as shown in figure sheets 382-383. Neutralization of down-regulated genes in a differentially regulated miRNA profile is an increase of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90%, or at least 100%, or at least 125%, or at least 130%, or at least 140%, or at least 150%, or at least 175%, or at least 200%, or at least 250%, or at least 300%, or at least 500% of any of the at least one, at least two, at least three, at least five, at least seven, at least eight, at least ten, at least twelve, at least fifteen, at least twenty, or at least twenty five miRNAs whose expression is downregulated in any miRNA profile. Alternatively, neutralization of the differentially regulated miRNA profile refers to an increase in expression of miRNAs to within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those miRNAs in a control sample. If the agent that binds to and modulates type I IFN or IFNα activity is a biologic agent, such as an antibody, the agent may neutralize the differentially regulated miRNA profile at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The patient may further comprise a type I IFN or IFNα-inducible PD marker expression profile. The type I IFN or IFNα-inducible PD marker expression profile may be a strong profile, a moderate profile, or a weak profile. The type I IFN or IFNα-inducible PD marker expression profile can readily be designated as strong, moderate, or weak by determining the fold dysregulation of the type I IFN or IFNα-inducible PD marker expression profile of the patient, (e.g., the fold increase in expression of upregulated type I IFN or IFNα-inducible PD markers in the patient), relative to a control sample(s) or control patient(s) and comparing the patient's fold dysregulation to that of other patients having a type I IFN or IFNα-inducible PD marker expression profile. The type I IFN or IFNα-inducible PD marker expression profile may be any disclosed in PCT/US2007/024947 filed December 6, 2007, herein incorporated by reference.

The group of genes that may be included in the type I IFN or IFNα-inducible PD marker expression profile of the patient may be MX1, LY6E, IFI27, OAS1, IFIT1, IFI6, IFI44L, ISG15, LAMP3, OASL, RASD2, and IFI44. The genes or genes detected by the probes may include IFI44, IFI27, IFI44L, DNAPTP6, LAMP3, LY6E, RSAD2, HERC5, IFI6, ISG15, OAS3, SIGLEC1, OAS2, USP18, RTP4, IFIT1, MX1, OAS1, EPSTI1, PLSCR1, and IFRG28. The IFNα-inducible PD markers in such an expression profile may further include at least one or more gene listed in drawing sheets 235-381.

The genes may include any at least 2, any at least 3, any at least 4, any at least 5, any at least 6, any at least 7, any at least 8, any at least 9, any at least 10, or any at least 11, or any at least 12, or any at least 13, or any at least 14, or any at least 15, or any at least 16, or any at least 17, or any at least 18, or any at least 19, or at least 20, or any at least 21, or any at least 22, or any at least 23, or any at least 24, or any least 25, or any at least 26, or any at least 27, or any at least 28, or any at least 29, or any at least 30 of LAMP3, DNAPTP6, FLJ31033, HERC6, SERPING1, EPST11, RTP4, OASL, FBXO6, IFIT2, IFI44, OAS3, BATF2, ISG15, IRF7, RSAD2, IFI35, OAS1, LAP3, IFIT1, IFIT5, PLSCR1, IFI44L, MS4A4A, GALM, UBE2L6, TOR1B, SAMD9L, HERC5, TDRD7, TREX1, PARP12, and AXUD1. The IFNα-inducible PD markers in such an expression profile may further include at least one or more gene listed in drawing sheets 235-381.

The IFNα-inducible PD markers in an expression profile may include at least genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1. The IFNα-inducible PD markers in such an expression profile may further include at least one or more gene listed in drawing sheets 235-381.

The IFNα-inducible PD markers in an expression profile may include at least genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2. The IFNα-inducible PD markers in such an expression profile may further include at least one or more gene listed in drawing sheets 235-381.

The IFNα-inducible PD markers in an expression profile may include at least genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1. The IFNα-inducible PD markers in such an expression profile may further include at least one or more gene listed in drawing sheets 235-381.

The IFNα-inducible PD markers in an expression profile may include at least genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, EPSTI1, and RSAD2. The IFNα-inducible PD markers in such an expression profile may further include at least one or more gene listed in drawing sheets 235-381.

The IFNα-inducible PD markers in an expression profile may include at least genes BCL2, BAK1, BAD, BAX, and BCL2L1. The IFNα-inducible PD markers in such an expression profile may further include at least one or more gene listed in drawing sheets 235-381.

The IFNα-inducible PD markers in an expression profile may include at least genes RTP4, RSAD2, HERC5, SIGLEC1, USP18, LY6E, ETV7, SERPING1, IFIT3, OAS1, HSXIAPAF1, G1P3, MX1, OAS3, IFI27, DNAPTP6, LAMP3, EPSTI1, IFI44, OAS2, IFIT2, and ISG15. The IFNα-inducible PD markers in such an expression profile may further include at least one or more gene listed in drawing sheets 235-381.

The IFNα-inducible PD markers in an expression profile may include at least genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27. The IFNα-inducible PD markers in such an expression profile may further include at least one or more gene listed in drawing sheets 235-381.

The IFNα-inducible PD markers in an expression profile may include at least genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18. The IFNα-inducible PD markers in such an expression profile may further include at least one or more gene listed in drawing sheets 235-381.

The IFNα-inducible PD markers in an expression profile may include at least genes SAMD9L, IFI6, IFI44, IFIT2, ZC3HAV1, ETV6, DAPP1, IL1RN, CEACAM1, OAS1, IFI27, OAS3, IFI44L, HERC5, IFIT1, EPSTI1, ISG15, SERPING1, OASL, GBP1, and MX1. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, IFI27, MX1, IFIT1, HERC5, ISG15, LAMP3, OAS3, OAS1, EPSTI1, IFIT3, OAS2, LY6E, SIGLEC1, and USP18. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, IFI27, MX1, and IFIT1. The IFNα-inducible PD markers in such an expression profile may further include at least one or more gene listed in drawing sheets 235-381.

The IFNα-inducible PD markers in an expression profile may include at least genes SAMD9L, IFI6, IFI44, IFIT2, OAS1, IFI27, OAS3, IFI44L, HERC5, IFIT1, EPSTI1, ISG15, SERPING1, OASL, GBP1, and MX1. The IFNα-inducible PD markers in such an expression profile may further include at least one or more gene listed in drawing sheets 235-381.

The IFNα-inducible PD markers in an expression profile may include at least genes IFI27, RSAD2, IFI44L, IFI44, OAS1, IFIT1, ISG15, OAS3, HERC5, MX1, ESPTI1, IFIT3, and IFI6. The IFNα-inducible PD markers in such an expression profile may further include at least one or more gene listed in drawing sheets 235-381.

The IFNα-inducible PD markers in an expression profile may include at least genes IFI44L, RSAD2, IFI27, and IFI44. The IFNα-inducible PD markers in such an expression profile may further include at least one or more gene listed in drawing sheets 235-381.

The IFNα-inducible PD markers in an expression profile may include at least genes IFI44L and RSAD2. The IFNα-inducible PD markers in such an expression profile may further include at least one or more gene listed in drawing sheets 235-381.

The IFNα-inducible PD markers in an expression profile may include at least gene IFI444L. The IFNα-inducible PD markers in such an expression profile may further include at least one or more gene listed in drawing sheets 235-381.

The IFNα-inducible PD markers in an expression profile may include at least gene RSAD2. The IFNα-inducible PD markers in such an expression profile may further include at least one or more gene listed in drawing sheets 235-381.

The patient comprising the differentially regulated miRNA profile may further comprise downregulated type I IFN or IFNα PD marker(s). The downregulated PD markers may include any one, any two, any three, any four, any five, any six, or any seven, of genes CYP1B1, TGST1, RRAGD, IRS2, MGST1, TGFBR3, and RGS2.

The patient comprising the differentially regulated miRNA profile may further comprise upregulation of expression of any number of IFNα or type-I IFN subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. The patient may comprise upregulation of expression of IFN subtypes IFNα1, IFNα2, IFNα8, and IFNα14.

The patient comprising the differentially regulated miRNA profile may further comprise upregulation of expression of IFNα receptors, either IFNAR1 or IFNAR2, or both, or TNFα, or IFNγ, or IFNγ receptors (either IFNGR1, IFNGR2, or both IFNGR1 and IFNGR2). The patient may simply be identified as one who comprises upregulation of expression of IFNα receptors, either IFNAR1 or IFNAR2, or both, or TNFα, or IFNγ, or IFNγ receptors (either IFNGR1, IFNGR2, or both IFNGR1 and IFNGR2).

The upregulation or downregulation of the type I IFN or IFNα-inducible PD markers in the patient's expression profile may be by any degree relative to that of a sample from a control (which may be from a sample that is not disease tissue of the patient (e.g., non-lesional skin of a psoriasis patient) or from a healthy person not afflicted with the disease or disorder). The degree upregulation or downregulation may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control or control sample.

Furthermore, the patient may overexpress or have a tissue that overexpresses a type I IFN subtype at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control. The type I IFN subtype may be any one of IFNα1, IFNα2, IFNα4, IFNα5, IFNα6, IFNα7, IFNα8, IFNα10, IFNα14, IFNα17, IFNα21, IFNβ, or IFNω. The type I IFN subtypes may include all of IFNα1, IFNα2, IFNα8, and IFNα14.

The patient may further comprise or alternatively comprise alterations in levels of proteins in serum. The patient may have increased serum levels of proteins such as adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, or vWF. The patient may have increased serum levels of any 1, 2,3, 4, 5, 6, 7, 8, 9, 10, 11, 12, 13 14, 15, 16, 17, 18, 19, 20, 21, o22, 23, 24, 25, or 26 of these proteins in serum. The increased level may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of a control, e.g., a healthy subject. The alteration may be a decrease in serum levels of proteins such as BDNK, complement 3, CD40 ligand, EGF, ENA-78, EN-RAGE, IGF-1, MDC, myeloperoxidase, RANTES, or thrombopoietin, The patient may have decreased serum levels of any 1, 2, 3, 4, 5, 6, 7, 8, 9, 10, or 11 or these proteins. The decreased level may be at least 10%, at least 15%, at least 20%, at least 25%, at least 30%, at least 40%, at least 50%, at least 60%, at least 70%, at least 75%, at least 80%, at least 90%, or at least 100% that of a control, e.g., a healthy subject. The PD marker profile may comprise one or more of these increased or decreased serum levels of proteins.

The patient may further comprise auto-antibodies that bind to any one of the following auto-antigens: (a) Myxovirus (influenza virus) resistance 1, interferon-inducible protein p78; (b) surfeit 5, transcript variant c; (c) proteasome (posome, macropain) activator subunit 3 (PA28 gamma; Ki) transc; (d) retinoic acid receptor, alpha; (e) Heat shock 10 kDa protein 1 (chaperonin 10); (f) tropomyosin 3; (g) pleckstrin homology-like domain, family A, member 1; (h) cytoskeleton-associated protein 1; (i) Sjogren syndrome antigen A2 (60 kDa, ribonucleoprotein auto-antigen SS-A/Ro); (j) NADH dehydrogenase (ubiquinone) 1, alpha/beta subcomplex 1, 8 kDa; (k) NudE nuclear distribution gene E homolog 1 (A. nidulans); (1) MutL homolog 1, colon cancer, nonpolyposis type 2 (E. coli); (m) leucine rich repeat (in FLII) interacting protein 2; (n) tropomyosin 1 (alpha); (o) spastic paraplegia 20, spartin (Troyer syndrome); (p) preimplantation protein, transcript variant 1; (r) mitochondrial ribosomal protein L45; (s) Lin-28 homolog (C. elegans); (t) heat shock 90 kDa protein 1, alpha; (u) dom-3 homolog Z (C. elegans); (v) dynein, cytoplasmic, light intermediate polypeptide 2; (w) Ras-related C3 botulinum toxin substrate 1 (rho family, small GTP binding protein); (x) synovial sarcoma, X breakpoint 2, transcript variant 2; (y) moesin; (z) homer homolog (Drosophila), transcript variant 1; (aa) GCN5 general control of amino-acid synthesis 5-like 2 (yeast); (bb) eukaryotic translation elongation factor 1 gamma; (cc) eukaryotic translation elongation factor 1, delta; (dd) DNA-damage-inducible transcript 3; (ee) CCAAT/enhancer binding protein (C/EBP) gamma; and any other auto-antigen described in provisional application entitled "Auto-antibody markers of autoimmune disease" filed May 3, 2007 or in provisional application entitled entitled "Auto-antibody markers of autoimmune disease" to be filed November 6, 2007 (for example, but not limited to, those described on Tables 2, 4, 5, and 9). The patient may comprise auto-antibodies that bind to any number of these auto-antigens, e.g., any at least 2, at least 3, at least 4, at least 5, at least 6, at least 7, at least 8, at least 9 at least 10, at least 11, at least 12, at least 13, at least 14, at least 15, at least 20, at least 25.

Administration of the agent that binds to and modulates type I IFN or an IFNα activity may further neutralize a type I IFN or IFNα-inducible profile to within at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% that of a control sample.

In methods of monitoring or prognosing disease progression of a patient, samples from the patient may be obtained before and after administration of an agent.

Samples include any biological fluid or tissue, such as whole blood, serum, muscle, saliva, urine, synovial fluid, bone marrow, cerebrospinal fluid, nasal secretions, sputum, amniotic fluid, bronchoalveolar lavage fluid, peripheral blood mononuclear cells, total white blood cells, lymph node cells, spleen cells, tonsil cells, or skin. The samples may be obtained by any means known in the art.

miRNA profiles are obtained in the (before and after agent administration) samples. The miRNA profiles in the samples are compared. Comparison may be of the number of miRNAs present in the samples or may be of the quantity of miRNAs present in the samples, or any combination thereof. Variance indicating efficacy of the therapeutic agent may be indicated if the number or level (or any combination thereof) of up-regulated miRNAs decreases in the sample obtained after administration of the therapeutic agent relative to the sample obtained before administration of the therapeutic agent. The number of up-regulated miRNAs may decrease by at least 1, at least 2, at least 3, at least 4, at least 5, at least 6, at least 7, at least 8, at least 9, or at least 10. The level of any given up-regulated miRNAs may decrease by at least 10%, at least 20%, at least 25%, at least 30%, at least 35%, at least 40%, at least 50%, at least 60%, at least 70%, at least 80%, at least 90%, or at least 95%. The number of up-regulated miRNAs with decreased levels may be at least 1, at least 2, at least 3, at least 4, at least 5, at least 6, at least 7, at least 8, at least 9, at least 10, at least 15, at least 20, at least 25, at least 30, or at least 35. Any combination of decreased number and decreased level of up-regulated miRNAs may indicate efficacy. Variance indicating efficacy of the therapeutic agent may be indicated if the number or level (or any combination thereof) of down-regulated miRNAs decreases in the sample obtained after administration of the therapeutic agent relative to the sample obtained before administration of the therapeutic agent. The number of down-regulated miRNAs may decrease by at least 1, at least 2, at least 3, at least 4, at least 5, at least 6, at least 7, at least 8, at least 9, or at least 10. The level of any given down-regulated miRNA may increase by at least 10%, at least 20%, at least 25%, at least 30%, at least 35%, at least 40%, at least 50%, at least 60%, at least 70%, at least 80%, at least 90%, or at least 95%. The number of down-regulated miRNAs with increased levels may be at least 1, at least 2, at least 3, at least 4, at least 5, at least 6, at least 7, at least 8, at least 9, at least 10, at least 15, at least 20, at least 25, at least 30, or at least 35. Any combination of decreased number and increased level of down-regulated miRNAs may indicate efficacy.

The sample obtained from the patient may be obtained prior to a first administration of the agent, *i.e*., the patient is naïve to the agent. Alternatively, the sample obtained from the patient may occur after administration of the agent in the course of treatment. For example, the agent may have been administered prior to the initiation of the monitoring protocol. Following administration of the agent an additional samples may be obtained from the patient and type I IFN or IFNα inducible PD markers in the samples are compared. The samples may be of the same or different type, e.g., each sample obtained may be a blood sample, or each sample obtained may be a serum sample. The type I IFN or IFNα inducible PD markers detected in each sample may be the same, may overlap substantially, or may be similar.

The samples may be obtained at any time before and after the administration of the therapeutic agent. The sample obtained after administration of the therapeutic agent may be obtained at least 2, at least 3, at least 4, at least 5, at least 6, at least 7, at least 8, at least 9, at least 10, at least 12, or at least 14 days after administration of the therapeutic agent. The sample obtained after administration of the therapeutic agent may be obtained at least 2, at least 3, at least 4, at least 5, at least 6, at least 7, or at least 8 weeks after administration of the therapeutic agent. The sample obtained after administration of the therapeutic agent may be obtained at least 2, at least 3, at least 4, at least 5, or at least 6 months following administration of the therapeutic agent.

Additional samples may be obtained from the patient following administration of the therapeutic agent. At least 2, at least 3, at least 4, at least 5, at least 6, at least 7, at least 8, at least 9, at least 10, at least 12, at least 15, at least 20, at least 25 samples may be obtained from the patient to monitor progression or regression of the disease or disorder over time. Disease progression may be monitored over a time period of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least 1 year, at least 2 years, at least 3 years, at least 4 years, at least 5 years, at least 10 years, or over the lifetime of the patient. Additional samples may be obtained from the patient at regular intervals such as at monthly, bi-monthly, once a quarter year, twice a year, or yearly intervals. The samples may be obtained from the patient following administration of the agent at regular intervals. For instance, the samples may be obtained from the patient at one week following each administration of the agent, or at two weeks following each administration of the agent, or at three weeks following each administration of the agent, or at one month following each administration of the agent, or at two months following each administration of the agent. Alternatively, multiple samples may be obtained from the patient following each administration of the agent.

Disease progression in a patient may similarly be monitored in the absence of administration of an agent. Samples may periodically be obtained from the patient having the disease or disorder. Disease progression may be identified if the number of miRNAs increases in a later-obtained sample relative to an earlier obtained sample. The number of miRNAs may increase by at least 1, at least 2, at least 3, at least 4, at least 5, at least 6, at least 7, at least 8, at least 9, or at least 10. Disease progression may be identified if level of any given up-regulated miRNAs increases by at least 10%, at least 20%, at least 25%, at least 30%, at least 35%, at least 40%, at least 50%, at least 60%, at least 70%, at least 80%, at least 90%, or at least 95%. Disease progression may be identified if level of any given down-regulated miRNAs decreases by at least 10%, at least 20%, at least 25%, at least 30%, at least 35%, at least 40%, at least 50%, at least 60%, at least 70%, at least 80%, at least 90%, or at least 95%. The number of up-regulated miRNAs with increased levels may be at least 1, at least 2, at least 3, at least 4, at least 5, at least 6, at least 7, at least 8, at least 9, at least 10, at least 15, at least 20, at least 25, at least 30, or at least 35. The number of down-regulated miRNAs with decreased levels may be at least 1, at least 2, at least 3, at least 4, at least 5, at least 6, at least 7, at least 8, at least 9, at least 10, at least 15, at least 20, at least 25, at least 30, or at least 35. Any combination of increased number and increased level of up-regulated miRNA may indicate disease progression. Alternatively, or in combination, any combination of decreased number and decreased level of down-regulated type miRNA may indicate disease progression. Disease regression may also be identified in a patient having a disease or disorder, not treated by an agent. In this instance, regression may be identified if the number of miRNAs decreases in a later-obtained sample relative to an earlier obtained sample. The number of miRNAs may decrease by at least 1, at least 2, at least 3, at least 4, at least 5, at least 6, at least 7, at least 8, at least 9, or at least 10. Disease regression may be identified if level of any given up-regulated miRNA decreases by at least 10%, at least 20%, at least 25%, at least 30%, at least 35%, at least 40%, at least 50%, at least 60%, at least 70%, at least 80%, at least 90%, or at least 95%. Disease regression may be identified if level of any given down-regulated miRNA increases by at least 10%, at least 20%, at least 25%, at least 30%, at least 35%, at least 40%, at least 50%, at least 60%, at least 70%, at least 80%, at least 90%, or at least 95%. The number of up-regulated miRNAs with decreased levels may be at least 1, at least 2, at least 3, at least 4, at least 5, at least 6, at least 7, at least 8, at least 9, at least 10, at least 15, at least 20, at least 25, at least 30, or at least 35. The number of down-regulated miRNAs with increased levels may be at least 1, at least 2, at least 3, at least 4, at least 5, at least 6, at least 7, at least 8, at least 9, at least 10, at least 15, at least 20, at least 25, at least 30, or at least 35. Disease progression or disease regression may be monitored by obtaining samples over any period of time and at any interval. Disease progression or disease regression may be monitored by obtaining samples over the course of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least 1 year, at least 2 years, at least 3 years, at least 4 years, at least 5 years, at least 10 years, or over the lifetime of the patient. Disease progression or disease regression may be monitored by obtaining samples at least monthly, bi-monthly, once a quarter year, twice a year, or yearly. The samples need not be obtained at strict intervals.

Variance in the samples may guide treatment strategy of a disease or disorder. Treatment strategy may be dosage of a particular therapeutic, or may be removal or addition of particular therapeutics administered to a patient.

The invention also encompasses methods employing IFNα-inducible PD markers to treat, diagnose, prognose, and monitor myositis. These IFNα-inducible PD markers can also be used to guide dosage and treatment of myositis patients or models of myositis disease.

The type I IFN or IFNα-inducible PD marker expression profile may comprise upregulation or downregulation of any type I IFN or IFNα-inducible PD marker expression profile genes or group of genes relative to a control, e.g. healthy, patient or sample of non-disease tissue of a patient. The gene or group of genes may include any at least 2, any at least 3, any at least 4, any at least 5, any at least 6, any at least 7, any at least 8, any at least 9, any at least 10, any at least 11, any at least 12, any at least 13, any at least 14, any at least 15, any at least 16, any at least 17, any at least 18, any at least 19, any at least 20, any at least 21, any at least 22, any at least 23, any at least 24, any at least 25, any at least 26, any at least 27, any at least 28, any at least 29, any at least 30, any at least 40, any at least 50 genes, any at least 75 gene, any at least 100 genes, or any at least 150 genes.

The IFNα-inducible PD markers in an expression profile may include at least genes EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, USP18. The IFNα-inducible PD markers in such an expression profile may further include at least one or more gene listed in drawing sheets 235-381.

The IFNα-inducible PD markers in an expression profile may include at least genes IFI27, SIGLEC1, RSAD2, IFI6, IFI44L, IFI44, USP18, IFIT2, SAMD9L, BIRC4BP, DNAPTP6, OAS3, LY6E, IFIT1, LIPA, LOC129607, ISG15, PARP14, MX1, OAS2, OASL, CCL2, HERC5, OAS1. The IFNα-inducible PD markers in such an expression profile may further include at least one or more gene listed in drawing sheets 235-381.

The IFNα-inducible PD markers in an expression profile may include at least genes IFIT1, IFIT3, IRF7, IFI6, IL6ST, IRF2, LY6E, MARCKS, MX1, MX2, OAS1, EIF2AK2, ISG15, STAT2, OAS3, IFI44, IFI44L, HERC5, RAB8B, LILRA5, RSAD2, and FCHO2. The IFNα-inducible PD markers in such an expression profile may further include at least one or more gene listed in drawing sheets 235-381.

The IFNα-inducible PD markers in an expression profile may include at least genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, and EPSTI1. The IFNα-inducible PD markers in such an expression profile may further include at least one or more gene listed in drawing sheets 235-381.

The IFNα-inducible PD markers in an expression profile may include at least genes SERPING1, IFIT2, IFIT3, IFI6, LY6E, MX1, OAS1, ISG15, IFI27, OAS3, IFI44, LAMP3, DNAPTP6, ETV7, HERC5, OAS2, USP18, XAF1, RTP4, SIGLEC1, EPSTI1, and RSAD2. The IFNα-inducible PD markers in such an expression profile may further include at least one or more gene listed in drawing sheets 235-381.

The IFNα-inducible PD markers in an expression profile may include at least genes RTP4, RSAD2, HERC5, SIGLEC1, USP18, LY6E, ETV7, SERPING1, IFIT3, OAS1, HSXIAPAF1, G1P3, MX1, OAS3, IFI27, DNAPTP6, LAMP3, EPSTI1, IFI44, OAS2, IFIT2, and ISG15. The IFNα-inducible PD markers in such an expression profile may further include at least one or more gene listed in drawing sheets 235-381.

The IFNα-inducible PD markers in an expression profile may include at least genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27. The IFNα-inducible PD markers in such an expression profile may further include at least one or more gene listed in drawing sheets 235-381.

The IFNα-inducible PD markers in an expression profile may include at least genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18. The IFNα-inducible PD markers in such an expression profile may further include at least one or more gene listed in drawing sheets 235-381.

The IFNα-inducible PD markers in an expression profile may include at least genes SAMD9L, IFI6, IFI44, IFIT2, ZC3HAV1, ETV6, DAPP1, IL1RN, CEACAM1, OAS1, IFI27, OAS3, IFI44L, HERC5, IFIT1, EPSTI1, ISG15, SERPING1, OASL, GBP1, and MX1. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, IFI27, MX1, IFIT1, HERC5, ISG15, LAMP3, OAS3, OAS1, EPSTI1, IFIT3, OAS2, LY6E, SIGLEC1, and USP18. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, IFI27, MX1, and IFIT1. The IFNα-inducible PD markers in such an expression profile may further include at least one or more gene listed in drawing sheets 235-381.

The IFNα-inducible PD markers in an expression profile may include at least genes IFI6, RSAD2, IFI44, IFI44L, and IFI27. The IFNα-inducible PD markers in such an expression profile may further include at least one or more gene listed in drawing sheets 235-381.

The IFNα-inducible PD markers in an expression profile may include at least genes SAMD9L, IFI6, IFI44, IFIT2, OAS1, IFI27, OAS3, IFI44L, HERC5, IFIT1, EPSTI1, ISG15, SERPING1, OASL, GBP1, and MX1. The IFNα-inducible PD markers in such an expression profile may further include at least one or more gene listed in drawing sheets 235-381.

The IFNα-inducible PD markers in an expression profile may include at least genes IFI27, RSAD2, IFI44L, IFI44, OAS1, IFIT1, ISG15, OAS3, HERC5, MX1, ESPTI1, IFIT3, and IFI6. The IFNα-inducible PD markers in such an expression profile may further include at least one or more gene listed in drawing sheets 235-381.

The IFNα-inducible PD markers in an expression profile may include at least genes IFI44L, RSAD2, IFI27, and IFI44. The IFNα-inducible PD markers in such an expression profile may further include at least one or more gene listed in drawing sheets 235-381.

The IFNα-inducible PD markers in an expression profile may include at least genes IFI44L and RSAD2. The IFNα-inducible PD markers in such an expression profile may further include at least one or more gene listed in drawing sheets 235-381.

The IFNα-inducible PD markers in an expression profile may include at least gene IFI444L. The IFNα-inducible PD markers in such an expression profile may further include at least one or more gene listed in drawing sheets 235-381.

The IFNα-inducible PD markers in an expression profile may include at least gene RSAD2. The IFNα-inducible PD markers in such an expression profile may further include at least one or more gene listed in drawing sheets 235-381.

The IFNα-inducible PD markers in an expression profile may include at least genes IFI27, IL-121R beta2, IL-15R alpha, IL-15, suppressor of cytokine signaling 1 (SOCS1), janus kinase 2, CXCL11 (T-TAC), TNFSF13B (BAFF), TRAF-type domain 1 (TRAFD1), SERPING1, CD274 (PD1-L), indoleamine 2,3 dioxygenase (INDO), lymphocyte-activation gene 3 (LAG3), and caspase 5. The IFNα-inducible PD markers in such an expression profile may further include at least one or more gene listed in drawing sheets 235-381.

IFNα-inducible PD markers or a PD marker profile may include any at least 5 genes such as, for example: MX1, LLY6E, IFI27, OAS1, IFIT1; or MX1, LLY6E, IFI27, OAS1, IFI6; or MX1, LLY6E, IFI27, OAS1, IFI44L; or MX1, LLY6E, IFI27, OAS1, ISG15; or MX1, LLY6E, IFI27, OAS1, LAMP3; or MX1, LLY6E, IFI27, OAS1, OASL; or MX1, LLY6E, IFI27, OAS1, RSAD2; or MX1, LLY6E, IFI27, OAS1, IFI44; or MX1, LLY6E, IFI27, OAS1, IFIT2; or MX1, LLY6E, IFI27, OAS1, OAS3; or MX1, LLY6E, IFI27, OAS1, USP18; or MX1, LLY6E, IFI27, OAS1, SIGLEC1; or MX1, LLY6E, IFI27, OAS1, HERC5; or MX1, LLY6E, IFI27, OAS1, DNAPTP6; or MX1, LLY6E, IFI27, OAS1, LOC129607; or MX1, LLY6E, IFI27, OAS1, EPSTI1; or MX1, LLY6E, IFI27, OAS1, BIRC4BP; or MX1, LLY6E, IFI27, OAS1, SIGLEC1; or MX1, LLY6E, IFI27, OAS1, gene detected by probe 229450_at; or MX1, LLY6E, IFI27, OAS1, gene detected by probe 235276_at; or LLY6E, IFI27, OAS1, IFIT1, IFI6; or LLY6E, IFI27, OAS1, IFIT1, IFI44L; or LLY6E, IFI27, OAS1, IFIT1, ISG15; or LLY6E, IFI27, OAS1, IFIT1, LAMP3; or LLY6E, IFI27, OAS1, IFIT1, OASL; or LLY6E, IFI27, OAS1, IFIT1, RSAD2; or LLY6E, IFI27, OAS1, IFIT1, IFI44; or LLY6E, IFI27, OAS1, IFIT1, IFIT2; or LLY6E, IFI27, OAS1, IFIT1, OAS3; or LLY6E, IFI27, OAS1, IFIT1, USP18; or LLY6E, IFI27, OAS1, IFIT1, SIGLEC1; or LLY6E, IFI27, OAS1, IFIT1, HERC5; or LLY6E, IFI27, OAS1, IFIT1, DNAPTP6; or LLY6E, IFI27, OAS1, IFIT1, LOC129607; or LLY6E, IFI27, OAS1, IFIT1, EPSTI1; or LLY6E, IFI27, OAS1, IFIT1, BIRC4BP; or LLY6E, IFI27, OAS1, IFIT1, SIGLEC1; or LLY6E, IFI27, OAS1, IFIT1, gene detected by probe 229450_at; or LLY6E, IFI27, OAS1, IFIT1, gene detected by probe 235276_at; or IFI27, OAS1, IFIT1, IFI6, IFI44L, ISG15; or IFI27, OAS1, IFIT1, IFI6, LAMP3; or IFI27, OAS1, IFIT1, IFI6, OASL; or IFI27, OAS1, IFIT1, IFI6, RSAD2; or IFI27, OAS1, IFIT1, IFI6, IFI44; or IFI27, OAS1, IFIT1, IFI6, IFIT2; or IFI27, OAS1, IFIT1, IFI6, OAS3; or IFI27, OAS1, IFIT1, IFI6, USP18; or IFI27, OAS1, IFIT1, IFI6, SIGLEC1; or IFI27, OAS1, IFIT1, IFI6, HERC5; or IFI27, OAS1, IFIT1, IFI6, DNAPTP6; or IFI27, OAS1, IFIT1, IFI6, LOC129607; or IFI27, OAS1, IFIT1, IFI6, EPSTI1; or IFI27, OAS1, IFIT1, IFI6, BIRC4BP; or IFI27, OAS1, IFIT1, IFI6, SIGLEC1; or IFI27, OAS1, IFIT1, IFI6, gene detected by probe 229450_at; or IFI27, OAS1, IFIT1, IFI6, gene detected by probe 235276_at; or OAS1, IFIT1, IFI6, IFI44L, ISG15; or OAS1, IFIT1, IFI6, IFI44L, LAMP3; or OAS1, IFIT1, IFI6, IFI44L, OASL; or OAS1, IFIT1, IFI6, IFI44L, RSAD2; or OAS1, IFIT1, IFI6, IFI44L, IFI44; or OAS1, IFIT1, IFI6, IFI44L, IFIT2; or OAS1, IFIT1, IFI6, IFI44L, OAS3; or OAS1, IFIT1, IFI6, IFI44L, USP18; or OAS1, IFIT1, IFI6, IFI44L, SIGLEC1; or OAS1, IFIT1, IFI6, IFI44L, HERC5; or OAS1, IFIT1, IFI6, IFI44L, DNAPTP6; or OAS1, IFIT1, IFI6, IFI44L, LOC129607; or OAS1, IFIT1, IFI6, IFI44L, EPSTI1; or OAS1, IFIT1, IFI6, IFI44L, BIRC4BP; or OAS1, IFIT1, IFI6, IFI44L, SIGLEC1; or OAS1, IFIT1, IFI6, IFI44L, gene detected by probe 229450_at; or OAS1, IFIT1, IFI6, IFI44L, gene detected by probe 235276_at; or IFIT1, IFI6, IFI44L, ISG15, LAMP3; or IFIT1, IFI6, IFI44L, ISG15, OASL; or IFIT1, IFI6, IFI44L, ISG15, RSAD2; or IFIT1, IFI6, IFI44L, ISG15, IFI44; or IFIT1, IFI6, IFI44L, ISG15, IFIT2 or IFIT1, IFI6, IFI44L, ISG15, OAS3; or IFIT1, IFI6, IFI44L, ISG15, USP18; or IFIT1, IFI6, IFI44L, ISG15, SIGLEC1; or IFIT1, IFI6, IFI44L, ISG15, HERC5; or IFIT1, IFI6, IFI44L, ISG15, DNAPTP6; or IFIT1, IFI6, IFI44L, ISG15, LOC129607; or IFIT1, IFI6, IFI44L, ISG15, EPSTI1; or IFIT1, IFI6, IFI44L, ISG15, BIRC4BP; or IFIT1, IFI6, IFI44L, ISG15, gene detected by probe 229450_at; or IFIT1, IFI6, IFI44L, ISG15, gene detected by probe 235276_at; or IFI6, IFI44L, ISG15, LAMP3, HERC5; or IFI6, IFI44L, ISG15, LAMP3, DNAPTP6; or IFI6, IFI44L, ISG15, LAMP3, LOC129607; or IFI6, IFI44L, ISG15, LAMP3, EPSTI1; or IFI6, IFI44L, ISG15, LAMP3, BIRC4BP; or IFI6, IFI44L, ISG15, LAMP3, gene detected by probe 229450_at; or IFI6, IFI44L, ISG15, LAMP3, gene detected by probe 235276_at; or IFI6, IFI44L, ISG15, LAMP3, SIGLEC1; or IFI6, IFI44L, ISG15, LAMP3, USP18; or IFI6, IFI44L, ISG15, LAMP3, OAS3; or IFI6, IFI44L, ISG15, LAMP3, IFIT2; or IFI6, IFI44L, ISG15, LAMP3, IFI44; or IFI6, IFI44L, ISG15, LAMP3, RSAD2; or IFI6, IFI44L, ISG15, LAMP3, OASL; or IFI44L, ISG15, LAMP3, OASL, RSAD2; or IFI44L, ISG15, LAMP3, OASL, IFI44; or IFI44L, ISG15, LAMP3, OASL, IFIT2; or IFI44L, ISG15, LAMP3, OASL, OAS3; or IFI44L, ISG15, LAMP3, OASL, USP18; or IFI44L, ISG15, LAMP3, OASL, SIGLEC1; or IFI44L, ISG15, LAMP3, OASL, HERC5; or IFI44L, ISG15, LAMP3, OASL, DNAPTP6; or IFI44L, ISG15, LAMP3, OASL, LOC129607; or IFI44L, ISG15, LAMP3, OASL, EPSTI1;or IFI44L, ISG15, LAMP3, OASL, BIRC4BP; or IFI44L, ISG15, LAMP3, OASL, gene detected by probe 229450_at; or IFI44L, ISG15, LAMP3, OASL, gene detected by probe 235276_at; or ISG15, LAMP3, OASL, RSAD2, IFI44; or ISG15, LAMP3, OASL, RSAD2, IFIT2; or ISG15, LAMP3, OASL, RSAD2, OAS3; or ISG15, LAMP3, OASL, RSAD2, USP18; or ISG15, LAMP3, OASL, RSAD2, SIGLEC1; or ISG15, LAMP3, OASL, RSAD2, HERC5; or ISG15, LAMP3, OASL, RSAD2, DNAPTP6; or ISG15, LAMP3, OASL, RSAD2, LOC129607; or ISG15, LAMP3, OASL, RSAD2, EPSTI1; or ISG15, LAMP3, OASL, RSAD2, BIRC4BP; or ISG15, LAMP3, OASL, RSAD2, gene detected by probe 229450_at; or ISG15, LAMP3, OASL, RSAD2, gene detected by probe 235276_at; or LAMP3, OASL, RSAD2, IFI44, IFIT2; or LAMP3, OASL, RSAD2, IFI44, OAS3; or LAMP3, OASL, RSAD2, IFI44, USP18; or LAMP3, OASL, RSAD2, IFI44, SIGLEC1; or LAMP3, OASL, RSAD2, IFI44, HERC5; or LAMP3, OASL, RSAD2, IFI44, DNAPTP6; or LAMP3, OASL, RSAD2, IFI44, LOC1129607; or LAMP3, OASL, RSAD2, IFI44, EPSTI1; or LAMP3, OASL, RSAD2, IFI44, BIRC4BP; or LAMP3, OASL, RSAD2, IFI44, gene detected by probe 229450_at; or LAMP3, OASL, RSAD2, IFI44, gene detected by probe 235276_at; or OASL, RSAD2, IFI44, IFIT2, OAS3; or OASL, RSAD2, IFI44, IFIT2, USP18; or OASL, RSAD2, IFI44, IFIT2, SIGLEC1; or OASL, RSAD2, IFI44, IFIT2, HERC5; or OASL, RSAD2, IFI44, IFIT2, DNAPTP6; or OASL, RSAD2, IFI44, IFIT2, LOC129607; or OASL, RSAD2, IFI44, IFIT2, EPSTI1; or OASL, RSAD2, IFI44, IFIT2, BIRC4BP; or OASL, RSAD2, IFI44, IFIT2, gene detected by probe 229450_at; or OASL, RSAD2, IFI44, IFIT2, gene detected by probe 235276_at; or RSAD2, IFI44, IFIT2, OAS3, USP18; or RSAD2, IFI44, IFIT2, OAS3, SIGLEC1; or RSAD2, IFI44, IFIT2, OAS3, HERC5; or RSAD2, IFI44, IFIT2, OAS3, DNAPTP6; or RSAD2, IFI44, IFIT2, OAS3, LOC129607; or RSAD2, IFI44, IFIT2, OAS3, EPSTI1; or RSAD2, IFI44, IFIT2, OAS3, BIRC4BP; or RSAD2, IFI44, IFIT2, OAS3, gene detected by probe 229450_at; or RSAD2, IFI44, IFIT2, OAS3, gene detected by probe 235276_at; or IFI44, IFIT2, OAS3, USP18, SIGLEC1; or IFI44, IFIT2, OAS3, USP18, HERC5; or IFI44, IFIT2, OAS3, USP18, DNAPTP6; or IFI44, IFIT2, OAS3, USP 18, LOC129607; or IFI44, IFIT2, OAS3, USP18, EPSTI1; or IFI44, IFIT2, OAS3, USP18, BIRC4BP; or IFI44, IFIT2, OAS3, USP18, gene detected by probe 229450_at; or IFI44, IFIT2, OAS3, USP18, gene detected by probe 235276_at; or IFIT2, OAS3, USP18, SIGLEC1, HERC5; or IFIT2, OAS3, USP18, SIGLEC1, DNAPTP6; or IFIT2, OAS3, USP18, SIGLEC1, LOC129607; or IFIT2, OAS3, USP18, SIGLEC1, EPSTI1; or IFIT2, OAS3, USP18, SIGLEC1, BIRC4BP; or IFIT2, OAS3, USP18, SIGLEC1, gene detected by probe 229450_at; or IFIT2, OAS3, USP18, SIGLEC1, gene detected by probe 235276_at; or OAS3, USP18, SIGLEC1, HERC5, DNAPTP6; or OAS3, USP18, SIGLEC1, HERC5, LOC129607; or OAS3, USP18, SIGLEC1, HERC5, EPSTI1; or OAS3, USP18, SIGLEC1, HERC5, BIRC4BP; or OAS3, USP18, SIGLEC1, HERC5, gene detected by probe 229450_at; or OAS3, USP18, SIGLEC1, HERC5, gene detected by probe 235276_at; or USP18, SIGLEC1, HERC5, DNAPTP6, LOC129607; or USP18, SIGLEC1, HERC5, DNAPTP6, EPSTI1; or USP18, SIGLEC1, HERC5, DNAPTP6, BIRC4BP; or USP18, SIGLEC1, HERC5, DNAPTP6, gene detected by probe 229450_at; or USP18, SIGLEC1, HERC5, DNAPTP6, gene detected by probe 235276_at; or SIGLEC1, HERC5, DNAPTP6, LOC129607, EPSTI1; or SIGLEC1, HERC5, DNAPTP6, LOC129607, BIRC4BP; or SIGLEC1, HERC5, DNAPTP6, LOC129607, gene detected by probe 229450_at; or SIGLEC1, HERC5, DNAPTP6, LOC129607, gene detected by probe 235276_at; or HERC5, DNAPTP6, LOC129607, EPSTI1, BIRC4BP; or HERC5, DNAPTP6, LOC129607, EPSTI1, gene detected by probe 229450_at; or HERC5, DNAPTP6, LOC129607, EPSTI1, gene detected by probe 235276_at; or DNAPTP6, LOC129607, EPSTI1, BIRC4BP, gene detected by probe 229450_at; or DNAPTP6, LOC129607, EPSTI1, BIRC4BP, gene detected by probe 235276_at; or LOC129607, EPSTI1, BIRC4BP, gene detected by probe 229450_at, gene detected by probe 235276_at. The IFNα-inducible PD markers in such an expression profile may further include at least one or more gene listed in drawing sheets 235-381.

The IFNα-inducible PD markers in an expression profile may include any at least 6 genes such as, for example: MX1, LLY6E, IFI27, OAS1, IFIT1, IFI6; or MX1, LLY6E, IFI27, OAS1, IFIT1, IFI44L; or MX1, LLY6E, IFI27, OAS1, IFIT1, ISG15; or MX1, LLY6E, IFI27, OAS1, IFIT1, LAMP3; or MX1, LLY6E, IFI27, OAS1, IFIT1, OASL; or MX1, LLY6E, IFI27, OAS1, IFIT1, RSAD2; or MX1, LLY6E, IFI27, OAS1, IFIT1, IFI44; or MX1, LLY6E, IFI27, OAS1, IFIT1, IFIT2; or MX1, LLY6E, IFI27, OAS1, IFIT1, OAS3; or MX1, LLY6E, IFI27, OAS1, IFIT1, USP18; or MX1, LLY6E, IFI27, OAS1, IFIT1, SIGLEC1; or MX1, LLY6E, IFI27, OAS1, IFIT1, HERC5; or MX1, LLY6E, IFI27, OAS1, IFIT1, DNAPTP6; or MX1, LLY6E, IFI27, OAS1, IFIT1, LOC129607; or MX1, LLY6E, IFI27, OAS1, IFIT1, EPSTI1; or MX1, LLY6E, IFI27, OAS1, IFIT1, BIRC4BP;or MX1, LLY6E, IFI27, OAS1, IFIT1, gene detected by probe 229450_at; or MX1, LLY6E, IFI27, OAS1, IFIT1, gene detected by probe 235276_at; or LLY6E, IFI27, OAS1, IFIT1, IFI6, IFI44L; or LLY6E, IFI27, OAS1, IFIT1, IFI6, ISG15; or LLY6E, IFI27, OAS1, IFIT1, IFI6, LAMP3; or LLY6E, IFI27, OAS1, IFIT1, IFI6, OASL ; or LLY6E, IFI27, OAS1, IFIT1, IFI6, RSAD2; or LLY6E, IFI27, OAS1, IFIT1, IFI6, IFI44; or LLY6E, IFI27, OAS1, IFIT1, IFI6, IFIT2; or LLY6E, IFI27, OAS1, IFIT1, IFI6, OAS3; or LLY6E, IFI27, OAS1, IFIT1, IFI6, USP18; or LLY6E, IFI27, OAS1, IFIT1, IFI6, SIGLEC1; or LLY6E, IFI27, OAS1, IFIT1, IFI6, HERC5; or LLY6E, IFI27, OAS1, IFIT1, IFI6, DNAPTP6; or LLY6E, IFI27, OAS1, IFIT1, IFI6, LOC129607; or LLY6E, IFI27, OAS1, IFIT1, IFI6, EPSTI1; or LLY6E, IFI27, OAS1, IFIT1, IFI6, BIRC4BP; or LLY6E, IFI27, OAS1, IFIT1, IFI6, gene detected by probe 229450_at; or LLY6E, IFI27, OAS1, IFIT1, IFI6, gene detected by probe 235276_at; or IFI27, OAS1, IFIT1, IFI6, IFI44L, ISG15; or IFI27, OAS1, IFIT1, IFI6, IFI44L, LAMP3; or IFI27, OAS1, IFIT1, IFI6, IFI44L, OASL; or IFI27, OAS1, IFIT1, IFI6, IFI44L, RSAD2; or IFI27, OAS1, IFIT1, IFI6, IFI44L, IFI44; or IFI27, OAS1, IFIT1, IFI6, IFI44L, IFIT2; or IFI27, OAS1, IFIT1, IFI6, IFI44L, OAS3; or IFI27, OAS1, IFIT1, IFI6, IFI44L, USP18; or IFI27, OAS1, IFIT1, IFI6, IFI44L, SIGLEC1; or IFI27, OAS1, IFIT1, IFI6, IFI44L, HERC5; or IFI27, OAS1, IFIT1, IFI6, IFI44L, DNAPTP6; or IFI27, OAS1, IFIT1, IFI6, IFI44L, LOC129607; or IFI27, OAS1, IFIT1, IFI6, IFI44L, EPSTI1; or IFI27, OAS1, IFIT1, IFI6, IFI44L, BIRC4BP; or IFI27, OAS1, IFIT1, IFI6, IFI44L, gene detected by probe 229450_at; or IFI27, OAS1, IFIT1, IFI6, IFI44L, gene detected by probe 235276_at; or OAS1, IFIT1, IFI6, IFI44L, ISG15, LAMP3; or OAS1, IFIT1, IFI6, IFI44L, ISG15, OASL; or OAS1, IFIT1, IFI6, IFI44L, ISG15, RSAD2; or OAS1, IFIT1, IFI6, IFI44L, ISG15, IFI44; or OAS1, IFIT1, IFI6, IFI44L, ISG15, IFIT2; or OAS1, IFIT1, IFI6, IFI44L, ISG15, OAS3; or OAS1, IFIT1, IFI6, IFI44L, ISG15, USP18; or OAS1, IFIT1, IFI6, IFI44L, ISG15, SIGLEC1; or OAS1, IFIT1, IFI6, IFI44L, ISG15, HERC5; or OAS1, IFIT1, IFI6, IFI44L, ISG15, DNAPTP6; or OAS1, IFIT1, IFI6, IFI44L, ISG15, LOC129607; or OAS1, IFIT1, IFI6, IFI44L, ISG15, EPSTI1; or OAS1, IFIT1, IFI6, IFI44L, ISG15, BIRC4BP; or OAS1, IFIT1, IFI6, IFI44L, ISG15, gene detected by probe 229450_at; or OAS1, IFIT1, IFI6, IFI44L, ISG15, gene detected by probe 235276_at; or IFIT1, IFI6, IFI44L, ISG15, LAMP3, OASL; or IFIT1, IFI6, IFI44L, ISG15, LAMP3, RSAD2; or IFIT1, IFI6, IFI44L, ISG15, LAMP3, IFI44; or IFIT1, IFI6, IFI44L, ISG15, LAMP3, IFIT2; or IFIT1, IFI6, IFI44L, ISG15, LAMP3, OAS3; or IFIT1, IFI6, IFI44L, ISG15, LAMP3, USP18; or IFIT1, IFI6, IFI44L, ISG15, LAMP3, SIGLEC1; or IFIT1, IFI6, IFI44L, ISG15, LAMP3, HERC5; or IFIT1, IFI6, IFI44L, ISG15, LAMP3, DNAPTP6; or IFIT1, IFI6, IFI44L, ISG15, LAMP3, LOC129607; or IFIT1, IFI6, IFI44L, ISG15, LAMP3, EPSTI1; or IFIT1, IFI6, IFI44L, ISG15, LAMP3, BIRC4BP; or IFIT1, IFI6, IFI44L, ISG15, LAMP3, gene detected by probe 229450_at; or IFIT1, IFI6, IFI44L, ISG15, LAMP3, gene detected by probe 235276_at; or IFI6, IFI44L, ISG15, LAMP3, OASL, RSAD2; or IFI6, IFI44L, ISG15, LAMP3, OASL, IFI44; or IFI6, IFI44L, ISG15, LAMP3, OASL, IFIT2; or IFI6, IFI44L, ISG15, LAMP3, OASL, OAS3; or IFI6, IFI44L, ISG15, LAMP3, OASL, USP18; or IFI6, IFI44L, ISG15, LAMP3, OASL, SIGLEC1; or IFI6, IFI44L, ISG15, LAMP3, OASL, HERC5; or IFI6, IFI44L, ISG15, LAMP3, OASL, DNAPTP6; or IFI6, IFI44L, ISG15, LAMP3, OASL, LOC129607; or IFI6, IFI44L, ISG15, LAMP3, OASL, EPSTI1; or IFI6, IFI44L, ISG15, LAMP3, OASL, BIRC4BP; or IFI6, IFI44L, ISG15, LAMP3, OASL, gene detected by probe 229450_at; or IFI6, IFI44L, ISG15, LAMP3, OASL, gene detected by probe 235276_at; or IFI44L, ISG15, LAMP3, OASL, RSAD2, IFI44; or IFI44L, ISG15, LAMP3, OASL, RSAD2, IFIT2; or IFI44L, ISG15, LAMP3, OASL, RSAD2, OAS3; or IFI44L, ISG15, LAMP3, OASL, RSAD2, USP18; or IFI44L, ISG15, LAMP3, OASL, RSAD2, SIGLEC1; or IFI44L, ISG15, LAMP3, OASL, RSAD2, HERC5; or IFI44L, ISG15, LAMP3, OASL, RSAD2, DNAPTP6; or IFI44L, ISG15, LAMP3, OASL, RSAD2, LOC129607; or IFI44L, ISG15, LAMP3, OASL, RSAD2, EPSTI1; or IFI44L, ISG15, LAMP3, OASL, RSAD2, BIRC4BP; or IFI44L, ISG15, LAMP3, OASL, RSAD2, gene detected by probe 229450_at; or IFI44L, ISG15, LAMP3, OASL, RSAD2, gene detected by probe 235276_at; or ISG15, LAMP3, OASL, RSAD2, IFI44, IFIT2; or ISG15, LAMP3, OASL, RSAD2, IFI44, OAS3; or ISG15, LAMP3, OASL, RSAD2, IFI44, USP18; or ISG15, LAMP3, OASL, RSAD2, IFI44, SIGLEC1; or ISG15, LAMP3, OASL, RSAD2, IFI44, HERC5; or ISG15, LAMP3, OASL, RSAD2, IFI44, DNAPTP6; or ISG15, LAMP3, OASL, RSAD2, IFI44, LOC129607; or ISG15, LAMP3, OASL, RSAD2, IFI44, EPSTI1; or ISG15, LAMP3, OASL, RSAD2, IFI44, BIRC4BP; or ISG15, LAMP3, OASL, RSAD2, IFI44, gene detected by probe 229450_at; or ISG15, LAMP3, OASL, RSAD2, IFI44, gene detected by probe 235276_at; or LAMP3, OASL, RSAD2, IFI44, IFIT2, OAS3; or LAMP3, OASL, RSAD2, IFI44, IFIT2, USP18; or LAMP3, OASL, RSAD2, IFI44, IFIT2, SIGLEC1; or LAMP3, OASL, RSAD2, IFI44, IFIT2, HERC5; or LAMP3, OASL, RSAD2, IFI44, IFIT2, DNAPTP6; or LAMP3, OASL, RSAD2, IFI44, IFIT2, LOC129607; or LAMP3, OASL, RSAD2, IFI44, IFIT2, EPSTI1; or LAMP3, OASL, RSAD2, IFI44, IFIT2, BIRC4BP; or LAMP3, OASL, RSAD2, IFI44, IFIT2, gene detected by probe 229450_at; or LAMP3, OASL, RSAD2, IFI44, IFIT2, gene detected by probe 235276_at; or OASL, RSAD2, IFI44, IFIT2, OAS3, USP18; or OASL, RSAD2, IFI44, IFIT2, OAS3, SIGLEC1; or OASL, RSAD2, IFI44, IFIT2, OAS3, HERC5; or OASL, RSAD2, IFI44, IFIT2, OAS3, DNAPTP6; or OASL, RSAD2, IFI44, IFIT2, OAS3, LOC129607; or OASL, RSAD2, IFI44, IFIT2, OAS3, EPSTI1; or OASL, RSAD2, IFI44, IFIT2, OAS3, BIRC4BP; or OASL, RSAD2, IFI44, IFIT2, OAS3, gene detected by probe 229450_at; or OASL, RSAD2, IFI44, IFIT2, OAS3, gene detected by probe 235276_at; or RSAD2, IFI44, IFIT2, OAS3, USP18, SIGLEC1; or RSAD2, IFI44, IFIT2, OAS3, USP18, HERC5; or RSAD2, IFI44, IFIT2, OAS3, USP18, DNAPTP6; or RSAD2, IFI44, IFIT2, OAS3, USP18, LOC129607; or RSAD2, IFI44, IFIT2, OAS3, USP18, EPSTI1; or RSAD2, IFI44, IFIT2, OAS3, USP18, BIRC4BP; or RSAD2, IFI44, IFIT2, OAS3, USP18, gene detected by probe 229450_at; or RSAD2, IFI44, IFIT2, OAS3, USP18, gene detected by probe 235276_at; or IFI44, IFIT2, OAS3, USP18, SIGLEC1, HERC5; or IFI44, IFIT2, OAS3, USP18, SIGLEC1, DNAPTP6; or IFI44, IFIT2, OAS3, USP18, SIGLEC1, LOC129607; or IFI44, IFIT2, OAS3, USP18, SIGLEC1, EPSTI1; or IFI44, IFIT2, OAS3, USP18, SIGLEC1, BIRC4BP; or IFI44, IFIT2, OAS3, USP18, SIGLEC1, gene detected by probe 229450_at; or IFI44, IFIT2, OAS3, USP18, SIGLEC1, gene detected by probe 235276_at; or IFIT2, OAS3, USP18, SIGLEC1, HERC5, DNAPTP6; or IFIT2, OAS3, USP18, SIGLEC1, HERC5, LOC129607; or IFIT2, OAS3, USP18, SIGLEC1, HERC5, EPSTI1; or IFIT2, OAS3, USP18, SIGLEC1, HERC5, BIRC4BP; or IFIT2, OAS3, USP18, SIGLEC1, HERC5, gene detected by probe 229450_at; or IFIT2, OAS3, USP18, SIGLEC1, HERC5, gene detected by probe 235276_at; or OAS3, USP18, SIGLEC1, HERC5, DNAPTP6, LOC129607; or OAS3, USP18, SIGLEC1, HERC5, DNAPTP6, EPSTI1; or OAS3, USP18, SIGLEC1, HERC5, DNAPTP6, BIRC4BP; or OAS3, USP18, SIGLEC1, HERC5, DNAPTP6, gene detected by probe 229450_at; or OAS3, USP18, SIGLEC1, HERC5, DNAPTP6, gene detected by probe 235276_at; or USP18, SIGLEC1, HERC5, DNAPTP6, LOC129607, EPSTI1; or USP18, SIGLEC1, HERC5, DNAPTP6, LOC129607, BIRC4BP; or USP18, SIGLEC1, HERC5, DNAPTP6, LOC129607, gene detected by probe 229450_at; or USP18, SIGLEC1, HERC5, DNAPTP6, LOC129607, gene detected by probe 235276_at; or SIGLEC1, HERC5, DNAPTP6, LOC129607, EPSTI1, BIRC4BP; or SIGLEC1, HERC5, DNAPTP6, LOC129607, EPSTI1, gene detected by probe 229450_at; or SIGLEC1, HERC5, DNAPTP6, LOC129607, EPSTI1, gene detected by probe 235276_at ; or HERC5, DNAPTP6, LOC129607, EPSTI1, BIRC4BP, gene detected by probe 229450_at; or HERC5, DNAPTP6, LOC129607, EPSTI1, BIRC4BP, gene detected by probe 235276_at; or DNAPTP6, LOC129607, EPSTI1, BIRC4BP, gene detected by probe 229450_at, gene detected by probe 235276_at. The IFNα-inducible PD markers in such an expression profile may further include at least one or more gene listed in drawing sheets 235-381.

The IFNα-inducible PD markers in an expression profile may include any at least 7 genes such as, for example: MX1, LLY6E, IFI27, OAS1, IFIT1, IFI6, IFI44L; or MX1, LLY6E, IFI27, OAS1, IFIT1, IFI6, ISG15; or MX1, LLY6E, IFI27, OAS1, IFIT1, IFI6, LAMP3; or MX1, LLY6E, IFI27, OAS1, IFIT1, IFI6, OASL; or MX1, LLY6E, IFI27, OAS1, IFIT1, IFI6, RSAD2; or MX1, LLY6E, IFI27, OAS1, IFIT1, IFI6, IFI44; or MX1, LLY6E, IFI27, OAS1, IFIT1, IFI6, IFIT2; or MX1, LLY6E, IFI27, OAS1, IFIT1, IFI6, OAS3; or MX1, LLY6E, IFI27, OAS1, IFIT1, IFI6, USP18; or MX1, LLY6E, IFI27, OAS1, IFIT1, IFI6, SIGLEC1; or MX1, LLY6E, IFI27, OAS1, IFIT1, IFI6, HERC5; or MX1, LLY6E, IFI27, OAS1, IFIT1, IFI6, DNAPTP6; or MX1, LLY6E, IFI27, OAS1, IFIT1, IFI6, LOC129607; or MX1, LLY6E, IFI27, OAS1, IFIT1, IFI6, EPSTI1; or MX1, LLY6E, IFI27, OAS1, IFIT1, IFI6, BIRC4BP; or MX1, LLY6E, IFI27, OAS1, IFIT1, IFI6, gene detected by probe 229450_at; or MX1, LLY6E, IFI27, OAS1, IFIT1, IFI6, gene detected by probe 235276_at; or LLY6E, IFI27, OAS1, IFIT1, IFI6, IFI44L, ISG15; or LLY6E, IFI27, OAS1, IFIT1, IFI6, IFI44L, LAMP3; or LLY6E, IFI27, OAS1, IFIT1, IFI6, IFI44L, OASL; or LLY6E, IFI27, OAS1, IFIT1, IFI6, IFI44L, RSAD2; or LLY6E, IFI27, OAS1, IFIT1, IFI6, IFI44L, IFI44; or LLY6E, IFI27, OAS1, IFIT1, IFI6, IFI44L, IFIT2; or LLY6E, IFI27, OAS1, IFIT1, IFI6, IFI44L, OAS3; or LLY6E, IFI27, OAS1, IFIT1, IFI6, IFI44L, USP18; or LLY6E, IFI27, OAS1, IFIT1, IFI6, IFI44L, SIGLEC1; or LLY6E, IFI27, OAS1, IFIT1, IFI6, IFI44L, HERC5; or LLY6E, IFI27, OAS1, IFIT1, IFI6, IFI44L, DNAPTP6; or LLY6E, IFI27, OAS1, IFIT1, IFI6, IFI44L, LOC129607; or LLY6E, IFI27, OAS1, IFIT1, IFI6, IFI44L, EPSTI1; or LLY6E, IFI27, OAS1, IFIT1, IFI6, IFI44L, BIRC4BP; or LLY6E, IFI27, OAS1, IFIT1, IFI6, IFI44L, gene detected by probe 229450_at; or LLY6E, IFI27, OAS1, IFIT1, IFI6, IFI44L, gene detected by probe 235276_at; or IFI27, OAS1, IFIT1, IFI6, IFI44L, ISG15, LAMP3; or IFI27, OAS1, IFIT1, IFI6, IFI44L, ISG15, OASL; or IFI27, OAS1, IFIT1, IFI6, IFI44L, ISG15, RSAD2; or IFI27, OAS1, IFIT1, IFI6, IFI44L, ISG15, IFI44; or IFI27, OAS1, IFIT1, IFI6, IFI44L, ISG15, IFIT2; or IFI27, OAS1, IFIT1, IFI6, IFI44L, ISG15, OAS3; or IFI27, OAS1, IFIT1, IFI6, IFI44L, ISG15, USP18; or IFI27, OAS1, IFIT1, IFI6, IFI44L, ISG15, SIGLEC1; or IFI27, OAS1, IFIT1, IFI6, IFI44L, ISG15, HERC5; or IFI27, OAS1, IFIT1, IFI6, IFI44L, ISG15, DNAPTP6; or IFI27, OAS1, IFIT1, IFI6, IFI44L, ISG15, LOC129607; or IFI27, OAS1, IFIT1, IFI6, IFI44L, ISG15, EPSTI1; or IFI27, OAS1, IFIT1, IFI6, IFI44L, ISG15, BIRC4BP; or IFI27, OAS1, IFIT1, IFI6, IFI44L, ISG15, gene detected by probe 229450_at; or IFI27, OAS1, IFIT1, IFI6, IFI44L, ISG15, gene detected by probe 235276_at; or OAS1, IFIT1, IFI6, IFI44L, ISG15, LAMP3, OASL; or OAS1, IFIT1, IFI6, IFI44L, ISG15, LAMP3, RSAD2; or OAS1, IFIT1, IFI6, IFI44L, ISG15, LAMP3, IFI44; or OAS1, IFIT1, IFI6, IFI44L, ISG 15, LAMP3, IFIT2; or OAS1, IFIT1, IFI6, IFI44L, ISG 15, LAMP3, OAS3; or OAS1, IFIT1, IFI6, IFI44L, ISG15, LAMP3, USP18; or OAS1, IFIT1, IFI6, IFI44L, ISG15, LAMP3, SIGLEC1; or OAS1, IFIT1, IFI6, IFI44L, ISG15, LAMP3, HERC5; or OAS1, IFIT1, IFI6, IFI44L, ISG15, LAMP3, DNAPTP6; or OAS1, IFIT1, IFI6, IFI44L, ISG15, LAMP3, LOC129607; or OAS1, IFIT1, IFI6, IFI44L, ISG15, LAMP3, EPSTI1; or OAS1, IFIT1, IFI6, IFI44L, ISG15, LAMP3, BIRC4BP; or OAS1, IFIT1, IFI6, IFI44L, ISG15, LAMP3, gene detected by probe 229450_at; or OAS1, IFIT1, IFI6, IFI44L, ISG15, LAMP3, gene detected by probe 235276_at; or IFIT1, IFI6, IFI44L, ISG15, LAMP3, OASL, RSAD2; or IFIT1, IFI6, IFI44L, ISG15, LAMP3, OASL, IFI44; or IFIT1, IFI6, IFI44L, ISG15, LAMP3, OASL, IFIT2; or IFIT1, IFI6, IFI44L, ISG15, LAMP3, OASL, OAS3; or IFIT1, IFI6, IFI44L, ISG15, LAMP3, OASL, USP18; or IFIT1, IFI6, IFI44L, ISG15, LAMP3, OASL, SIGLEC1; or IFIT1, IFI6, IFI44L, ISG15, LAMP3, OASL, HERC5; or IFIT1, IFI6, IFI44L, ISG15, LAMP3, OASL, DNAPTP6; or IFIT1, IFI6, IFI44L, ISG15, LAMP3, OASL, LOC129607; or IFIT1, IFI6, IFI44L, ISG15, LAMP3, OASL, EPSTI1; or IFIT1, IFI6, IFI44L, ISG15, LAMP3, OASL, BIRC4BP; or IFIT1, IFI6, IFI44L, ISG15, LAMP3, OASL, gene detected by probe 229450_at; or IFIT1, IFI6, IFI44L, ISG15, LAMP3, OASL, gene detected by probe 235276_at; or IFI6, IFI44L, ISG15, LAMP3, OASL, RSAD2, IFI44; or IFI6, IFI44L, ISG15, LAMP3, OASL, RSAD2, IFIT2; or IFI6, IFI44L, ISG15, LAMP3, OASL, RSAD2, OAS3; or IFI6, IFI44L, ISG15, LAMP3, OASL, RSAD2, USP18; or IFI6, IFI44L, ISG15, LAMP3, OASL, RSAD2, SIGLEC1; or IFI6, IFI44L, ISG15, LAMP3, OASL, RSAD2, HERC5; or IFI6, IFI44L, ISG15, LAMP3, OASL, RSAD2, DNAPTP6; or IFI6, IFI44L, ISG15, LAMP3, OASL, RSAD2, LOC129607; or IFI6, IFI44L, ISG15, LAMP3, OASL, RSAD2, EPSTI1; or IFI6, IFI44L, ISG15, LAMP3, OASL, RSAD2, BIRC4BP; or IFI6, IFI44L, ISG15, LAMP3, OASL, RSAD2, gene detected by probe 229450_at; or IFI6, IFI44L, ISG15, LAMP3, OASL, RSAD2, gene detected by probe 235276_at; or IFI44L, ISG15, LAMP3, OASL, RSAD2, IFI44, IFIT2; or IFI44L, ISG15, LAMP3, OASL, RSAD2, IFI44, OAS3; or IFI44L, ISG15, LAMP3, OASL, RSAD2, IFI44, USP18; or IFI44L, ISG15, LAMP3, OASL, RSAD2, IFI44, SIGLEC1; or IFI44L, ISG15, LAMP3, OASL, RSAD2, IFI44, HERC5; or IFI44L, ISG15, LAMP3, OASL, RSAD2, IFI44, DNAPTP6; or IFI44L, ISG15, LAMP3, OASL, RSAD2, IFI44, LOC129607; or IFI44L, ISG15, LAMP3, OASL, RSAD2, IFI44, EPSTI1; or IFI44L, ISG15, LAMP3, OASL, RSAD2, IFI44, BIRC4BP; or IFI44L, ISG15, LAMP3, OASL, RSAD2, IFI44, gene detected by probe 229450_at; or IFI44L, ISG15, LAMP3, OASL, RSAD2, IFI44, gene detected by probe 235276_at; or ISG15, LAMP3, OASL, RSAD2, IFI44, IFIT2, OAS3; or ISG15, LAMP3, OASL, RSAD2, IFI44, IFIT2, USP18; or ISG15, LAMP3, OASL, RSAD2, IFI44, IFIT2, SIGLEC1; or ISG15, LAMP3, OASL, RSAD2, IFI44, IFIT2, HERC5; or ISG15, LAMP3, OASL, RSAD2, IFI44, IFIT2, DNAPTP6; or ISG15, LAMP3, OASL, RSAD2, IFI44, IFIT2, LOC129607; or ISG15, LAMP3, OASL, RSAD2, IFI44, IFIT2, EPSTI1; or ISG15, LAMP3, OASL, RSAD2, IFI44, IFIT2, BIRC4BP; or ISG15, LAMP3, OASL, RSAD2, IFI44, IFIT2, gene detected by probe 229450_at; or ISG15, LAMP3, OASL, RSAD2, IFI44, IFIT2, gene detected by probe 235276_at; or LAMP3, OASL, RSAD2, IFI44, IFIT2, OAS3, USP18; or LAMP3, OASL, RSAD2, IFI44, IFIT2, OAS3, SIGLEC1; or LAMP3, OASL, RSAD2, IFI44, IFIT2, OAS3, HERC5; or LAMP3, OASL, RSAD2, IFI44, IFIT2, OAS3, DNAPTP6; or LAMP3, OASL, RSAD2, IFI44, IFIT2, OAS3, LOC129607; or LAMP3, OASL, RSAD2, IFI44, IFIT2, OAS3, EPSTI1; or LAMP3, OASL, RSAD2, IFI44, IFIT2, OAS3, BIRC4BP; or LAMP3, OASL, RSAD2, IFI44, IFIT2, OAS3, gene detected by probe 229450_at; or LAMP3, OASL, RSAD2, IFI44, IFIT2, OAS3, gene detected by probe 235276_at; or OASL, RSAD2, IFI44, IFIT2, OAS3, USP18, SIGLEC1; or OASL, RSAD2, IFI44, IFIT2, OAS3, USP18, HERC5; or OASL, RSAD2, IFI44, IFIT2, OAS3, USP18, DNAPTP6; or OASL, RSAD2, IFI44, IFIT2, OAS3, USP18, LOC129607; or OASL, RSAD2, IFI44, IFIT2, OAS3, USP18, EPSTI1; or OASL, RSAD2, IFI44, IFIT2, OAS3, USP18, BIRC4BP; or OASL, RSAD2, IFI44, IFIT2, OAS3, USP18, gene detected by probe 229450_at; or OASL, RSAD2, IFI44, IFIT2, OAS3, USP18, gene detected by probe 235276_at; or RSAD2, IFI44, IFIT2, OAS3, USP18, SIGLEC1, HERC5; or RSAD2, IFI44, IFIT2, OAS3, USP18, SIGLEC1, DNAPTP6; or RSAD2, IFI44, IFIT2, OAS3, USP18, SIGLEC1, LOC129607; or RSAD2, IFI44, IFIT2, OAS3, USP18, SIGLEC1, EPSTI1; or RSAD2, IFI44, IFIT2, OAS3, USP18, SIGLEC1, BIRC4BP; or RSAD2, IFI44, IFIT2, OAS3, USP18, SIGLEC1, gene detected by probe 229450_at; or RSAD2, IFI44, IFIT2, OAS3, USP18, SIGLEC1, gene detected by probe 235276_at; or IFI44, IFIT2, OAS3, USP18, SIGLEC1, HERC5, DNAPTP6; or IFI44, IFIT2, OAS3, USP18, SIGLEC1, HERC5, LOC129607; or IFI44, IFIT2, OAS3, USP18, SIGLEC1, HERC5, EPSTI1; or IFI44, IFIT2, OAS3, USP18, SIGLEC1, HERC5, BIRC4BP; or IFI44, IFIT2, OAS3, USP18, SIGLEC1, HERC5, gene detected by probe 229450_at; or IFI44, IFIT2, OAS3, USP18, SIGLEC1, HERC5, gene detected by probe 235276_at; or IFIT2, OAS3, USP18, SIGLEC1, HERC5, DNAPTP6, LOC129607; or IFIT2, OAS3, USP18, SIGLEC1, HERC5, DNAPTP6, EPSTI1; or IFIT2, OAS3, USP18, SIGLEC1, HERC5, DNAPTP6, BIRC4BP; or IFIT2, OAS3, USP 18, SIGLEC1, HERC5, DNAPTP6, gene detected by probe 229450_at ; or IFIT2, OAS3, USP18, SIGLEC1, HERC5, DNAPTP6, gene detected by probe 235276_at; or OAS3, USP18, SIGLEC1, HERC5, DNAPTP6, LOC129607, EPSTI1; or OAS3, USP18, SIGLEC1, HERC5, DNAPTP6, LOC129607, BIRC4BP; or OAS3, USP18, SIGLEC1, HERC5, DNAPTP6, LOC129607, gene detected by probe 229450_at; or OAS3, USP18, SIGLEC1, HERC5, DNAPTP6, LOC129607, gene detected by probe 235276_at; or USP18, SIGLEC1, HERC5, DNAPTP6, LOC129607, EPSTI1, BIRC4BP; or USP18, SIGLEC1, HERC5, DNAPTP6, LOC129607, EPSTI1, gene detected by probe 229450_at; or USP18, SIGLEC1, HERC5, DNAPTP6, LOC129607, EPSTI1, gene detected by probe 235276_at; or SIGLEC1, HERC5, DNAPTP6, LOC129607, EPSTI1, BIRC4BP, gene detected by probe 229450_at; or SIGLEC1, HERC5, DNAPTP6, LOC129607, EPSTI1, BIRC4BP, gene detected by probe 235276_at; or HERC5, DNAPTP6, LOC129607, EPSTI1, BIRC4BP, gene detected by probe 229450_at, gene detected by probe 235276_at. The IFNα-inducible PD markers in such an expression profile may further include at least one or more gene listed in drawing sheets 235-381.

The IFNα-inducible PD markers in an expression profile may include any at least 8 genes such as, for example: MX1, LLY6E, IFI27, OAS1, IFIT1, IFI6, IFI44L, ISG15; or MX1, LLY6E, IFI27, OAS1, IFIT1, IFI6, IFI44L, LAMP3; or MX1, LLY6E, IFI27, OAS1, IFIT1, IFI6, IFI44L, OASL; or MX1, LLY6E, IFI27, OAS1, IFIT1, IFI6, IFI44L, RSAD2; or MX1, LLY6E, IFI27, OAS1, IFIT1, IFI6, IFI44L, IFI44; or MX1, LLY6E, IFI27, OAS1, IFIT1, IFI6, IFI44L, IFIT2; or MX1, LLY6E, IFI27, OAS1, IFIT1, IFI6, IFI44L, OAS3; or MX1, LLY6E, IFI27, OAS1, IFIT1, IFI6, IFI44L, USP18; or MX1, LLY6E, IFI27, OAS1, IFIT1, IFI6, IFI44L, SIGLEC1; or MX1, LLY6E, IFI27, OAS1, IFIT1, IFI6, IFI44L, HERC5; or MX1, LLY6E, IFI27, OAS1, IFIT1, IFI6, IFI44L, DNAPTP6; or MX1, LLY6E, IFI27, OAS1, IFIT1, IFI6, IFI44L, LOC129607; or MX1, LLY6E, IFI27, OAS1, IFIT1, IFI6, IFI44L, EPSTI1; or MX1, LLY6E, IFI27, OAS1, IFIT1, IFI6, IFI44L, BIRC4BP; or MX1, LLY6E, IFI27, OAS1, IFIT1, IFI6, IFI44L, gene detected by probe 229450_at; or MX1, LLY6E, IFI27, OAS1, IFIT1, IFI6, IFI44L, gene detected by probe 235276_at; or LLY6E, IFI27, OAS1, IFIT1, IFI6, IFI44L, ISG15, LAMP3; or LLY6E, IFI27, OAS1, IFIT1, IFI6, IFI44L, ISG15, OASL; or LLY6E, IFI27, OAS1, IFIT1, IFI6, IFI44L, ISG15, RSAD2; or LLY6E, IFI27, OAS1, IFIT1, IFI6, IFI44L, ISG15, IFI44; or LLY6E, IFI27, OAS1, IFIT1, IFI6, IFI44L, ISG15, IFIT2; or LLY6E, IFI27, OAS1, IFIT1, IFI6, IFI44L, ISG15, OAS3; or LLY6E, IFI27, OAS1, IFIT1, IFI6, IFI44L, ISG15, USP18; or LLY6E, IFI27, OAS1, IFIT1, IFI6, IFI44L, ISG15, SIGLEC1; or LLY6E, IFI27, OAS1, IFIT1, IFI6, IFI44L, ISG15, HERC5; or LLY6E, IFI27, OAS1, IFIT1, IFI6, IFI44L, ISG15, DNAPTP6; or LLY6E, IFI27, OAS1, IFIT1, IFI6, IFI44L, ISG15, LOC129607; or LLY6E, IFI27, OAS1, IFIT1, IFI6, IFI44L, ISG15, EPSTI1; or LLY6E, IFI27, OAS1, IFIT1, IFI6, IFI44L, ISG15, BIRC4BP; or LLY6E, IFI27, OAS1, IFIT1, IFI6, IFI44L, ISG15, gene detected by probe 229450_at; or LLY6E, IFI27, OAS1, IFIT1, IFI6, IFI44L, ISG15, gene detected by probe 235276_at; orIFI27, OAS1, IFIT1, IFI6, IFI44L, ISG15, LAMP3, OASL; or IFI27, OAS1, IFIT1, IFI6, IFI44L, ISG15, LAMP3, RSAD2; or IFI27, OAS1, IFIT1, IFI6, IFI44L, ISG15, LAMP3, IFI44; or IFI27, OAS1, IFIT1, IFI6, IFI44L, ISG15, LAMP3, IFIT2; or IFI27, OAS1, IFIT1, IFI6, IFI44L, ISG15, LAMP3, OAS3; or IFI27, OAS1, IFIT1, IFI6, IFI44L, ISG15, LAMP3, USP18; or IFI27, OAS1, IFIT1, IFI6, IFI44L, ISG15, LAMP3, SIGLEC1; or IFI27, OAS1, IFIT1, IFI6, IFI44L, ISG15, LAMP3, HERC5; or IFI27, OAS1, IFIT1, IFI6, IFI44L, ISG15, LAMP3, DNAPTP6; or IFI27, OAS1, IFIT1, IFI6, IFI44L, ISG15, LAMP3, LOC129607; or IFI27, OAS1, IFIT1, IFI6, IFI44L, ISG15, LAMP3, EPSTI1; or IFI27, OAS1, IFIT1, IFI6, IFI44L, ISG15, LAMP3, gene detected by probe 229450_at; or IFI27, OAS1, IFIT1, IFI6, IFI44L, ISG15, LAMP3, BIRC4BP; or IFI27, OAS1, IFIT1, IFI6, IFI44L, ISG15, LAMP3, gene detected by probe 235276_at; or OAS1, IFIT1, IFI6, IFI44L, ISG15, LAMP3, OASL, RSAD2; or OAS1, IFIT1, IFI6, IFI44L, ISG15, LAMP3, OASL, IFI44; or OAS1, IFIT1, IFI6, IFI44L, ISG15, LAMP3, OASL, IFIT2; or OAS1, IFIT1, IFI6, IFI44L, ISG15, LAMP3, OASL, OAS3; or OAS1, IFIT1, IFI6, IFI44L, ISG15, LAMP3, OASL, USP18; or OAS1, IFIT1, IFI6, IFI44L, ISG15, LAMP3, OASL, SIGLEC1; or OAS1, IFIT1, IFI6, IFI44L, ISG15, LAMP3, OASL, HERC5; or OAS1, IFIT1, IFI6, IFI44L, ISG15, LAMP3, OASL, DNAPTP6; or OAS1, IFIT1, IFI6, IFI44L, ISG15, LAMP3, OASL, LOC129607; or OAS1, IFIT1, IFI6, IFI44L, ISG15, LAMP3, OASL, EPSTI1; or OAS1, IFIT1, IFI6, IFI44L, ISG15, LAMP3, OASL, BIRC4BP; or OAS1, IFIT1, IFI6, IFI44L, ISG15, LAMP3, OASL, gene detected by probe 229450_at; or OAS1, IFIT1, IFI6, IFI44L, ISG15, LAMP3, OASL, gene detected by probe 235276_at; or IFIT1, IFI6, IFI44L, ISG15, LAMP3, OASL, RSAD2, IFI44; or IFIT1, IFI6, IFI44L, ISG15, LAMP3, OASL, RSAD2, IFIT2; or IFIT1, IFI6, IFI44L, ISG15, LAMP3, OASL, RSAD2, OAS3; or IFIT1, IFI6, IFI44L, ISG15, LAMP3, OASL, RSAD2, USP18; or IFIT1, IFI6, IFI44L, ISG15, LAMP3, OASL, RSAD2, SIGLEC1; or IFIT1, IFI6, IFI44L, ISG15, LAMP3, OASL, RSAD2, HERC5; or IFIT1, IFI6, IFI44L, ISG15, LAMP3, OASL, RSAD2, DNAPTP6; or IFIT1, IFI6, IFI44L, ISG15, LAMP3, OASL, RSAD2, LOC129607; or IFIT1, IFI6, IFI44L, ISG15, LAMP3, OASL, RSAD2, EPSTI1; or IFIT1, IFI6, IFI44L, ISG15, LAMP3, OASL, RSAD2, BIRC4BP; or IFIT1, IFI6, IFI44L, ISG15, LAMP3, OASL, RSAD2, gene detected by probe 229450_at; or IFIT1, IFI6, IFI44L, ISG15, LAMP3, OASL, RSAD2, gene detected by probe 235276_at; or IFI6, IFI44L, ISG15, LAMP3, OASL, RSAD2, IFI44, IFIT2; or IFI6, IFI44L, ISG15, LAMP3, OASL, RSAD2, IFI44, OAS3; or IFI6, IFI44L, ISG15, LAMP3, OASL, RSAD2, IFI44, USP18; or IFI6, IFI44L, ISG15, LAMP3, OASL, RSAD2, IFI44, SIGLEC1; or IFI6, IFI44L, ISG15, LAMP3, OASL, RSAD2, IFI44, HERC5; or IFI6, IFI44L, ISG15, LAMP3, OASL, RSAD2, IFI44, DNAPTP6; or IFI6, IFI44L, ISG15, LAMP3, OASL, RSAD2, IFI44, LOC129607; or IFI6, IFI44L, ISG15, LAMP3, OASL, RSAD2, IFI44, EPSTI1; or IFI6, IFI44L, ISG15, LAMP3, OASL, RSAD2, IFI44, BIRC4BP; or IFI6, IFI44L, ISG15, LAMP3, OASL, RSAD2, IFI44, gene detected by probe 229450_at; or IFI6, IFI44L, ISG15, LAMP3, OASL, RSAD2, IFI44, gene detected by probe 235276_at; or IFI44L, ISG15, LAMP3, OASL, RSAD2, IFI44, IFIT2, OAS3; or IFI44L, ISG15, LAMP3, OASL, RSAD2, IFI44, IFIT2, USP18; or IFI44L, ISG15, LAMP3, OASL, RSAD2, IFI44, IFIT2, SIGLEC1; or IFI44L, ISG15, LAMP3, OASL, RSAD2, IFI44, IFIT2, HERC5; or IFI44L, ISG15, LAMP3, OASL, RSAD2, IFI44, IFIT2, DNAPTP6; or IFI44L, ISG15, LAMP3, OASL, RSAD2, IFI44, IFIT2, LOC129607; or IFI44L, ISG15, LAMP3, OASL, RSAD2, IFI44, IFIT2, EPSTI1; or IFI44L, ISG15, LAMP3, OASL, RSAD2, IFI44, IFIT2, BIRC4BP; or IFI44L, ISG15, LAMP3, OASL, RSAD2, IFI44, IFIT2, gene detected by probe 229450_at; or IFI44L, ISG15, LAMP3, OASL, RSAD2, IFI44, IFIT2, gene detected by probe 235276_at; or ISG15, LAMP3, OASL, RSAD2, IFI44, IFIT2, OAS3, USP18; or ISG15, LAMP3, OASL, RSAD2, IFI44, IFIT2, OAS3, SIGLEC1; or ISG15, LAMP3, OASL, RSAD2, IFI44, IFIT2, OAS3, HERC5; or ISG15, LAMP3, OASL, RSAD2, IFI44, IFIT2, OAS3, DNAPTP6; or ISG15, LAMP3, OASL, RSAD2, IFI44, IFIT2, OAS3, LOC129607; or ISG15, LAMP3, OASL, RSAD2, IFI44, IFIT2, OAS3, EPSTI1; or ISG15, LAMP3, OASL, RSAD2, IFI44, IFIT2, OAS3, BIRC4BP; or ISG15, LAMP3, OASL, RSAD2, IFI44, IFIT2, OAS3, gene detected by probe 229450_at; or ISG15, LAMP3, OASL, RSAD2, IFI44, IFIT2, OAS3, gene detected by probe 235276_at; or LAMP3, OASL, RSAD2, IFI44, IFIT2, OAS3, USP18, SIGLEC1; or LAMP3, OASL, RSAD2, IFI44, IFIT2, OAS3, USP18, HERC5; or LAMP3, OASL, RSAD2, IFI44, IFIT2, OAS3, USP18, DNAPTP6; or LAMP3, OASL, RSAD2, IFI44, IFIT2, OAS3, USP18, LOC129607; or LAMP3, OASL, RSAD2, IFI44, IFIT2, OAS3, USP18, EPSTI1; or LAMP3, OASL, RSAD2, IFI44, IFIT2, OAS3, USP18, BIRC4BP; or LAMP3, OASL, RSAD2, IFI44, IFIT2, OAS3, USP18, gene detected by probe 229450_at; or LAMP3, OASL, RSAD2, IFI44, IFIT2, OAS3, USP18, gene detected by probe 235276_at; or OASL, RSAD2, IFI44, IFIT2, OAS3, USP18, SIGLEC1, HERC5; or OASL, RSAD2, IFI44, IFIT2, OAS3, USP18, SIGLEC1, DNAPTP6; or OASL, RSAD2, IFI44, IFIT2, OAS3, USP18, SIGLEC1, LOC129607; or OASL, RSAD2, IFI44, IFIT2, OAS3, USP18, SIGLEC1, EPSTI1; or OASL, RSAD2, IFI44, IFIT2, OAS3, USP18, SIGLEC1, BIRC4BP; or OASL, RSAD2, IFI44, IFIT2, OAS3, USP18, SIGLEC1, gene detected by probe 229450_at; or OASL, RSAD2, IFI44, IFIT2, OAS3, USP18, SIGLEC1, gene detected by probe 235276_at; or RSAD2, IFI44, IFIT2, OAS3, USP18, SIGLEC1, HERC5, DNAPTP6; or RSAD2, IFI44, IFIT2, OAS3, USP18, SIGLEC1, HERC5, LOC129607; or RSAD2, IFI44, IFIT2, OAS3, USP18, SIGLEC1, HERC5, EPSTI1; or RSAD2, IFI44, IFIT2, OAS3, USP18, SIGLEC1, HERC5, gene detected by probe 229450_at; or RSAD2, IFI44, IFIT2, OAS3, USP18, SIGLEC1, HERC5, BIRC4BP; or RSAD2, IFI44, IFIT2, OAS3, USP18, SIGLEC1, HERC5, gene detected by probe 235276_at; or IFI44, IFIT2, OAS3, USP18, SIGLEC1, HERC5, DNAPTP6, LOC129607; or IFI44, IFIT2, OAS3, USP18, SIGLEC1, HERC5, DNAPTP6, EPSTI1; or IFI44, IFIT2, OAS3, USP18, SIGLEC1, HERC5, DNAPTP6, BIRC4BP; or IFI44, IFIT2, OAS3, USP18, SIGLEC1, HERC5, DNAPTP6, gene detected by probe 229450_at; or IFI44, IFIT2, OAS3, USP18, SIGLEC1, HERC5, DNAPTP6, gene detected by probe 235276_at; or IFIT2, OAS3, USP18, SIGLEC1, HERC5, DNAPTP6, LOC129607, EPSTI1; or IFIT2, OAS3, USP18, SIGLEC1, HERC5, DNAPTP6, LOC129607, BIRC4BP; or IFIT2, OAS3, USP18, SIGLEC1, HERC5, DNAPTP6, LOC129607, gene detected by probe 229450_at; or IFIT2, OAS3, USP18, SIGLEC1, HERC5, DNAPTP6, LOC129607, gene detected by probe 235276_at; or OAS3, USP18, SIGLEC1, HERC5, DNAPTP6, LOC129607, EPSTI1, BIRC4BP; or OAS3, USP18, SIGLEC1, HERC5, DNAPTP6, LOC129607, EPSTI1, gene detected by probe 229450_at; or OAS3, USP18, SIGLEC1, HERC5, DNAPTP6, LOC129607, EPSTI1, gene detected by probe 235276_at; or USP18, SIGLEC1, HERC5, DNAPTP6, LOC129607, EPSTI1, BIRC4BP, gene detected by probe 229450_at; or USP18, SIGLEC1, HERC5, DNAPTP6, LOC129607, EPSTI1, BIRC4BP, gene detected by probe 235276_at; or SIGLEC1, HERC5, DNAPTP6, LOC129607, EPSTI1, BIRC4BP, gene detected by probe 229450_at, gene detected by probe 235276_at. The IFNα-inducible PD markers in such an expression profile may further include at least one or more gene listed in drawing sheets 235-381.

The IFNα-inducible PD markers in an expression profile may include any at least 12 genes such as, for example: MX1, LLY6E, IFI27, OAS1, IFIT1, IFI6, IFI44L, ISG15, LAMP3, OASL, RSAD2, IFI44; or MX1, LLY6E, IFI27, OAS1, IFIT1, IFI6, IFI44L, ISG15, LAMP3, OASL, RSAD2, IFIT2; or MX1, LLY6E, IFI27, OAS1, IFIT1, IFI6, IFI44L, ISG15, LAMP3, OASL, RSAD2, OAS3; or MX1, LLY6E, IFI27, OAS1, IFIT1, IFI6, IFI44L, ISG15, LAMP3, OASL, RSAD2, USP18; or MX1, LLY6E, IFI27, OAS1, IFIT1, IFI6, IFI44L, ISG15, LAMP3, OASL, RSAD2, SIGLEC1; or MX1, LLY6E, IFI27, OAS1, IFIT1, IFI6, IFI44L, ISG15, LAMP3, OASL, RSAD2, HERC5; or MX1, LLY6E, IFI27, OAS1, IFIT1, IFI6, IFI44L, ISG15, LAMP3, OASL, RSAD2, DNAPTP6; or MX1, LLY6E, IFI27, OAS1, IFIT1, IFI6, IFI44L, ISG15, LAMP3, OASL, RSAD2, LOC129607; or MX1, LLY6E, IFI27, OAS1, IFIT1, IFI6, IFI44L, ISG15, LAMP3, OASL, RSAD2, EPSTI1; or MX1, LLY6E, IFI27, OAS1, IFIT1, IFI6, IFI44L, ISG15, LAMP3, OASL, RSAD2, BIRC4BP; or MX1, LLY6E, IFI27, OAS1, IFIT1, IFI6, IFI44L, ISG15, LAMP3, OASL, RSAD2, gene detected by probe 229450_at; or MX1, LLY6E, IFI27, OAS1, IFIT1, IFI6, IFI44L, ISG15, LAMP3, OASL, RSAD2, gene detected by probe 235276_at; or LLY6E, IFI27, OAS1, IFIT1, IFI6, IFI44L, ISG15, LAMP3, OASL, RSAD2, IFI44, IFIT2; or LLY6E, IFI27, OAS1, IFIT1, IFI6, IFI44L, ISG15, LAMP3, OASL, RSAD2, IFI44, OAS3; or LLY6E, IFI27, OAS1, IFIT1, IFI6, IFI44L, ISG15, LAMP3, OASL, RSAD2, IFI44, USP18; or LLY6E, IFI27, OAS1, IFIT1, IFI6, IFI44L, ISG15, LAMP3, OASL, RSAD2, IFI44, SIGLEC1; or LLY6E, IFI27, OAS1, IFIT1, IFI6, IFI44L, ISG15, LAMP3, OASL, RSAD2, IFI44, HERC5; or LLY6E, IFI27, OAS1, IFIT1, IFI6, IFI44L, ISG15, LAMP3, OASL, RSAD2, IFI44, DNAPTP6; or LLY6E, IFI27, OAS1, IFIT1, IFI6, IFI44L, ISG15, LAMP3, OASL, RSAD2, IFI44, LOC129607; or LLY6E, IFI27, OAS1, IFIT1, IFI6, IFI44L, ISG15, LAMP3, OASL, RSAD2, IFI44, EPSTI1; or LLY6E, IFI27, OAS1, IFIT1, IFI6, IFI44L, ISG15, LAMP3, OASL, RSAD2, IFI44, BIRC4BP; or LLY6E, IFI27, OAS1, IFIT1, IFI6, IFI44L, ISG15, LAMP3, OASL, RSAD2, IFI44, gene detected by probe 229450_at; or LLY6E, IFI27, OAS1, IFIT1, IFI6, IFI44L, ISG15, LAMP3, OASL, RSAD2, IFI44, gene detected by probe 235276_at; or IFI27, OAS1, IFIT1, IFI6, IFI44L, ISG15, LAMP3, OASL, RSAD2, IFI44, IFIT2, OAS; or IFI27, OAS1, IFIT1, IFI6, IFI44L, ISG15, LAMP3, OASL, RSAD2, IFI44, IFIT2, USP18; or IFI27, OAS1, IFIT1, IFI6, IFI44L, ISG15, LAMP3, OASL, RSAD2, IFI44, IFIT2, SIGLEC1; or IFI27, OAS1, IFIT1, IFI6, IFI44L, ISG15, LAMP3, OASL, RSAD2, IFI44, IFIT2, HERC5; or IFI27, OAS1, IFIT1, IFI6, IFI44L, ISG15, LAMP3, OASL, RSAD2, IFI44, IFIT2, DNAPTP6; or IFI27, OAS1, IFIT1, IFI6, IFI44L, ISG15, LAMP3, OASL, RSAD2, IFI44, IFIT2, LOC129607; or IFI27, OAS1, IFIT1, IFI6, IFI44L, ISG15, LAMP3, OASL, RSAD2, IFI44, IFIT2, EPSTI1; or IFI27, OAS1, IFIT1, IFI6, IFI44L, ISG15, LAMP3, OASL, RSAD2, IFI44, IFIT2, BIRC4BP; or IFI27, OAS1, IFIT1, IFI6, IFI44L, ISG15, LAMP3, OASL, RSAD2, IFI44, IFIT2, gene detected by probe 229450_at; or IFI27, OAS1, IFIT1, IFI6, IFI44L, ISG15, LAMP3, OASL, RSAD2, IFI44, IFIT2, gene detected by probe 235276_at; or OAS1, IFIT1, IFI6, IFI44L, ISG15, LAMP3, OASL, RSAD2, IFI44, IFIT2, OAS3, USP18; or OAS1, IFIT1, IFI6, IFI44L, ISG15, LAMP3, OASL, RSAD2, IFI44, IFIT2, OAS3, SIGLEC1; or OAS1, IFIT1, IFI6, IFI44L, ISG15, LAMP3, OASL, RSAD2, IFI44, IFIT2, OAS3, HERC5; or OAS1, IFIT1, IFI6, IFI44L, ISG15, LAMP3, OASL, RSAD2, IFI44, IFIT2, OAS3, DNAPTP6; or OAS1, IFIT1, IFI6, IFI44L, ISG15, LAMP3, OASL, RSAD2, IFI44, IFIT2, OAS3, LOC129607; or OAS1, IFIT1, IFI6, IFI44L, ISG15, LAMP3, OASL, RSAD2, IFI44, IFIT2, OAS3, EPSTI1; or OAS1, IFIT1, IFI6, IFI44L, ISG15, LAMP3, OASL, RSAD2, IFI44, IFIT2, OAS3, BIRC4BP; or OAS1, IFIT1, IFI6, IFI44L, ISG15, LAMP3, OASL, RSAD2, IFI44, IFIT2, OAS3, gene detected by probe 229450_at; or OAS1, IFIT1, IFI6, IFI44L, ISG15, LAMP3, OASL, RSAD2, IFI44, IFIT2, OAS3, gene detected by probe 235276_at; or IFIT1, IFI6, IFI44L, ISG15, LAMP3, OASL, RSAD2, IFI44, IFIT2, OAS3, USP18, SIGLEC1; or IFIT1, IFI6, IFI44L, ISG15, LAMP3, OASL, RSAD2, IFI44, IFIT2, OAS3, USP18, HERC5; or IFIT1, IFI6, IFI44L, ISG15, LAMP3, OASL, RSAD2, IFI44, IFIT2, OAS3, USP18, DNAPTP6; or IFIT1, IFI6, IFI44L, ISG15, LAMP3, OASL, RSAD2, IFI44, IFIT2, OAS3, USP18, LOC129607; or IFIT1, IFI6, IFI44L, ISG15, LAMP3, OASL, RSAD2, IFI44, IFIT2, OAS3, USP18, EPSTI1; or IFIT1, IFI6, IFI44L, ISG15, LAMP3, OASL, RSAD2, IFI44, IFIT2, OAS3, USP18, BIRC4BP; or IFIT1, IFI6, IFI44L, ISG15, LAMP3, OASL, RSAD2, IFI44, IFIT2, OAS3, USP18, gene detected by probe 229450_at; or IFIT1, IFI6, IFI44L, ISG15, LAMP3, OASL, RSAD2, IFI44, IFIT2, OAS3, USP18, gene detected by probe 235276_at; or IFI6, IFI44L, ISG15, LAMP3, OASL, RSAD2, IFI44, IFIT2, OAS3, USP18, SIGLEC1, HERC5; or IFI6, IFI44L, ISG15, LAMP3, OASL, RSAD2, IFI44, IFIT2, OAS3, USP18, SIGLEC1, DNAPTP6; or IFI6, IFI44L, ISG15, LAMP3, OASL, RSAD2, IFI44, IFIT2, OAS3, USP18, SIGLEC1, LOC129607; or IFI6, IFI44L, ISG15, LAMP3, OASL, RSAD2, IFI44, IFIT2, OAS3, USP18, SIGLEC1, EPSTI1; or IFI6, IFI44L, ISG15, LAMP3, OASL, RSAD2, IFI44, IFIT2, OAS3, USP18, SIGLEC1, BIRC4BP; or IFI6, IFI44L, ISG15, LAMP3, OASL, RSAD2, IFI44, IFIT2, OAS3, USP18, SIGLEC1, gene detected by probe 229450_at; or IFI6, IFI44L, ISG15, LAMP3, OASL, RSAD2, IFI44, IFIT2, OAS3, USP18, SIGLEC1, gene detected by probe 235276_at; or IFI44L, ISG15, LAMP3, OASL, RSAD2, IFI44, IFIT2, OAS3, USP18, SIGLEC1, HERC5, DNAPTP6; or IFI44L, ISG15, LAMP3, OASL, RSAD2, IFI44, IFIT2, OAS3, USP18, SIGLEC1, HERC5, LOC129607; or IFI44L, ISG15, LAMP3, OASL, RSAD2, IFI44, IFIT2, OAS3, USP18, SIGLEC1, HERC5, EPSTI1; or IFI44L, ISG15, LAMP3, OASL, RSAD2, IFI44, IFIT2, OAS3, USP18, SIGLEC1, HERC5, BIRC4BP; or IFI44L, ISG15, LAMP3, OASL, RSAD2, IFI44, IFIT2, OAS3, USP18, SIGLEC1, HERC5, gene detected by probe 229450_at; or IFI44L, ISG15, LAMP3, OASL, RSAD2, IFI44, IFIT2, OAS3, USP18, SIGLEC1, HERC5, gene detected by probe 235276_at; or ISG15, LAMP3, OASL, RSAD2, IFI44, IFIT2, OAS3, USP18, SIGLEC1, HERC5, DNAPTP6, LOC129607; or ISG15, LAMP3, OASL, RSAD2, IFI44, IFIT2, OAS3, USP18, SIGLEC1, HERC5, DNAPTP6, EPSTI1; or ISG15, LAMP3, OASL, RSAD2, IFI44, IFIT2, OAS3, USP18, SIGLEC1, HERC5, DNAPTP6, BIRC4BP; or ISG15, LAMP3, OASL, RSAD2, IFI44, IFIT2, OAS3, USP18, SIGLEC1, HERC5, DNAPTP6, gene detected by probe 229450_at; or ISG15, LAMP3, OASL, RSAD2, IFI44, IFIT2, OAS3, USP18, SIGLEC1, HERC5, DNAPTP6, gene detected by probe 235276_at; or LAMP3, OASL, RSAD2, IFI44, IFIT2, OAS3, USP18, SIGLEC1, HERC5, DNAPTP6, LOC129607, EPSTI1; or LAMP3, OASL, RSAD2, IFI44, IFIT2, OAS3, USP18, SIGLEC1, HERC5, DNAPTP6, LOC129607, BIRC4BP; or LAMP3, OASL, RSAD2, IFI44, IFIT2, OAS3, USP18, SIGLEC1, HERC5, DNAPTP6, LOC129607, gene detected by probe 229450_at; or LAMP3, OASL, RSAD2, IFI44, IFIT2, OAS3, USP18, SIGLEC1, HERC5, DNAPTP6, LOC129607, gene detected by probe 235276_at; or OASL, RSAD2, IFI44, IFIT2, OAS3, USP18, SIGLEC1, HERC5, DNAPTP6, LOC129607, EPSTI1, BIRC4BP; or OASL, RSAD2, IFI44, IFIT2, OAS3, USP18, SIGLEC1, HERC5, DNAPTP6, LOC129607, EPSTI1, gene detected by probe 229450_at; or OASL, RSAD2, IFI44, IFIT2, OAS3, USP18, SIGLEC1, HERC5, DNAPTP6, LOC129607, EPSTI1, gene detected by probe 235276_at; or RSAD2, IFI44, IFIT2, OAS3, USP18, SIGLEC1, HERC5, DNAPTP6, LOC129607, EPSTI1, BIRC4BP, gene detected by probe 229450_at; or RSAD2, IFI44, IFIT2, OAS3, USP 18, SIGLEC1, HERC5, DNAPTP6, LOC129607, EPSTI1, BIRC4BP, gene detected by probe 229450_at; or IFI44, IFIT2, OAS3, USP18, SIGLEC1, HERC5, DNAPTP6, LOC129607, EPSTI1, BIRC4BP, gene detected by probe 229450_at gene detected by probe 235276_at. The IFNα-inducible PD markers in such an expression profile may further include at least one or more gene listed in drawing sheets 235-381.

The IFNα-inducible PD markers in an expression profile may include alterations in any one or more of serum protein levels of adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin. The IFNα-inducible PD markers in such an expression profile may further include at least one or more gene listed in drawing sheets 235-381.

The IFNα-inducible PD markers in an expression profile may include alterations in any one or more of serum protein levels of adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, or vWF. The IFNα-inducible PD markers in such an expression profile may further include at least one or more gene listed in drawing sheets 235-381.

The IFNα-inducible PD markers in an expression profile may include alterations in any one or more of serum protein levels of BDNK, complement 3, CD40 ligand, EGF, ENA-78, EN-RAGE, IGF-1, MDC, myeloperoxidase, RANTES, or thrombopoietin. The IFNα-inducible PD markers in such an expression profile may further include at least one or more gene listed in drawing sheets 235-381.

An IFNα-inducible PD marker expression profile may further include genes whose expression or activity is down-regulated in cells exposed to non-baseline IFNα levels. The genes may include any one or more of SLC4A1, PRSS33, FCER1A, BACH2, KLRB1, D4S234E, T cell receptor alpha locus/T cell receptor delta locus, FEZ1, AFF3, CD160, ABCB1, PTCH1, OR2W3, IGHD, NOG, NR3C2, TNS1, PDZK1IP1, SH2D1B, STRBP, ZMYND11, TMOD1, FCRLA, DKFZp761P0423, EPB42, NR6A1, LOC341333, MS4A1, IGHM, SIGLECP3, KIR2DS2, PKIA, BLR1, C5orf4, MYLK, LOC283663, MAD1L1, CXCL5, D4S234E, FCRLA, KRT1, c16orf74, ABCB4, or GPRASP1.

PD markers may be upregulated or may be downregulated relative to those of healthy subjects or non-afflicted patient tissues. The upregulation or downregulation of the type I IFN or IFNα-inducible PD markers in the patient's expression profile may be by any degree relative to that of a sample from a control (which may be from a sample that is not disease tissue of the patient (e.g., non-lesional skin of a psoriasis patient) or from a healthy person not afflicted with the disease or disorder). The degree upregulation or downregulation may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control or control sample.

A patient comprising the type I IFN or IFNα-inducible PD marker expression profile may further comprise upregulation of expression of any number of IFNα or type-I IFN subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. The patient may comprise upregulation of expression of IFN subtypes IFNα1, IFNα2, IFNα8, and IFNα14. The upregulation of expression may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of the control.

A patient comprising a type I IFN or IFNα-inducible PD marker expression profile may further comprise upregulation of expression of IFNα receptors, either IFNAR1 or IFNAR2, or both, or TNFα, or IFNγ, or IFNγ receptors (either IFNGR1, IFNGR2, or both IFNGR1 and IFNGR2). The patient may simply be identified as one who comprises upregulation of expression of IFNα receptors, either IFNAR1 or IFNAR2, or both, or TNFα, or IFNγ, or IFNγ receptors (either IFNGR1, IFNGR2, or both IFNGR1 and IFNGR2).

The patient may further comprise or alternatively comprise alterations in levels of proteins in serum. The patient may have increased serum levels of proteins such as adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, or vWF. The patient may have increased serum levels of any 1, 2,3, 4, 5, 6, 7, 8, 9, 10, 11, 12, 13 14, 15, 16, 17, 18, 19, 20, 21, o22, 23, 24, 25, or 26 of these proteins in serum. The increased level may be at least 10%, at least 15%, at least 20%, at least 25%, at least 30%, at least 40%, at least 50%, at least 60%, at least 70%, at least 75%, at least 80%, at least 90%, at least 100%, at least 125%, at least 150%, or at least 200%, or at least 300%, or at least 400%, or at least 500% that of a control, e.g., a healthy subject. The alteration may be a decrease in serum levels of proteins such as BDNK, complement 3, CD40 ligand, EGF, ENA-78, EN-RAGE, IGF-1, MDC, myeloperoxidase, RANTES, or thrombopoietin, The patient may have decreased serum levels of any 1, 2, 3, 4, 5, 6, 7, 8, 9, 10, or 11 or these proteins. The decreased level may be at least 10%, at least 15%, at least 20%, at least 25%, at least 30%, at least 40%, at least 50%, at least 60%, at least 70%, at least 75%, at least 80%, at least 90%, or at least 100% that of a control, e.g., a healthy subject. The PD marker profile may comprise one or more of these increased or decreased serum levels of proteins.

The patient may further comprise auto-antibodies that bind to any one of the following auto-antigens: (a) Myxovirus (influenza virus) resistance 1, interferon-inducible protein p78; (b) surfeit 5, transcript variant c; (c) proteasome (posome, macropain) activator subunit 3 (PA28 gamma; Ki) transc; (d) retinoic acid receptor, alpha; (e) Heat shock 10 kDa protein 1 (chaperonin 10); (f) tropomyosin 3; (g) pleckstrin homology-like domain, family A, member 1; (h) cytoskeleton-associated protein 1; (i) Sjogren syndrome antigen A2 (60 kDa, ribonucleoprotein auto-antigen SS-A/Ro); (j) NADH dehydrogenase (ubiquinone) 1, alpha/beta subcomplex 1, 8 kDa; (k) NudE nuclear distribution gene E homolog 1 (A. nidulans); (l) MutL homolog 1, colon cancer, nonpolyposis type 2 (E. coli); (m) leucine rich repeat (in FLII) interacting protein 2; (n) tropomyosin 1 (alpha); (o) spastic paraplegia 20, spartin (Troyer syndrome); (p) preimplantation protein, transcript variant 1; (r) mitochondrial ribosomal protein L45; (s) Lin-28 homolog (C. elegans); (t) heat shock 90 kDa protein 1, alpha; (u) dom-3 homolog Z (C. elegans); (v) dynein, cytoplasmic, light intermediate polypeptide 2; (w) Ras-related C3 botulinum toxin substrate 1 (rho family, small GTP binding protein); (x) synovial sarcoma, X breakpoint 2, transcript variant 2; (y) moesin; (z) homer homolog (Drosophila), transcript variant 1; (aa) GCN5 general control of amino-acid synthesis 5-like 2 (yeast); (bb) eukaryotic translation elongation factor 1 gamma; (cc) eukaryotic translation elongation factor 1, delta; (dd) DNA-damage-inducible transcript 3; (ee) CCAAT/enhancer binding protein (C/EBP) gamma; and any other auto-antigen described in provisional application entitled "Auto-antibody markers of autoimmune disease" filed May 3, 2007 or in provisional application entitled entitled "Auto-antibody markers of autoimmune disease" filed November 6, 2007 (for example, but not limited to, those described on Tables 2, 4, 5, and 9). The patient may comprise auto-antibodies that bind to any number of these auto-antigens, e.g., any at least 2, at least 3, at least 4, at least 5, at least 6, at least 7, at least 8, at least 9 at least 10, at least 11, at least 12, at least 13, at least 14, at least 15, at least 20, at least 25.

A therapeutic agent may be administered to a myositis patient. A therapeutic agent may be any molecule that binds to and modulates type I IFN or IFNα activity. The therapeutic agent may be a small molecule or a biological agent. If the therapeutic agent is a small molecule it may be synthesized or identified and isolated from a natural source.

If the therapeutic agent is a biological agent, it may be an antibody specific for any subtype(s) of type I IFN or IFNα. For instance, the antibody may be specific for any one of IFNα1, IFNα2, IFNα4, IFNα5, IFNα6, IFNα7, IFNα8, IFNα10, IFNα14, IFNα17, IFNα21, IFNβ, or IFNω. Alternatively, the antibody may be specific for any two, any three, any four, any five, any six, any seven, any eight, any nine, any ten, any eleven, any twelve type I IFN of IFNα subtypes. If the antibody is specific for more than one type I IFN subtype, the antibody may be specific for IFNα1, IFNα2, IFNα4, IFNα5, IFNα8, IFNα10, and IFNα21; or it may be specific for IFNα1, IFNα2, IFNα4, IFNα5, IFNα8, and IFNα10; or it may be specific for IFNα1, IFNα2, IFNα4, IFNα5, IFNα8, and IFNα21; or it may be specific for IFNα1, IFNα2, IFNα4, IFNα5, IFNα10, and IFNα21. Antibodies specific for type I IFN or IFNα include MEDI-545, any biologic or antibody other than MEDI-545, antibodies described in U.S. patent applications 11/009,410 filed December 10, 2004 and 11/157,494 filed June 20, 2005, 9F3 and other antibodies described in U.S. Patent No. 7,087,726 (Example 1 and Example 2, those disclosed in Table 3 and Table 4, and/or those disclosed in the table entitled "Deposit of Material" on lines 25-54, column 56), NK-2 and YOK5/19 (WO 84/03105), LO-22 (U.S. Patent 4,902,618), 144 BS (U.S. Patent 4,885,166), and EBI-1, EBI-2, and EBI-3 (EP 119476). A therapeutic agent that modulates IFNα activity may neutralize IFNα activity. One of skill in the art is well aware of preparation and formulation of such biological agents and methods of their administration.

The antibody may be a synthetic antibody, a monoclonal antibody, polyclonal antibodies, a recombinantly produced antibody, an intrabody, a multispecific antibody (including bi-specific antibodies), a human antibody, a humanized antibody, a chimeric antibody, a single-chain Fv (scFv) (including bi-specific scFv), a BiTE molecule, a single chain antibody, a Fab fragments, a F(ab') fragment, a disulfide-linked Fv (sdFv), or an epitope-binding fragment of any of the above. The antibody may be any of an immunoglobulin molecule or immunologically active portion of an immunoglobulin molecule. Furthermore, the antibody may be of any isotype. For example, it may be any of isotypes IgG1, IgG2, IgG3 or IgG4. The antibody may be a full-length antibody comprising variable and constant regions, or an antigen-binding fragment thereof, such as a single chain antibody, or a Fab or Fab'2 fragment. The antibody may also be conjugated or linked to a therapeutic agent, such as a cytotoxin or a radioactive isotope.

A second agent other than the agent that binds to modulates IFNα activity may be administered to a myositis patient. Second agents include, but are not limited to non-steroidal anti-inflammatory drugs such as ibuprofen, naproxen, sulindac, diclofenac, piroxicam, ketoprofen, diflunisal, nabumetone, etodolac, and oxaprozin, indomethacin; anti-malarial drugs such as hydroxychloroquine; corticosteroid hormones, such as prednisone, hydrocortisone, methylprednisolone, and dexamethasone; methotrexate; immunosuppressive agents, such as azathioprine and cyclophosphamide; and biologic agents that, e.g., target T cells such as Alefacept and Efalizumab, or target TNFα, such as, Enbrel, Remicade, and Humira.

Treatment with the agent may result in neutralization of the type I IFN or IFNα-inducible profile. Treatment with the agent may result in a decrease in one or more symptoms of myositis or disease flare-ups. The symptoms may include muscle weakness, difficulty swallowing, difficulty breathing, fever, weight loss, pain, muscle tenderness, arthritis, and rash. Treatment with the agent may result in improved prognosis. Treatment with the agent may result in a higher quality of life for the patient. Treatment with the agent may alleviate the need to co-administer second agents or may lessen the dosage of administration of the second agent to the patient. Treatment with the agent may reduce the number of hospitalizations of the patient.

The agent that binds to and modulates type I IFN or IFNα activity may neutralize a type I IFN or IFNα-inducible profile. Neutralization of the type I IFN or IFNα-inducible profile may be a reduction in at least one, at least two, at least three, at least five, at least seven, at least eight, at least ten, at least twelve, at least fifteen, at least twenty, at least twenty five, at least thirty, at least thirty five, at least forty, at least forty five, or at least fifty genes up-regulated by type I IFN or IFNα. The genes upregulated by type I IFN or IFNα may be any group of genes in Figures 235-381 or as discussed above. Neutralization of the type I IFN or IFNα-inducible profile is a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, at least ten, at least twelve, at least fifteen, at least twenty, at least twenty five, at least thirty, at least thirty five, at least forty, at least forty five, or at least fifty genes up-regulated in any type I IFN or IFNα-inducible profile. Alternatively, neutralization of the type I IFN or IFNα-inducible profile may refer to a reduction of expression of up-regulated type I IFN or IFNα-inducible genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those type I IFN or IFNα-inducible genes in a control sample. If the agent that binds to and modulates type I IFN or IFNα activity is a biologic agent, such as an antibody, the agent may neutralize the type I IFN or IFNα profile at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

Neutralization of the type I IFN or IFNα-inducible profile may be increased expression of at least one, at least two, at least three, at least five, at least seven, at least eight, at least ten, at least twelve, at least fifteen, at least twenty, at least twenty five, at least thirty, at least thirty five, at least forty, at least forty five, or at least fifty genes whose expression is reduced by type I IFN or IFNα. The genes whose expression is reduced by type I IFN or IFNα may be any group of genes in Figures 235-381 or as discussed above. Neutralization of down-regulated genes in a type I IFN or IFNα-inducible profile may be an increase of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90%, or at least 100%, or at least 125%, or at least 130%, or at least 140%, or at least 150%, or at least 175%, or at least 200%, or at least 250%, or at least 300%, or at least 500% of any of the at least one, at least two, at least three, at least five, at least seven, at least eight, at least ten, at least twelve, at least fifteen, at least twenty, or at least twenty five genes whose expression is downregulated in any type I IFN or IFNα-inducible profile. Alternatively, neutralization of the type I IFN or IFNα-inducible profile refers to an increase in expression of type I IFN or IFNα-inducible genes to within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those type I IFN or IFNα-inducible (downregulated) genes in a control sample. If the agent that binds to and modulates type I IFN or IFNα activity is a biologic agent, such as an antibody, the agent may neutralize the type I IFN or IFNα profile at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively neutralize expression of one or more type I IFN or IFNα subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. These subtypes may include all of IFNα1, IFNα2, IFNα8, and IFNα14. Alternatively, these subtypes may include IFNα1, IFNα2, IFNα4, IFNα5, IFNα8, IFNα10, IFNα21. Neutralization of the IFNα or type-I IFN subtypes may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, or at least ten of the subtypes. Neutralization of the IFNα or type-I IFN subtypes may be a reduction in expression of IFNα or type-I IFN subtype genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those IFNα or type I IFN subtypes in a control sample. If the agent that binds to and modulates IFNα activity or type I IFN activity is a biologic agent, such as an antibody, the agent may neutralize the IFNα or type I IFN subtypes at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively neutralize expression of IFNα receptors, either IFNAR1 or IFNAR2, or both, or TNFα, or IFNγ, or IFNγ receptors (either IFNGR1, IFNGR2, or both IFNGR1 and IFNGR2). Neutralization of expression of IFNα receptors, either IFNAR1 or IFNAR2, or both, or TNFα, or IFNγ, or IFNγ receptors (either IFNGR1, IFNGR2, or both IFNGR1 and IFNGR2) may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, or at least six of these genes. Neutralization of expression of IFNα receptors, either IFNAR1 or IFNAR2, or TNFα, or IFNγ, or IFNγ receptors (either IFNGR1, IFNGR2, or both IFNGR1 and IFNGR2) is a reduction of expression of at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of these genes in a control sample. If the agent that binds to and modulates type I IFN or IFNα activity is a biologic agent, such as an antibody, the agent may neutralize expression of IFNα receptors IFNAR1 or IFNAR2, or TNFα, or IFNγ, or IFNγ receptors IFNGR1 or IFNGR2 at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively neutralize alterations of levels of proteins in serum, e.g., increase levels of those proteins whose serum levels are downregulated or decrease levels of those proteins whose serum levels are upregulated to levels closer to those of control subjects. Neutralization of expression of proteins in serum, such as adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin may be by bringing the level of at least one, at least two, at least three, at least five, at least six, at least seven, at least eight, at least nine, at least ten, at least twelve, at least fifteen, at least twenty, or at least 25 of these proteins to within at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% levels of the protein in serum of a healthy subject. If the agent that binds to and modulates type I IFN or IFNα activity is a biologic agent, such as an antibody, the agent may neutralize levels of the serum proteins, *e*.*g*., adiponectin, alpha-fetoprotein, apolipoprotein CIII, beta-2 microglobulin, cancer antigen 125, cancer antigen 19-9, eotaxin, FABP, factor VII, ferritin, IL-10, IL-12p70, IL-16, IL-18, IL-1ra, IL-3, MCP-1, MMP-3, myoglobin, SGOT, tissue factor, TIMP-1, TNF RII, TNF-alpha, VCAM-1, vWF, BDNK, complement 3, CD40 ligand, EGF, ENA-78, EN-RAGE, IGF-1, MDC, myeloperoxidase, RANTES, or thrombopoietin, at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may further or alternatively reduce number or level of auto-antibodies that bind to any one, any at least 2, any at least 3, any at least 4, any at least 5, any at least 6, any at least 7, any at least 8, any at least 9, any at least 10, any at least 15, or any at least 20 of the following auto-antigens: (a) Myxovirus (influenza virus) resistance 1, interferon-inducible protein p78; (b) surfeit 5, transcript variant c; (c) proteasome (posome, macropain) activator subunit 3 (PA28 gamma; Ki) transc; (d) retinoic acid receptor, alpha; (e) Heat shock 10 kDa protein 1 (chaperonin 10); (f) tropomyosin 3; (g) pleckstrin homology-like domain, family A, member 1; (h) cytoskeleton-associated protein 1; (i) Sjogren syndrome antigen A2 (60 kDa, ribonucleoprotein auto-antigen SS-A/Ro); (j) NADH dehydrogenase (ubiquinone) 1, alpha/beta subcomplex 1, 8 kDa; (k) NudE nuclear distribution gene E homolog 1 (A. nidulans); (l) MutL homolog 1, colon cancer, nonpolyposis type 2 (E. coli); (m) leucine rich repeat (in FLII) interacting protein 2; (n) tropomyosin 1 (alpha); (o) spastic paraplegia 20, spartin (Troyer syndrome); (p) preimplantation protein, transcript variant 1; (r) mitochondrial ribosomal protein L45; (s) Lin-28 homolog (C. elegans); (t) heat shock 90 kDa protein 1, alpha; (u) dom-3 homolog Z (C. elegans); (v) dynein, cytoplasmic, light intermediate polypeptide 2; (w) Ras-related C3 botulinum toxin substrate 1 (rho family, small GTP binding protein); (x) synovial sarcoma, X breakpoint 2, transcript variant 2; (y) moesin; (z) homer homolog (Drosophila), transcript variant 1; (aa) GCN5 general control of amino-acid synthesis 5-like 2 (yeast); (bb) eukaryotic translation elongation factor 1 gamma; (cc) eukaryotic translation elongation factor 1, delta; (dd) DNA-damage-inducible transcript 3; (ee) CCAAT/enhancer binding protein (C/EBP) gamma; and any other auto-antigen described in provisional application entitled "Auto-antibody markers of autoimmune disease" filed May 3, 2007; and any other auto-antigen described in provisional application entitled "Auto-antibody markers of autoimmune disease" filed November 6, 2007 (for example, but not limited to, those described on Tables 2, 4, 5, and 9). Reduction in level of auto-antibody may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% in presence of any of the auto-antibodies. If the agent that binds to and modulates type I IFN or IFNα activity is a biologic agent, such as an antibody, the agent may reduce number or level or auto-antibodies at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates type I IFN or IFNα activity may not neutralize expression of genes that are not included in an interferon-inducible signature or PD marker profile.

Monitoring disease progression may be performed by obtaining patient samples before and after administration of an agent, *e.g.,* an agent that binds to and modulates type I IFN or IFNα activity, or an agent that binds to and does not modulate type I IFN or IFNα activity, or a combination of agents that may or may not include an agent that binds to and modulates type I IFN or IFNα activity. Samples include any biological fluid or tissue, such as whole blood, saliva, urine, synovial fluid, bone marrow, cerebrospinal fluid, nasal secretions, sputum, amniotic fluid, bronchoalveolar lavage fluid, peripheral blood mononuclear cells, total white blood cells, lymph node cells, spleen cells, tonsil cells, or skin. The samples may be obtained by any means known in the art.

Type I IFN or IFNα inducible PD marker expression profiles are obtained in the (before and after agent administration) samples. The type I IFN or IFNα inducible PD marker expression profiles in the samples are compared. Comparison may be of the number of type I IFN or IFNα inducible PD markers present in the samples or may be of the quantity of type I IFN or IFNα inducible PD markers present in the samples, or any combination thereof. Variance indicating efficacy of the therapeutic agent may be indicated if the number or level (or any combination thereof) of up-regulated type I IFN or IFNα inducible PD markers decreases in the sample obtained after administration of the therapeutic agent relative to the sample obtained before administration of the therapeutic agent. The number of up-regulated type I IFN or IFNα inducible PD markers may decrease by at least 1, at least 2, at least 3, at least 4, at least 5, at least 6, at least 7, at least 8, at least 9, or at least 10. The level of any given up-regulated type I IFN or IFNα inducible PD marker may decrease by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. Any combination of decreased number and decreased level of up-regulated type I IFN or IFNα inducible PD markers may indicate efficacy. Variance indicating efficacy of the therapeutic agent may be indicated if the number or level (or any combination thereof) of down-regulated type I IFN or IFNα inducible PD markers decreases in the sample obtained after administration of the therapeutic agent relative to the sample obtained before administration of the therapeutic agent. The number of down-regulated type I IFN or IFNα inducible PD markers may decrease by at least 1, at least 2, at least 3, at least 4, at least 5, at least 6, at least 7, at least 8, at least 9, or at least 10. The level of any given down-regulated type I IFN or IFNα inducible PD marker may increase by at least 10%, at least 20%, at least 25%, at least 30%, at least 35%, at least 40%, at least 50%, at least 60%, at least 70%, at least 80%, at least 90%, or at least 95%. The number of down-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. Any combination of decreased number and increased level of down-regulated type I IFN or IFNα inducible PD markers may indicate efficacy.

The sample obtained from the patient may be obtained prior to a first administration of the agent, *i.e.,* the patient is naïve to the agent. Alternatively, the sample obtained from the patient may occur after administration of the agent in the course of treatment. For example, the agent may have been administered prior to the initiation of the monitoring protocol. Following administration of the agent an additional samples may be obtained from the patient and type I IFN or IFNα inducible PD markers in the samples are compared. The samples may be of the same or different type, e.g., each sample obtained may be a blood sample, or each sample obtained may be a serum sample. The type I IFN or IFNα inducible PD markers detected in each sample may be the same, may overlap substantially, or may be similar.

The samples may be obtained at any time before and after the administration of the therapeutic agent. The sample obtained after administration of the therapeutic agent may be obtained at least 2, at least 3, at least 4, at least 5, at least 6, at least 7, at least 8, at least 9, at least 10, at least 12, or at least 14 days after administration of the therapeutic agent. The sample obtained after administration of the therapeutic agent may be obtained at least 2, at least 3, at least 4, at least 5, at least 6, at least 7, or at least 8 weeks after administration of the therapeutic agent. The sample obtained after administration of the therapeutic agent may be obtained at least 2, at least 3, at least 4, at least 5, or at least 6 months following administration of the therapeutic agent.

Additional samples may be obtained from the patient following administration of the therapeutic agent. At least 2, at least 3, at least 4, at least 5, at least 6, at least 7, at least 8, at least 9, at least 10, at least 12, at least 15, at least 20, at least 25 samples may be obtained from the patient to monitor progression or regression of the disease or disorder over time. Disease progression may be monitored over a time period of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least 1 year, at least 2 years, at least 3 years, at least 4 years, at least 5 years, at least 10 years, or over the lifetime of the patient. Additional samples may be obtained from the patient at regular intervals such as at monthly, bi-monthly, once a quarter year, twice a year, or yearly intervals. The samples may be obtained from the patient following administration of the agent at regular intervals. For instance, the samples may be obtained from the patient at one week following each administration of the agent, or at two weeks following each administration of the agent, or at three weeks following each administration of the agent, or at one month following each administration of the agent, or at two months following each administration of the agent. Alternatively, multiple samples may be obtained from the patient following an or each administration of the agent.

Disease progression in a patient may similarly be monitored in the absence of administration of an agent. Samples may periodically be obtained from the patient having the disease or disorder. Disease progression may be identified if the number of type I IFN or IFNα inducible PD markers increases in a later-obtained sample relative to an earlier obtained sample. The number of type I IFN or IFNα inducible PD markers may increase by at least 1, at least 2, at least 3, at least 4, at least 5, at least 6, at least 7, at least 8, at least 9, or at least 10. Disease progression may be identified if level of any given up-regulated type I IFN or IFNα inducible PD marker increases by at least 10%, at least 20%, at least 25%, at least 30%, at least 35%, at least 40%, at least 50%, at least 60%, at least 70%, at least 80%, at least 90%, or at least 95%. Disease progression may be identified if level of any given down-regulated type I IFN or IFNα inducible PD marker decreases by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. The number of down-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. Any combination of increased number and increased level of up-regulated type I IFN or IFNα inducible PD marker may indicate disease progression. Alternatively, or in combination, any combination of decreased number and decreased level of down-regulated type I IFN or IFNα inducible PD marker may indicate disease progression. Disease regression may also be identified in a patient having a disease or disorder, not treated by an agent. In this instance, regression may be identified if the number of type I IFN or IFNα inducible PD markers decreases in a later-obtained sample relative to an earlier obtained sample. The number of type I IFN or IFNα inducible PD markers may decrease by at least 1, at least 2, at least 3, at least 4, at least 5, at least 6, at least 7, at least 8, at least 9, or at least 10. Disease regression may be identified if level of any given up-regulated type I IFN or IFNα inducible PD marker decreases by at least 10%, at least 20%, at least 25%, at least 30%, at least 35%, at least 40%, at least 50%, at least 60%, at least 70%, at least 80%, at least 90%, or at least 95%. Disease regression may be identified if level of any given down-regulated type I IFN or IFNα inducible PD marker increases by at least 10%, at least 20%, at least 25%, at least 30%, at least 35%, at least 40%, at least 50%, at least 60%, at least 70%, at least 80%, at least 90%, or at least 95%. The number of up-regulated type I IFN or IFNα inducible PD markers with decreased levels may be at least 1, at least 2, at least 3, at least 4, at least 5, at least 6, at least 7, at least 8, at least 9, at least 10, at least 15, at least 20, at least 25, at least 30, or at least 35. The number of down-regulated type I IFN or IFNα inducible PD markers with increased levels may be at least 1, at least 2, at least 3, at least 4, at least 5, at least 6, at least 7, at least 8, at least 9, at least 10, at least 15, at least 20, at least 25, at least 30, or at least 35. Disease progression or disease regression may be monitored by obtaining samples over any period of time and at any interval. Disease progression or disease regression may be monitored by obtaining samples over the course of at least 1 week, at least 2 weeks, at least 3 weeks, at least 4 weeks, at least 5 weeks, at least 6 weeks, at least 7 weeks, at least 2 months, at least 3 months, at least 4 months, at least 5 months, at least 6 months, at least 1 year, at least 2 years, at least 3 years, at least 4 years, at least 5 years, at least 10 years, or over the lifetime of the patient. Disease progression or disease regression may be monitored by obtaining samples at least monthly, bi-monthly, once a quarter year, twice a year, or yearly. The samples need not be obtained at strict intervals.

The invention also encompasses kits and probes. The probes may be any molecule that detects any expression or activity of any gene that may be included in an IFNα-inducible PD marker expression profile.

Applicants provide a set of non-limiting embodiments to describe some of the aspects of the invention.

### Embodiments

1. A method of treating a patient having a type I IFN or an IFNα-mediated disease or disorder comprising:
   administering an agent that binds to and modulates type I IFN or IFNα activity;
      wherein the patient comprises a differentially regulated miRNA marker profile; and
      wherein the agent neutralizes the differentially regulated miRNA marker profile of the patient.
2. The method of embodiment 1 further comprising detecting neutralization of the differentially regulated miRNA marker profile of the patient.
3. The method of embodiment 1 wherein the differentially regulated miRNA marker profile comprises up-regulated expression or activity of at least one of the miRNAs detected by the detectors identified in Tables 2 or 4.
4. The method of embodiment 3 wherein the differentially regulated miRNA marker profile comprises up-regulated expression or activity of at least two of the miRNAs detected by the detectors identified in Tables 2 or 4.
5. The method of embodiment 4 wherein the differentially regulated miRNA marker profile comprises up-regulated expression or activity of at least five of the miRNAs detected by the detectors identified in Tables 2 or 4.
6. The method of embodiment 5 wherein the differentially regulated miRNA marker profile comprises up-regulated expression or activity of at least ten of the miRNAs detected by the detectors identified in Tables 2 or 4.
7. The method of embodiment 6 wherein the differentially regulated miRNA marker profile comprises up-regulated expression or activity of at least fifteen of the miRNAs detected by the detectors identified in Tables 2 or 4.
8. The method of embodiment 3 wherein the at least one of the miRNAs is detected by the detector hsa-miR-34b-4373037.
9. The method of embodiment 1 wherein the differentially regulated miRNA marker profile comprises down-regulated expression or activity of at least one of the miRNAs detected by the detectors identified in Tables 3 or 5.
10. The method of embodiment 9 wherein the differentially regulated miRNA marker profile comprises down-regulated expression or activity of at least two of the miRNAs detected by the detectors identified in Tables 3 or 5.
11. The method of embodiment 10 wherein the differentially regulated miRNA marker profile comprises down-regulated expression or activity of at least five of the miRNAs detected by the detectors identified in Tables 3 or 5.
12. The method of embodiment 11 wherein the differentially regulated miRNA marker profile comprises down-regulated expression or activity of at least ten of the miRNAs detected by the detectors identified in Tables 3 or 5.
13. The method of embodiment 12 wherein the differentially regulated miRNA marker profile comprises down-regulated expression or activity of at least fifteen of the miRNAs detected by the detectors identified in Tables 3 or 5.
14. The method of embodiment 9 wherein the at least one of the miRNAs is detected by the detector hsa-miR-1-4373161.
15. The method of embodiment 3 wherein the differentially regulated miRNA marker profile further comprises down-regulated expression or activity of at least one of the miRNAs detected by the detectors identified in Tables 3 or 5.
16. The method of embodiment 15 wherein the down-regulated expression or activity is of at least two of the miRNAs detected by the detectors identified in Tables 3 or 5.
17. The method of embodiment 16 wherein the down-regulated expression or activity is of at least five of the miRNAs detected by the detectors identified in Tables 3 or 5.
18. The method of embodiment 17 wherein the down-regulated expression or activity is of at least ten of the miRNAs detected by the detectors identified in Tables 3 or 5.
19. The method of embodiment 18 wherein the down-regulated expression or activity is of at least fifteen of the miRNAs detected by the detectors identified in Tables 3 or 5.
20. The method of embodiment 15 wherein the at least one of the miRNAs detected by the detectors identified in Tables 3 or 5 is hsa-miR-1-4373161.
21. The method of embodiment 1 wherein the agent is a biologic agent.
22. The method of embodiment 21 wherein the agent is an antibody.
23. The method of embodiment 22 wherein the antibody is MEDI-545.
24. The method of embodiment 22 wherein the antibody is specific for one or more type I IFN or IFNα subtype but is not MEDI-545.
25. The method of embodiment 1 wherein the administering the agent alleviates one or more symptoms of the disease or disorder.
26. The method of embodiment 22 wherein the antibody is administered at a dose between approximately .03 and 30 mg/kg.
27. The method of embodiment 26 wherein the antibody is administered at a dose between 0.3 and 3 mg/kg.
28. The method of embodiment 27 wherein the antibody is administered at a dose between .03 and 1 mg/kg.
29. The method of any one of embodiments 21 wherein the agent neutralizes the differentially regulated miRNA marker profile of the patient by at least 10%.
30. The method of embodiment 29 wherein the agent neutralizes the differentially regulated miRNA marker profile of the patient by at least 20%.
31. The method of embodiment 30 wherein the agent neutralizes the differentially regulated miRNA marker profile of the patient by at least 30%.
32. The method of embodiment 31 wherein the agent neutralizes the differentially regulated miRNA marker profile of the patient by at least 40%.
33. The method of embodiment 32 wherein the agent neutralizes the differentially regulated miRNA marker profile of the patient by at least 50%.
34. The method of embodiment 1 wherein the disease or disorder is one of lupus, psoriasis, vasculitis, sarcoidosis, Sjogren's syndrome, or myositis.
35. The method of embodiment 34 wherein the disease or disorder is myositis.
36. The method of embodiment 1 wherein the patient further comprises up-regulated expression or activity of at least IFNα subtypes 1, 2, 8, and 14.
37. The method of embodiment 2 wherein the differentially regulated miRNA marker profile is detected in whole blood of the patient.
38. The method of embodiment 2 wherein the differentially regulated miRNA marker profile is detected in muscle tissue of the patient.
39. The method of embodiment 1 wherein the differentially regulated miRNA marker profile is strong or moderate.
40. A method of neutralizing a differentially regulated miRNA marker profile in a patient in need thereof, comprising:
   administering an agent that binds to and modulates type I IFN or IFNα activity to the patient;
      wherein the agent neutralizes the differentially regulated miRNA marker profile of the patient.
41. The method of embodiment 40 further comprising detecting neutralization of the differentially regulated miRNA marker profile of the patient.
42. The method of embodiment 40 wherein the differentially regulated miRNA marker profile comprises up-regulated expression or activity of at least one of the miRNAs detected by the detectors identified in Tables 2 or 4.
43. The method of embodiment 42 wherein the differentially regulated miRNA marker profile comprises up-regulated expression or activity of at least two of the miRNAs detected by the detectors identified in Tables 2 or 4.
44. The method of embodiment 43 wherein the differentially regulated miRNA marker profile comprises up-regulated expression or activity of at least five of the miRNAs detected by the detectors identified in Tables 2 or 4.
45. The method of embodiment 44 wherein the differentially regulated miRNA marker profile comprises up-regulated expression or activity of at least ten of the miRNAs detected by the detectors identified in Tables 2 or 4.
46. The method of embodiment 45 wherein the differentially regulated miRNA marker profile comprises up-regulated expression or activity of at least fifteen of the miRNAs detected by the detectors identified in Tables 2 or 4.
47. The method of embodiment 42 wherein the at least one of the miRNAs is detected by the detector hsa-miR-34b-4373037.
48. The method of embodiment 40 wherein the differentially regulated miRNA marker profile comprises down-regulated expression or activity of at least one of the miRNAs detected by the detectors identified in Tables 3 or 5.
49. The method of embodiment 48 wherein the differentially regulated miRNA marker profile comprises down-regulated expression or activity of at least two of the miRNAs detected by the detectors identified in Tables 3 or 5.
50. The method of embodiment 49 wherein the differentially regulated miRNA marker profile comprises down-regulated expression or activity of at least five of the miRNAs detected by the detectors identified in Tables 3 or 5.
51. The method of embodiment 50 wherein the differentially regulated miRNA marker profile comprises down-regulated expression or activity of at least ten of the miRNAs detected by the detectors identified in Tables 3 or 5.
52. The method of embodiment 51 wherein the differentially regulated miRNA marker profile comprises down-regulated expression or activity of at least fifteen of the miRNAs detected by the detectors identified in Tables 3 or 5.
53. The method of embodiment 48 wherein the at least one of the miRNAs is detected by the detector hsa-miR-1-4373161.
54. The method of embodiment 42 wherein the differentially regulated miRNA marker profile further comprises down-regulated expression or activity of at least one of the miRNAs detected by the detectors identified in Tables 3 or 5.
55. The method of embodiment 54 wherein the down-regulated expression or activity is of at least two of the miRNAs detected by the detectors identified in Tables 3 or 5.
56. The method of embodiment 55 wherein the down-regulated expression or activity is of at least five of the miRNAs detected by the detectors identified in Tables 3 or 5.
57. The method of embodiment 56 wherein the down-regulated expression or activity is of at least ten of the miRNAs detected by the detectors identified in Tables 3 or 5.
58. The method of embodiment 57 wherein the down-regulated expression or activity is of at least fifteen of the miRNAs detected by the detectors identified in Tables 3 or 5.
59. The method of embodiment 54 wherein the at least one of the miRNAs detected by the detectors identified in Tables 3 or 5 is hsa-miR-1-4373161.
60. The method of embodiment 40 wherein the agent is a biologic agent.
61. The method of embodiment 60 wherein the agent is an antibody.
62. The method of embodiment 61 wherein the antibody is MEDI-545.
63. The method of embodiment 61 wherein the antibody is specific for one or more type I IFN or IFNα subtype but is not MEDI-545.
64. The method of embodiment 40 wherein the administering the agent alleviates one or more symptoms of the disease or disorder.
65. The method of embodiment 61 wherein the antibody is administered at a dose between approximately .03 and 30 mg/kg.
66. The method of embodiment 65 wherein the antibody is administered at a dose between 0.3 and 3 mg/kg.
67. The method of embodiment 66 wherein the antibody is administered at a dose between .03 and 1 mg/kg.
68. The method of embodiment 40 wherein the agent neutralizes the differentially regulated miRNA marker profile of the patient by at least 10%.
69. The method of embodiment 68 wherein the agent neutralizes the differentially regulated miRNA marker profile of the patient by at least 20%.
70. The method of embodiment 69 wherein the agent neutralizes the differentially regulated miRNA marker profile of the patient by at least 30%.
71. The method of embodiment 70 wherein the agent neutralizes the differentially regulated miRNA marker profile of the patient by at least 40%.
72. The method of embodiment 71 wherein the agent neutralizes the differentially regulated miRNA marker profile of the patient by at least 50%.
73. The method of embodiment 40 wherein the patient has been diagnosed with one of lupus, psoriasis, vasculitis, sarcoidosis, Sjogren's syndrome, or myositis.
74. The method of embodiment 73 wherein the patient has been diagnosed with myositis.
75. The method of embodiment 40 wherein the patient further comprises up-regulated expression or activity of at least IFNα subtypes 1, 2, 8, and 14.
76. The method of embodiment 41 wherein the differentially regulated miRNA marker profile is detected in whole blood of the patient.
77. The method of embodiment 41 wherein the differentially regulated miRNA marker profile is detected in muscle tissue of the patient.
78. The method of embodiment 40 wherein the differentially regulated miRNA marker profile is strong or moderate.
79. A method of monitoring or prognosing an inflammatory disease progression of a patient comprising:
   obtaining a first differentially regulated miRNA marker profile in a first sample from a patient.
80. The method of embodiment 79 wherein the first differentially regulated miRNA marker profile comprises up-regulated expression or activity of at least one of the miRNAs detected by the detectors identified in Tables 2 or 4.
81. The method of embodiment 80 wherein the first differentially regulated miRNA marker profile comprises up-regulated expression or activity of at least two of the miRNAs detected by the detectors identified in Tables 2 or 4.
82. The method of embodiment 81 wherein the first differentially regulated miRNA marker profile comprises up-regulated expression or activity of at least five of the miRNAs detected by the detectors identified in Tables 2 or 4.
83. The method of embodiment 82 wherein the first differentially regulated miRNA marker profile comprises up-regulated expression or activity of at least ten of the miRNAs detected by the detectors identified in Tables 2 or 4.
84. The method of embodiment 83 wherein the first differentially regulated miRNA marker profile comprises up-regulated expression or activity of at least fifteen of the miRNAs detected by the detectors identified in Tables 2 or 4.
85. The method of embodiment 80 wherein the at least one of the miRNAs is detected by the detector hsa-miR-34b-4373037.
86. The method of embodiment 79 wherein the first differentially regulated miRNA marker profile comprises down-regulated expression or activity of at least one of the miRNAs detected by the detectors identified in Tables 3 or 5.
87. The method of embodiment 86 wherein the first differentially regulated miRNA marker profile comprises down-regulated expression or activity of at least two of the miRNAs detected by the detectors identified in Tables 3 or 5.
88. The method of embodiment 87 wherein the first differentially regulated miRNA marker profile comprises down-regulated expression or activity of at least five of the miRNAs detected by the detectors identified in Tables 3 or 5.
89. The method of embodiment 88 wherein the first differentially regulated miRNA marker profile comprises down-regulated expression or activity of at least ten of the miRNAs detected by the detectors identified in Tables 3 or 5.
90. The method of embodiment 89 wherein the first differentially regulated miRNA marker profile comprises down-regulated expression or activity of at least fifteen of the miRNAs detected by the detectors identified in Tables 3 or 5.
91. The method of embodiment 86 wherein the at least one of the miRNAs is detected by the detector hsa-miR-1-4373161.
92. The method of embodiment 80 wherein the first differentially regulated miRNA marker profile further comprises down-regulated expression or activity of at least one of the miRNAs detected by the detectors identified in Tables 3 or 5.
93. The method of embodiment 92 wherein the down-regulated expression or activity is of at least two of the miRNAs detected by the detectors identified in Tables 3 or 5.
94. The method of embodiment 93 wherein the down-regulated expression or activity is of at least five of the miRNAs detected by the detectors identified in Tables 3 or 5.
95. The method of embodiment 94 wherein the down-regulated expression or activity is of at least ten of the miRNAs detected by the detectors identified in Tables 3 or 5.
96. The method of embodiment 95 wherein the down-regulated expression or activity is of at least fifteen of the miRNAs detected by the detectors identified in Tables 3 or 5.
97. The method of embodiment 92 wherein the at least one of the miRNAs detected by the detectors identified in Tables 3 or 5 is hsa-miR-1-4373161.
98. The method of embodiment 79 wherein the first first differentially regulated miRNA marker profile is a strong profile and the patient prognosis is disease progression.
99. The method of embodiment 98 wherein the strong profile comprises a differential expression of the miRNAs of at least 10-fold that of a healthy individual.
100. The method of embodiment 98 wherein the autoimmune disease is myositis.
101. The method of embodiment 79 wherein the first IFNα-inducible PD marker expression profile is a weak profile and the patient prognosis is disease regression.
102. The method of embodiment 101 wherein the weak profile comprises a differential expression of the miRNAs no more than 2-fold that of a healthy individual.
103. The method of embodiment 98 wherein the strong profile indicates to a physician to change disease treatment.
104. The method of embodiment 79 further comprising:
   obtaining a second differentially regulated miRNA marker profile in a second sample from a patient;
   wherein an increase in number or level of miRNA markers in the second relative to the first profile prognoses disease progression; or
   wherein a decrease in number or level of miRNA markers in the second relative to the first profile prognoses disease regression.
105. A method of monitoring autoimmune or inflammatory disease progression of a patient receiving treatment with a therapeutic agent comprising:
   obtaining a first miRNA profile in a first sample from the patient;
   administering a therapeutic agent;
   obtaining a second miRNA profile in a second sample from the patient; and
   comparing the first and the second miRNA profiles,
      wherein a variance in the first and the second miRNA profiles indicates a level of efficacy of the therapeutic agent.
106. The method of embodiment 105 wherein the first miRNA profile comprises up-regulated expression or activity of at least one of the miRNAs detected by the detectors identified in Tables 2 or 4.
107. The method of embodiment 106 wherein the first miRNA profile comprises up-regulated expression or activity of at least two of the miRNAs detected by the detectors identified in Tables 2 or 4.
108. The method of embodiment 107 wherein the first miRNA profile comprises up-regulated expression or activity of at least five of the miRNAs detected by the detectors identified in Tables 2 or 4.
109. The method of embodiment 108 wherein the first miRNA marker profile comprises up-regulated expression or activity of at least ten of the miRNAs detected by the detectors identified in Tables 2 or 4.
110. The method of embodiment 109 wherein the first miRNA profile comprises up-regulated expression or activity of at least fifteen of the miRNAs detected by the detectors identified in Tables 2 or 4.
111. The method of embodiment 106 wherein the at least one of the miRNAs is detected by the detector hsa-miR-34b-4373037.
112. The method of embodiment 105 wherein the first miRNA profile comprises down-regulated expression or activity of at least one of the miRNAs detected by the detectors identified in Tables 3 or 5.
113. The method of embodiment 112 wherein the first miRNA profile comprises down-regulated expression or activity of at least two of the miRNAs detected by the detectors identified in Tables 3 or 5.
114. The method of embodiment 113 wherein the first miRNA profile comprises down-regulated expression or activity of at least five of the miRNAs detected by the detectors identified in Tables 3 or 5.
115. The method of embodiment 114 wherein the first miRNA profile comprises down-regulated expression or activity of at least ten of the miRNAs detected by the detectors identified in Tables 3 or 5.
116. The method of embodiment 115 wherein the first miRNA profile comprises down-regulated expression or activity of at least fifteen of the miRNAs detected by the detectors identified in Tables 3 or 5.
117. The method of embodiment 112 wherein the at least one of the miRNAs is detected by the detector hsa-miR-1-4373161.
118. The method of embodiment 106 wherein the first miRNA profile further comprises down-regulated expression or activity of at least one of the miRNAs detected by the detectors identified in Tables 3 or 5.
119. The method of embodiment 118 wherein the down-regulated expression or activity is of at least two of the miRNAs detected by the detectors identified in Tables 3 or 5.
120. The method of embodiment 119 wherein the down-regulated expression or activity is of at least five of the miRNAs detected by the detectors identified in Tables 3 or 5.
121. The method of embodiment 120 wherein the down-regulated expression or activity is of at least ten of the miRNAs detected by the detectors identified in Tables 3 or 5.
122. The method of embodiment 121 wherein the down-regulated expression or activity is of at least fifteen of the miRNAs detected by the detectors identified in Tables 3 or 5.
123. The method of embodiment 118 wherein the at least one of the miRNAs detected by the detectors identified in Tables 3 or 5 is hsa-miR-1-4373161.
124. The method of embodiment 105 wherein the autoimmune or inflammatory disease is lupus, idiopathic inflammatory myositis, Sjogren's syndrome, vasculitis, sarcoidosis, or psoriasis.
125. The method of embodiment 124 wherein the autoimmune disease is idiopathic inflammatory myositis.
126. The method of embodiment 105 wherein the therapeutic agent is a small molecule or a biologic agent.
127. The method of embodiment 126 wherein the biologic agent is an antibody.
128. The method of embodiment 127 wherein the antibody is MEDI-545.
129. The method of embodiment 105 wherein the first miRNA profile is obtained prior to administration of the therapeutic agent.
130. The method of embodiment 105 wherein the first miRNA profile is obtained at the time of administration of the therapeutic agent.
131. The method of embodiment 105 wherein the first and the second sample are whole blood, muscle, or serum.
132. The method of embodiment 105 further comprising obtaining a third miRNA profile in a third sample from the patient.
133. The method of embodiment 132 further comprising obtaining a fourth miRNA profile in a fourth sample from the patient.
134. The method of embodiment 133 further comprising obtaining a fifth miRNA profile in a fifth sample from the patient.
135. The method of embodiment 134 further comprising obtaining a sixth miRNA profile in a sixth sample from the patient.
136. The method of embodiment 105 wherein the second sample is obtained at least one week, at least 2 weeks, at least three weeks, at least one month or at least two months following administration of the therapeutic agent.
137. The method of embodiment 132 wherein the third sample is obtained at least 2 days, at least 5 days, at least one week, at least 2 weeks, at least three weeks, at least one month or at least two months following obtaining the second sample.
138. The method of embodiment 133 wherein the fourth sample is obtained at least 2 days, at least 5 days, at least one week, at least 2 weeks, at least three weeks, at least one month or at least two months following obtaining the third sample.
139. The method of embodiment 134 wherein the fifth sample is obtained at least 2 days, at least 5 days, at least one week, at least 2 weeks, at least three weeks, at least one month or at least two months following obtaining the fourth sample.
140. The method of embodiment 105 wherein variance is a decrease in differential miRNA levels of the patient relative to healthy levels.
141. The method of embodiment 105 wherein variance is an increase in differential miRNA levels of the patient relative to healthy levels.
142. The method of embodiment 141 wherein the increase in differential miRNA levels indicates poor disease prognosis or disease will flare.
143. The method of embodiment 141 wherein the increase in differential miRNA levels of the patients indicates a change in treatment of the patient.
144. A method of treating a myositis patient comprising:
   administering an agent that binds to and modulates type I IFN or IFNα activity;
      wherein the patient comprises a type I IFN or IFNα-inducible PD marker expression profile;
      and wherein the agent neutralizes the type I IFN or IFNα-inducible PD marker expression profile of the patient.
145. The method of embodiment 144 further comprising detecting neutralization of the type I IFN or IFNα-inducible PD marker expression profile of the patient.
146. The method of embodiment 144 wherein the type I IFN or IFNα-inducible PD marker expression profile comprises up-regulated expression or activity of genes MX1, LY6E, IFI27, OAS1 IFIT1, IFI6, IFI44L, ISG15, LAMP3, OASL, RSAD2, and IFI44.
147. The method of embodiment 144 wherein the type I IFN or IFNα-inducible PD marker expression profile comprises up-regulated expression or activity of genes LAMP3, SIGLEC1, DNAPTP6, IFIT2, ETV7, RTP4, SERPING1, HERC5, XAF1, MX1, EPSTI1, OAS2, OAS1, OAS3, IFIT3, IFI6, USP18, RSAD2, IFI44, LY6E, ISG15, and IFI27.
148. The method of embodiment 144 wherein the type I IFN or IFNα-inducible PD marker expression profile comprises up-regulated expression or activity of genes EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, and USP18
149. The method of embodiment 144 wherein the type I IFN or IFNα-inducible PD marker expression profile comprises up-regulated expression or activity of genes DNAPTP6, EPSTI1, HERC5, IFI27, IFI44, IFI44L, IFI6, IFIT1, IFIT3, ISG15, LAMP3, LY6E, MX1, OAS1, OAS2, OAS3, PLSCR1, RSAD2, RTP4, SIGLEC1, and USP18.
150. The method of embodiment 144 wherein the type I IFN or IFNα-inducible PD marker expression profile comprises up-regulated expression or activity of genes SAMD9L, IFI6, IFI44, IFIT2, OAS1, IFI27, OAS3, IFI44L, HERC5, IFIT1, EPSTI1, ISG15, SERPING1, OASL, GBP1, and MX1.
151. The method of embodiment 144 wherein the type I IFN or IFNα-inducible PD marker expression profile comprises up-regulated expression or activity of genes IFI6, RSAD2, IFI44, IFI44L, IFI27, MX1, IFIT1, ISG15, LAMP3, OAS3, OAS1, EPSTI1, IFIT3, OAS2, SIGLEC1, and USP18.
152. The method of embodiment 144 wherein the type I IFN or IFNα-inducible PD marker expression profile comprises up-regulated expression or activity of genes IFI6, RSAD2, IFI44, IFI44L, and IFI27.
153. The method of embodiment 144 wherein the agent is a biologic agent.
154. The method of embodiment 153 wherein the agent is an antibody.
155. The method of embodiment 154 wherein the antibody is MEDI-545.
156. The method of embodiment 154 wherein the antibody is specific for one or more type I IFN or IFNα subtype but is not MEDI-545.
157. The method of embodiment 144 wherein the administering the agent alleviates one or more myositis symptoms.
158. The method of embodiment 154 wherein the antibody is administered at a dose between approximately .03 and 30 mg/kg.
159. The method of embodiment 158 wherein the antibody is administered at a dose between 0.3 and 3 mg/kg.
160. The method of embodiment 159 wherein the antibody is administered at a dose between .03 and 1 mg/kg.
161. The method of any one of embodiments 153-155 wherein the agent neutralizes the type I IFN or IFNα-inducible PD marker expression profile of the patient by at least 10%.
162. The method of embodiment 144 wherein the agent neutralizes the type I IFN or IFNα-inducible PD marker expression profile of the patient by at least 20%.
163. The method of embodiment 162 wherein the agent neutralizes the type I IFN or IFNα-inducible PD marker expression profile of the patient by at least 30%.
164. The method of embodiment 163 wherein the agent neutralizes the type I IFN or IFNα-inducible PD marker expression profile of the patient by at least 40%.
165. The method of embodiment 164 wherein the agent neutralizes the type I IFN or IFNα-inducible PD marker expression profile of the patient by at least 50%.
166. The method of embodiment 144 wherein the type I IFN or IFNα-inducible PD marker expression profile comprises up-regulated expression or activity of at least IFNα subtypes 1, 2, 8, and 14.
167. The method of embodiment 144 wherein the type I IFN or IFNα-inducible PD marker expression profile comprises transcripts of PD marker genes.
168. The method of embodiment 144 wherein the type I IFN or IFNα-inducible PD marker expression profile comprises polypeptides expressed from PD marker genes.
169. A method of monitoring or prognosing myositis disease progression of a patient comprising:
   obtaining a first IFNα-inducible PD marker expression profile in a first sample from a patient.
170. The method of embodiment 169 wherein the first IFNα-inducible PD marker expression profile is a strong profile and the patient prognosis is myositis disease progression.
171. The method of embodiment 170 wherein the first IFNα-inducible PD marker expression profile comprises overexpression or activity of genes EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, and USP18.
172. The method of embodiment 169 wherein the first IFNα-inducible PD marker expression profile is a weak profile and the patient prognosis is myositis disease regression.
173. The method of embodiment 169 further comprising:
   obtaining a second IFNα-inducible PD marker expression profile in a second sample from the patient;
   wherein an increase in number or level of type I IFN or IFNα inducible PD markers in the second relative to the first expression profile prognoses myositis disease progression; or
   wherein a decrease in number or level of type I IFN or IFNα inducible PD markers in the second relative to the first expression profile prognoses myositis disease regression.
174. A method of monitoring myositis disease progression of a patient receiving treatment with a therapeutic agent comprising:
   obtaining a first IFNα-inducible PD marker expression profile in a first sample from the patient;
   administering a therapeutic agent;
   obtaining a second IFNα-inducible PD marker expression profile in a second sample from the patient; and
   comparing the first and the second IFNα-inducible PD marker expression profiles,
      wherein a variance in the first and the second IFNα-inducible PD marker expression profiles indicates a level of efficacy of the therapeutic agent.
175. The method of embodiment 174 wherein the first type I IFN or IFNα-inducible PD marker expression profile comprises up-regulated expression or activity of genes profile comprises overexpression or activity of genes EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, and USP18.
176. The method of embodiment 174 wherein the variance is a decrease in up-regulated expression of activity levels of the genes.
177. The method of embodiment 174 wherein the therapeutic agent is a small molecule or a biologic agent.
178. The method of embodiment 177 wherein the therapeutic agent is biologic agent that binds to and modulates IFNα activity
179. The method of embodiment 178 wherein the biologic agent is an antibody.
180. The method of embodiment 179 wherein the antibody is MEDI-545.
181. The method of embodiment 179 wherein the antibody binds to at least one IFNα subtype but is not MEDI-545.
182. The method of embodiment 174 wherein the first IFNα-inducible PD marker expression profile is obtained prior to administration of the therapeutic agent.
183. The method of embodiment 174 wherein the first IFNα-inducible PD marker expression profile is obtained at the time of administration of the therapeutic agent.
184. The method of embodiment 174 wherein the first and the second sample are whole blood, muscle, or serum.
185. The method of embodiment 174 further comprising obtaining a third IFNα-inducible PD marker expression profile in a third sample from the patient.
186. The method of embodiment 185 further comprising obtaining a fourth IFNα-inducible PD marker expression profile in a fourth sample from the patient.
187. The method of embodiment 186 further comprising obtaining a fifth IFNα-inducible PD marker expression profile in a fifth sample from the patient.
188. The method of embodiment 187 further comprising obtaining a sixth IFNα-inducible PD marker expression profile in a sixth sample from the patient.
189. The method of embodiment 174 wherein the second sample is obtained at least one week, at least 2 weeks, at least three weeks, at least one month or at least two months following administration of the therapeutic agent.
190. The method of embodiment 185 wherein the third sample is obtained at least 2 days, at least 5 days, at least one week, at least 2 weeks, at least three weeks, at least one month or at least two months following obtaining the second sample.
191. The method of 186 wherein the fourth sample is obtained at least 2 days, at least 5 days, at least one week, at least 2 weeks, at least three weeks, at least one month or at least two months following obtaining the third sample.
192. The method of embodiment 187 wherein the fifth sample is obtained at least 2 days, at least 5 days, at least one week, at least 2 weeks, at least three weeks, at least one month or at least two months following obtaining the fourth sample.
193. The method of embodiment 174 wherein variance is a decrease in up-regulated expression or activity of the gene.
194. The method of embodiment 193 wherein the decrease is at least 10%, at least 20%, at least 25%, at least 30%, at least 40%, at least 45%, at least 50%, at least 60%, at least 70%, at least 75%, at least 80%, at least 85%, at least 90%, at least 95%, at least 96%, at least 97%, at least 98%, at least 99%, or 100%.
195. The method of embodiment 144 wherein the type I IFN or IFNα-inducible PD marker expression profile comprises up-regulated expression or activity of genes IFI27, RSAD2, IFI44L, IFI44, OAS1, IFIT1, ISG15, OAS3, HERC5, MX1, ESPTI1, IFIT3, and IFI6.
196. The method of embodiment 144 wherein the type I IFN or IFNα-inducible PD marker expression profile comprises up-regulated expression or activity of genes IFI44L, RSAD2, IFI27, and IFI44.
197. The method of embodiment 144 wherein the type I IFN or IFNα-inducible PD marker expression profile comprises up-regulated expression or activity of genes IFI44L and RSAD2.
198. The method of embodiment 169 wherein the first IFNα-inducible PD marker expression profile comprises up-regulated expression or activity of genes IFI27, RSAD2, IFI44L, IFI44, OAS1, IFIT1, ISG15, OAS3, HERC5, MX1, ESPTI1, IFIT3, and IFI6.
199. The method of embodiment 169 wherein the first IFNα-inducible PD marker expression profile comprises up-regulated expression or activity of genes IFI44L, RSAD2, IFI27, and IFI44.
200. The method of embodiment 169 wherein the first IFNα-inducible PD marker expression profile comprises up-regulated expression or activity of genes IFI44L and RSAD2.
201. The method of embodiment 174 wherein the first IFNα-inducible PD marker expression profile comprises up-regulated expression or activity of genes IFI27, RSAD2, IFI44L, IFI44, OAS1, IFIT1, ISG15, OAS3, HERC5, MX1, ESPTI1, IFIT3, and IFI6.
202. The method of embodiment 174 wherein the first IFNα-inducible PD marker expression profile comprises up-regulated expression or activity of genes IFI44L, RSAD2, IFI27, and IFI44.
203. The method of embodiment 174 wherein the first IFNα-inducible PD marker expression profile comprises up-regulated expression or activity of genes IFI44L and RSAD2.
204. The method of embodiment 1 wherein the patient has a strong type I IFN or IFNα-inducible gene signature score.
205. The method of embodiment 1 wherein the patient has a moderate type I IFN or IFNα-inducible gene signature score.
206. The method of embodiment 204 wherein the strong score is greater than or equal to 10.
207. The method of embodiment 205 wherein the moderate score is greater than or equal to 4 but less than 10.
208. The method of embodiment 35 or 74 wherein the myositis is DM or PM.
209. The method of embodiment 144 wherein the myositis patient is a DM or PM patient.
210. The method of embodiment 144 wherein the myositis patient comprises a MITAX score greater than 6.
211. The method of embodiment 144 wherein the type I IFN or IFNα-inducible gene signature score is strong.
212. The method of embodiment 144 wherein the type I IFN or IFNα-inducible gene signature score is moderate.
213. The method of embodiment 211 wherein the score is greater than or equal to 10.
214. The method of embodiment 212 wherein the score is greater than or equal to 4 but less than 10.
215. The method of embodiment 174 wherein the first IFNα-inducible PD marker expression profile comprises a strong type I IFN or IFNα-inducible gene signature score.
216. The method of embodiment 215 wherein the strong score is a score greater than or equal to 10.
217. The method of embodiment 174 wherein the first IFNα-inducible PD marker expression profile comprises a moderate type I IFN or IFNα-inducible gene signature score.
218. The method of embodiment 217 wherein the moderate score is a score greater than or equal to 4 but less than 10.

All publications, patents and patent applications mentioned in this specification are herein incorporated by reference into the specification to the same extent as if each individual publication, patent or patent application was specifically and individually indicated to be incorporated herein by reference.

This application incorporates by reference, for all purposes, U.S. Provisional Application Serial No. 60/873,008 filed December 6, 2006, U.S. Provisional Application Serial No. 60/907,762 filed April 16, 2007, U.S. Provisional Application Serial No. 60/924,219 filed May 3, 2007, U.S. Provisional Application Serial No. 60/924,584 filed May 21, 2007, U.S. Provisional Application Serial No. 60/960,187 filed September 19, 2007, U.S. Provisional Application Serial No. 60/996,176 filed November 5, 2007 and PCT application No. PCT/US2007/024947 filed December 6, 2007. This application also incorporates by reference, for all purposes, U.S. Provisional Application Serial No. 61/129,366 filed June 20, 2008 and PCT application No. PCT/US2008/062646 filed May 5, 2008. This application also incorporates by reference, for all purposes, U.S. Provisional Application Serial No. 60/924,220 filed May 3, 2007, U.S. Provisional Application entitled "Auto-Antibody Markers of Autoimmune Disease" filed November 6, 2007 (serial number 60/996,219), U.S. Provisional Application entitled "Auto-Antibody Markers of Autoimmune Disease" filed December 6, 2007 (serial number 60/996,820), and PCT application No. PCT/US2008/062639 filed May 5, 2008.

Furthermore, this application claims priority to U.S. Provisional Application Serial No. 61/006,963 filed February 8, 2008, herein incorporated by reference for all purposes.

The set of examples that follow are provided for the purpose of illustration only and the invention should in no way be construed as being limited to these examples.

### EXAMPLES

### Example 1a: The majority of the most up-regulated genes in DM patient muscle samples are IFNα/β-inducible

DM muscle samples were examined to determine which genes were most up-regulated in DM patients.

*Total RNA extraction and microarray processing*: Total RNA was extracted from PAXgene muscle biopsies using the Qiagen RNeasy Fibrous Tissue Mini (Hilden, Germany). RNA purity and concentration were determined spectrophotometrically (260/280 > 1.9). RNA quality was assessed on an Agilent 2100 Bioanalyzer using the RNA 6000 Nano LabChip^{®}.

Generation of biotin-labeled amplified cRNA, from 2ug of total RNA, was accomplished with the Affymetrix GeneChip^{®} One-Cycle cDNA Synthesis kit and the Affymetrix GeneChip^{®} IVT Labeling kit. Concentration and purity of the cRNA product were determined spectrophotometrically. Twenty micrograms of each biotin-labeled cRNA was fragmented for hybridization on Affymetrix Human Genome U133 Plus 2.0 GeneChip^{®} arrays. Fragmented cRNA was prepared for hybridization as outlined in the Affymetrix GeneChip^{®} manual. Hybridization was conducted overnight in a model 320 rotating hybridization oven set at 45°C. All GeneChip^{®} wash and staining procedures were performed on an Affymetrix Model 450 Fluidics station. Arrays were scanned on an Affymetrix GeneChip^{®} Scanner 3000. Data capture and initial array quality assessment were performed with the GeneChip Operating Software (GCOS) tool.

*Results:* The majority of the most up-regulated genes in muscle specimens of DM patients are IFNα/β-inducible. See Figure 1a, which provides a list of the most up-regulated genes in the DM patient muscle samples, and fold-up-regulation of those genes in the DM patient muscle samples relative to healthy control muscle samples. See also Figure 1b which provides a scatter plot of the IFNα/β-inducible gene signature score in muscle of DM and PM patients.

### Example 1b: Overexpression of type I IFN-inducible genes in the majority of DM or PM patient blood samples

Prevalence and magnitude of overexpression of type I IFN-inducible genes in the blood of DM and PM patients were evaluated.

*Patients and patient samples:* Two groups of patients were used in this study. The first group included 24 myositis patients (15 with DM and 9 with PM) from a single hospital center prospectively enrolled into a longitudinal exploratory study of the association between whole genome gene expression in peripheral WB samples and clinical features including disease activity. Clinical data from a total of 150 patient-visits and gene expression data from 80 patient-visits were collected over a period of up to 6 years (mean period of participation in this study was 1.9 years). Clinical features of those patients are summarized in Table 6, below.

**Table 6. Clinical features for 24 DM or PM patients.**

| Disease, patient/ age/gender | # of Serial Visit Blood Protiling / Duration of Study (months) | Disease Duration Initial Visit (months) | Treatment Initial Visit | Treatment Duration at Initial Visit (months) | CK level, rauge, (units/liter) | MITAX | Other |
|---|---|---|---|---|---|---|---|
| | | | | | | SCORE | diagnoses |
| | | | | | | Initial (Min,Max) | |
| **Dermatomyositis (N=15)** | | | | | | | |
| BGE79/46/F | 3 (32) | 72 | None | 0 | 260 - 816 | 12(6,12) | Calcinosis |
| BGE99/21/M | 7(21) | 3 | None | 0 | 90 - 353 | 12(1,12) | - |
| BGE143/43/F | 3 (6) | 290 | None | 0 | 46 - 90 | 12(1,12) | - |
| BGE147/57/F | 7(13) | 18 | None | 0 | 36 - 252 | 9(4,9) | - |
| BGE10/38/F | 6 (43) | 108 | Pred/IVIG | 4 | 137 - 4590 | 12(6,12) | - |
| BGE15/62/F | 4 (78) | 36 | Pred/Myco | 12 | 25 - 80 | 2 (0,6) | - |
| BGE17/70/F | 2 (63) | 24 | Pried/ IVIG/Myco | 6 | 19 - 52 | 2(2,2) | - |
| BGE19/61/F | 2 (27) | 12 | Pred/Myco | 24 | 34 - 6554 | 2 (2,6) | Breast cancer |
| BGE46/25/F | 2 (4) | 8 | Pred | 0.1 | 126 - 393 | 12 (6,12) | - |
| BGE80/44/F | 2 (23) | 1 | Pred | 1 | 71 - 87 | 2 (2,6) | - |
| BGE92/27/F | 4 (6) | 17 | Pred | 11 | 1140 - 33877 | 13 (13,13) | ILD, Jo-1 |
| BGE95/53/M | 2 (13) | 25 | MTX | 23 | 260 - 6416 | 2 (2,13) | Diabetes |
| BGE126/58/F | 2 (2) | 2 | Prednisone | 0.2 | 46 - 350 | 12(1,12) | - |
| **BGE140/46/F** | **3(11)** | **42** | **Pred/MTX** | **36** | **1831 - 1925** | **12 (6,12)** | **-** |
| BGE155/46/F | 2 (5) | 15 | Pred | 14 | 860-4107 | 3 (3,13) | ILD, Jo-1 |
| **Polymyositis (N=9)** | | | | | | | |
| BGE106/59/F | 4(13) | 2 | None | 0 | 53 - 4720 | 9(1,9) | - |
| BGE119/47/F | 6 (12) | 5 | None | 0 | 62 - 1256 | 9 (1,9) | ILD/MCTD |
| **BGE3/55/F** | **4(47)** | **26** | **Pred/Myco** | **20** | **498 - 2094** | **6 (6,6)** | **-** |
| BGE11/65/F | 2 (49) | 81 | Aza/IVIG | 48 | 66 - 1156 | 3 (1,3) | ILD; Jo-1 |
| BGE47/72/F | 3 (45) | 11 | Pred/Myco | 9 | 209 - 3027 | 9 (3,9) | - |
| BGE50/40/F | 2 (26) | 2 | Pred/IVIG | 2 | 74 - 663 | 3 (1,3) | - |
| BGE91/55/F | 2 (4) | 6 | MTX | 5 | 123 | 3 (3,3) | - |
| BGE100/57/M | 3 (15) | 12 | Pred/MTX | 4 | 339 - 830 | 3 (3,3) | - |
| **BGE128/84/F** | **3 (7)** | **80** | **Pred** | **76** | **150 - 1437** | **1 (1,9)** | **-** |

Early cross-sectional studies of some of these patients after one or two visits were previously reported (Walsh RJ, Pinkus JL, et al. Type I interferon-inducible gene expression in blood is present and reflects disease activity in dermatomyositis and polymyositis. Arthritis Rheum. 2007; 56(11):3784-92); the current study increased the number of enrolled patients and performed a longitudinal analysis of up to 7 visits per patient. Diagnostic criteria for DM and PM are as previously reported (Walsh RJ, Pinkus JL, et al. Type I interferon-inducible gene expression in blood is present and reflects disease activity in dermatomyositis and polymyositis. Arthritis Rheum. 2007; 56(11):3784-92). WB samples were collected at routine clinical follow-up visits. Disease activity was assessed at each visit using the Myositis Intention to Treat scale (MITAX) as previously described (Walsh RJ, Pinkus JL, et al. Type I interferon-inducible gene expression in blood is present and reflects disease activity in dermatomyositis and polymyositis. Arthritis Rheum. 2007; 56(11):3784-92). Clinical parameters, including the MITAX score (Rider LG, Miller FW. Idiopathic inflammatory muscle disease: clinical aspects. Baillieres Best Pract Res Clin Rheumatol. 2000; 14(1):37-54), were assessed blinded to gene expression data. WB samples were not collected according to any predefined schedule. Patients gave informed consent in order to participate. These studies were approved by institutional review board (IRB) at Brigham and Women's Hospital.

The second group of myositis patients (18) was studied for single blood samples obtained at screening visits for a clinical trial in patients with DM or PM. These blood samples were used for initial evaluation of the prevalence of cytokine-inducible gene signatures across patients seen by multiple investigators at multiple centers, and were collected with informed consent under IRB approved protocols. These subjects were at least 18 years of age and had probable or definite DM or PM according to the Bohan and Peter criteria (Bohan A, Peter JB. Polymyositis and dermatomyositis. N Engl J Med. 1975 Feb 13;292(7):344-7; Bohan A, Peter JB. Polymyositis and dermatomyositis. N Engl J Med. 1975 Feb 20;292(8):403-7) and active disease. WB samples were collected in PAXgene RNA tubes at clinical visits prior to treatment. All subjects had to have at least 2 of the following: strength in manual muscle testing (MMT) ≥ 80/150 but ≤ 125/150 using the MMT-8 muscle group testing; patient global activity assessment by visual analogue scale ≥ 2.0 cm on a 10-cm scale; physician global activity assessment by visual analogue scale ≥ 2.0 cm on a 10-cm scale, CLINHQA disability index ≥ 0.25; and global extramuscular activity assessment ≥ 1.0 on a 10 cm visual analogue scale. Subjects with PM were required to have documentation of a muscle biopsy result consistent with the diagnosis of PM. At screening, subjects could not be receiving > 35 mg prednisone or equivalent per day, hydroxychloroquine > 600 mg/day, mycophenolate mofetil > 3 g/day, methotrexate > 25 mg/week, azathioprine > 3 mg/kg/day, leflunomide > 20 mg/day, or any dose of cyclophosphamide, cyclosporine, or thalidomide.

Normal blood samples were from 36 healthy donors as described (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312; Walsh RJ, Pinkus JL, et al. Type I interferon-inducible gene expression in blood is present and reflects disease activity in dermatomyositis and polymyositis. Arthritis Rheum. 2007; 56(11):3784-92), and were collected with informed consent under IRB approved protocols.

*Total RNA extraction*, *microarray assays, and TaqMan QRT-PCR assays:* RNA isolation from WB used PAXgene tubes or buffy coat samples, and Affymetrix U133 Plus 2.0 microarray assays were carried out as previously described (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312; Walsh RJ, Pinkus JL, et al. Type I interferon-inducible gene expression in blood is present and reflects disease activity in dermatomyositis and polymyositis. Arthritis Rheum. 2007; 56(11):3784-92). RNA was isolated using the PAXgene tube collection method for 24 normal healthy donor samples, while 12 normal healthy donor samples were processed with the buffy coat method. These normal healthy donor samples were used as baselines for the patient samples processed by one of the two approaches in order to eliminate any variability due to sample processing differences.

Fluidigm's Biomark system dynamic array platform (Fluidigm Corp, South San Francisco, CA, USA) was used to measure expression levels of the type 1 IFN-inducible genes, a panel of cytokine-inducible genes, and selected immune response genes in the WB of 15 patients with DM or PM. The general procedures for sample processing and data analysis were as described previously (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi: 10.4061/2009/374312).

*Microarray data analysis:* Microarray data analysis was performed as previously described (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312; Yao Y, Jallal J, et al. Type I IFN as a potential therapeutic target for psoriasis. PLoS ONE 2008; 3(7): e2737. doi:10.1371/journal.pone.0002737), using GC-Content Robust Multichip Analysis (GCRMA), which was implemented in the Bioconductor (http://www.bioconductor.org/) GCRMA package. All two-group comparisons involving the gene signature scores were calculated using a two-sample Wilcoxon-Mann-Whitney test, while paired comparisons were calculated using a Wilcoxon signed-rank test. All p-values reported are unadjusted for multiple testing, although significance is stated for adjusted p-values, using a Bonferroni correction. For identification of patients with a positive type 1 IFN-inducible gene signature score, we calculated significance analysis of microarrays (SAM) with false discovery rate (FDR) adjustment in R (R Development Core Team, University of Auckland, New Zealand).

*Results:* Affymetrix human genome U133 plus 2.0 GeneChip^{®} was employed to profile the peripheral WB of 42 patients with DM or PM (22 DM and 20 PM patients; from first and second patient groups). 1,072 and 703 transcripts were observed to be upregulated and downregulated (fold change >2, q<0.05), respectively, in the blood of patients with DM or PM compared with healthy controls. Of the 1,072 upregulated transcripts, 136 were type 1 IFN-inducible as defined by ex vivo stimulation of WB with type I IFN family members as described (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008;Volume 2009, Article ID 374312, doi:10.4061/2009/374312). A heatmap visualizing the expression of the 136 transcripts in WB of 42 patients with DM or PM, as compared to 24 healthy controls, is shown in Figure 384a. The type 1 IFN-inducible gene signature scores calculated using the dynamic 25 most overexpressed type 1 IFN-inducible genes (dynamic list potentially different between patients) in the blood of individual normal controls and patients with DM or PM are shown in Figure 384b. The patients evaluated in the study exhibited different degrees of overexpression of type 1 IFN-inducible genes in the blood, and this included a distinct population of patients with DM or PM that had weak type 1 IFN-inducible gene signatures (score < 4). Normal donors and these weak signature score patients are represented by the first and second horizontal bars, respectively, in Figure 384b. Based on classification criteria (in which a type 1 IFN-inducible gene signature was weak if it was assigned a score of less than 4, moderate if it was assigned a score that was greater than or equal to 4 but less than 10, and high if it was assigned a score greater than or equal to 10) 17% of patients with DM or PM had weak or no overexpression of type 1 IFN-inducible gene signature in the blood. A total of 45% and 38% of the patients had moderate or high type 1 IFN-inducible gene signatures, respectively. Thus, the overwhelming majority of patients with DM or PM showed moderate to high overexpression of type 1 IFN-inducible gene signature in the blood.

### Example 1c: Selection of a 13-gene panel to evaluate type 1 IFN signaling pathway activity in patients with DM or PM

Based on the results of Example 1b, a small panel of type 1 IFN-inducible genes was selected that could be used to evaluate the activation of the type 1 IFN signaling pathway in the blood of DM and PM patients.

*Ranking genes most overexpressed in the peripheral WB of patients with DM and PM:* To identify the most overexpressed probe sets across the 42 DM and PM patients used in the study described in Example 1b, fold-change values were calculated between each patient individually and the median of 24 healthy donors for each probe set on the Affymetrix U133 Plus 2.0 array. For each patient, the fold-change values were then ranked in descending order. The median ranking was calculated for each probe set and the top 200 overall probe sets were selected. The 807 probe sets that have been previously identified as type 1 IFN-inducible (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008;Volume 2009, Article ID 374312, doi:10.4061/2009/374312) were intersected with these 200 probe sets, and those probe sets in common were summated to identify type 1 IFN-inducible genes that were overexpressed in peripheral WB of patients with DM or PM.

*Results:* Composite scores of relative expression of multiple genes can provide a robust measurement of pathway activity, as described previously (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312, Yao, Y, Jallal J, et al. Neutralization of IFN-α/β-Inducible Genes and Downstream Effect in a Phase I Trial of an Anti-IFN-α Monoclonal Antibody in SLE. Accepted by Arthritis Rheum.). The average fold ranks for the 100 most overexpressed genes in peripheral WB of the 42 patients with DM or PM are listed in Table 7, shown below.

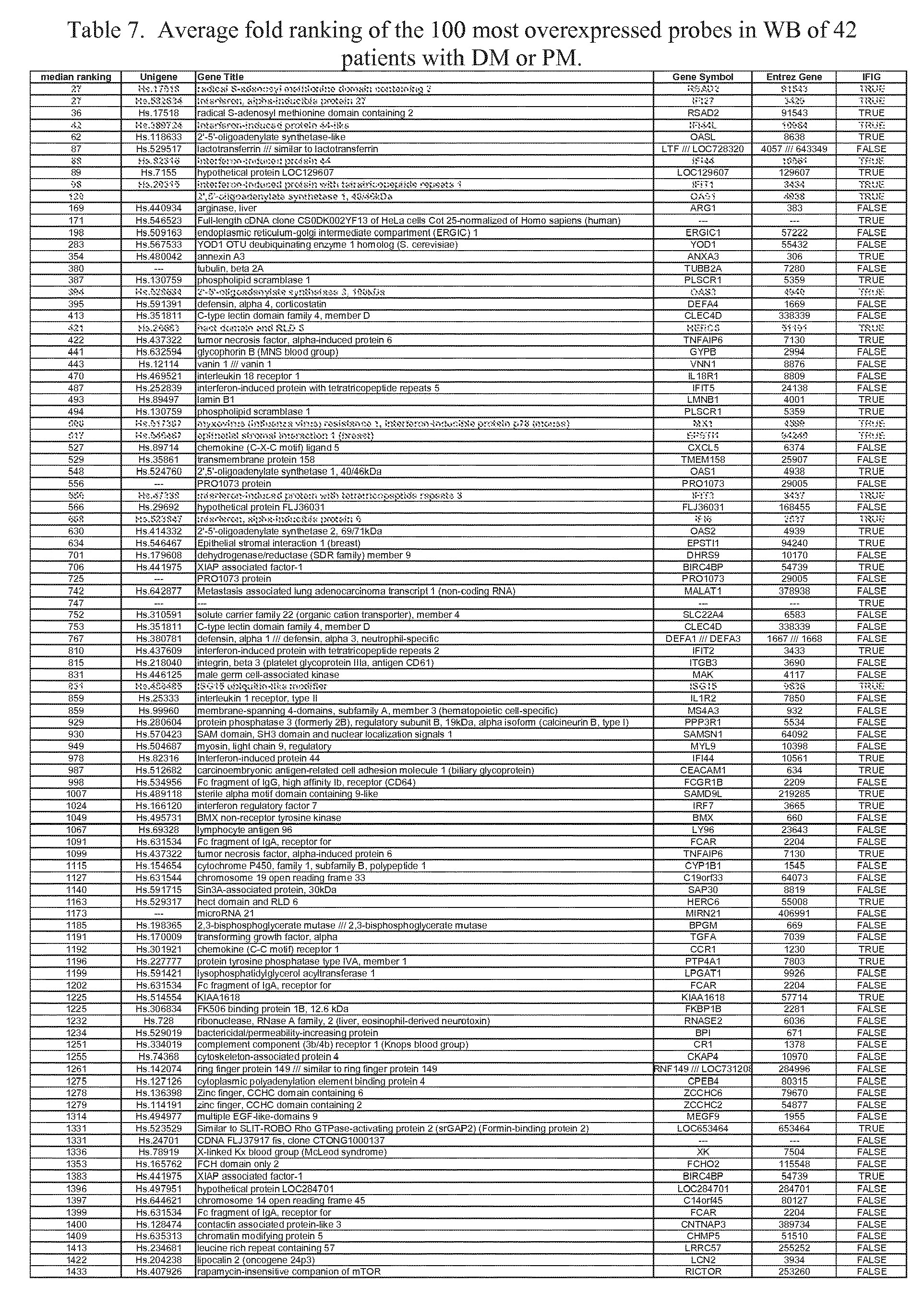

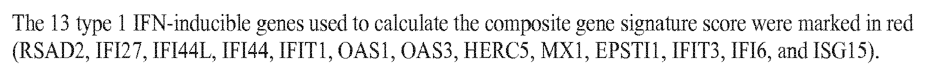

The thirteen overexpressed type 1 IFN-inducible genes were selected from this group in order to construct a type 1 IFN-inducible gene signature score reflecting type 1 IFN signaling pathway activation in the blood of DM and PM patients. The 13 selected genes were: the 6 most overexpressed type 1 IFN-inducible genes in the blood of DM and PM patients (IFI27, RSAD2, IFI44L, IFI44, OAS1, IFIT1), plus ISG15, one of the most overexpressed type 1 IFN-inducible genes in DM muscle specimens (Greenberg SA, Tawil R, et al. Interferon-alpha/beta-mediated innate immune mechanisms in dermatomyositis. Ann Neurol 2005; 57(5):664-78), and 6 well characterized type 1 IFN-inducible genes (OAS3, HERC5, MX1, ESPTI1, IFIT3, and IFI6). Gene signature scores with this 13-gene panel (a subset of the 21-gene panel for SLE) correlated very well (r=0.98) with scores for the 21-gene panel of type 1 IFN-inducible genes used to characterize type 1 IFN signaling pathway activation in SLE (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312), and also with the alternative gene signature score approach using the dynamic top 25 most overexpressed type 1 IFN-inducible genes (r=0.82) in individual patients (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312, Yao, Y, Jallal J, et al. Neutralization of IFN-α/β-Inducible Genes and Downstream Effect in a Phase I Trial of an Anti-IFN-α Monoclonal Antibody in SLE. Accepted by Arthritis Rheum.).

Besides among the most overexpressed type 1 IFN-inducible genes in the blood of patients with DM or PM, stronger overexpression of mRNAs of IFI27, RSAD2, IFI44LL and IFI44 was generally observed in DM and PM patients with high disease activity. For the 12 patients with high disease activity (MITAX > 6; see Table 6, above), at their peak MITAX score during the study, average fold changes of mRNAs of IFI27, RSAD2, IFI44LL and IFI44, compared to the average of 36 normal controls, were 89-, 53-, 90- and 19-fold, respectively. QRT-PCR was also used to validate the microarray results for these 4 genes. WB samples from 15 patients (9 DM, 6 PM) were selected for the purpose. QRT-PCR measurements for these 4 individual genes correlated highly with microarray measurements (IFI27 r=0.96; IFI44 r=0.95; IFI44L r=0.98; and RSAD2 r=0.98).

### Example 1d: Correlation of the 13 gene IFNα/β-inducible gene signature score with myositis disease activity

Longitudinal studies conducted for 24 DM and PM patients identified an association of the 13 gene type I IFN-inducible signature and disease activity (MITAX).

*Linear mixed model for longitudinal analysis*: A repeated measures analysis was conducted for only those patients with at least 3 follow-up visits (total N=53 follow-up visits for 12 patients). Ten different regression models were fit where MITAX score (low and high disease activity as defined previously) was regressed on the following, all modeled as random effects: 13-gene composite score, RSAD2, IFI27, IFI44, and IFI44L. Treatment status at the initial visit was used as a binary covariate (*i.e*., any treatment/no treatment) and modeled as a fixed effect in each model in order to control for the influence of medication on gene expression changes.

*Results*: Of the 24 DM and PM patients evaluated, 3 of the patients (specifically BGE128, BGE140 and BGE3) had low IFN-inducible gene signature scores always < 1 and never differed from scores of normal controls throughout their course. This was the case even when their disease activities as measured by MITAX scores changed significantly.

The remaining 21 patients in the longitudinal study were stratified into a two-group comparison with low (MITAX score ≤ 6; 9 patients) and high (MITAX score > 6; 12 patients) disease activity. Both of these categories demonstrated an association with type 1 IFN-inducible gene expression (13-gene score p=0.002, IFI27 p=0.002, and RSAD2 p=0.003 - all significant after adjustment; see Table 8 and Figure 385a). In a comparison between a panel of 36 normal healthy donors and patients with high disease activity, the difference using both the 13-gene composite score and 4 individual genes was found to be highly significant (Table 8; Figure 385a). These results suggested that both the type 1 IFN-inducible 13-gene signature score and the expression of the 4 individual type 1 IFN-inducible genes correlated strongly with disease activity of DM and PM patients.

**Table 8: P-values for DM and PM patients at visit one for 4 type 1 IFN-inducible genes, the 13-gene composite score, and 5 cytokine-inducible gene signature scores.**

| | **Normal vs MITAX≤6** | **Normal vs MITAX>6** | **MITAX≤6 vs MITAX>6** |
|---|---|---|---|
| **13-gene** | 0.032 | <0.0001 | 0.002 |
| **RSAD2** | 0.10 | <0.0001 | 0.003 |
| **IFI44L** | 0.024 | <0.0001 | 0.006 |
| **IFI44** | 0.021 | <0.0001 | 0.01 |
| **IFI27** | 0.005 | <0.0001 | 0.002 |
| **GM-CSF** | <0.0001 | <0.0001 | 0.46 |
| **TNF-a** | <0.0001 | <0.0001 | 0.86 |
| **IL-1B** | 0.001 | 0.0001 | 0.97 |
| **IL-10** | 0.0002 | <0.0001 | 0.70 |
| **IL-13** | <0.0001 | <0.0001 | 0.97 |

| | | | |
|---|---|---|---|
| Two-group comparisons are between normal donors, patients with high disease activity (MITAX score > 6 at visit 1), and patients with low disease activity (MITAX score ≤ 6 at visit 1). All p-values are unadjusted. | | | |

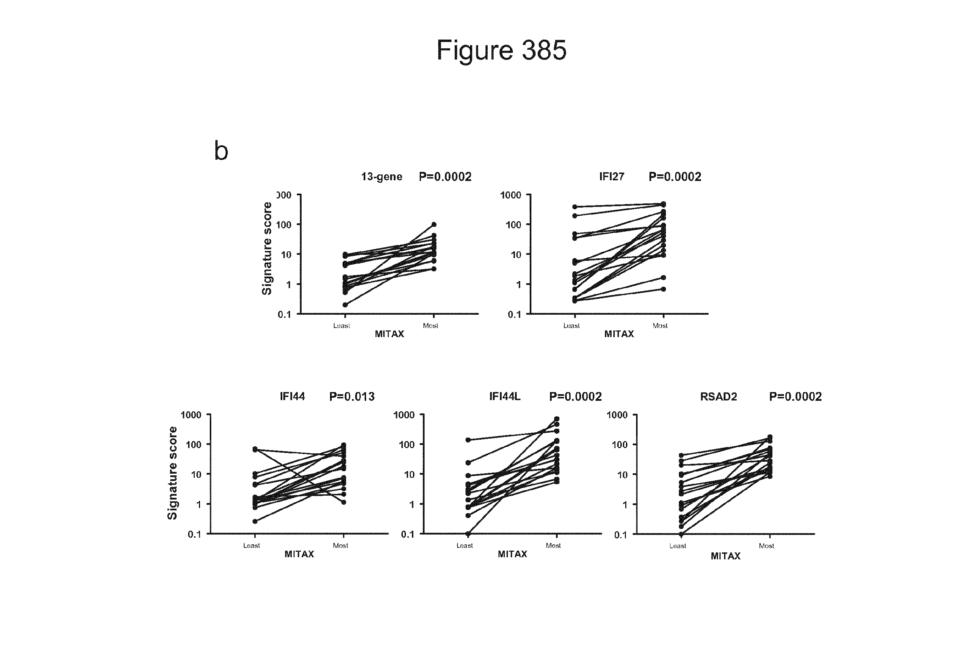

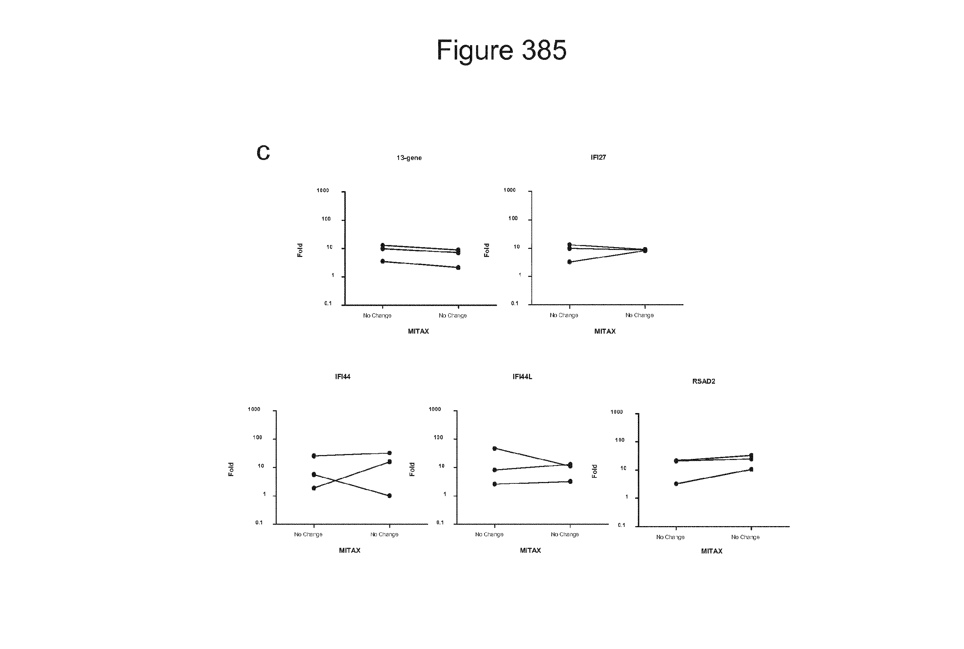

These biomarkers also showed high correlation with clinical disease activity within individual patients during longitudinal follow up. Patients (N=18) with any variation in disease activity among their visits showed strong correlation between MITAX score changes and changes in both the 13-gene composite score (p=0.0002) and individual gene expression levels (IFI27 p=0.0002, RSAD2 p=0.0002, and IFI44L p=0.0002 - all significant after adjustment; Figure 385b). IFI44 was not significant after adjustment. Furthermore, both the 13-gene score and expression of mRNAs of RSAD2 and IFI44L increased in all 18 patients from low to high MITAX scores. The expression of IFI44 mRNA decreased in 2 patients from low to high MITAX scores. These results suggest that the composite score is likely more robustly correlated with disease activity than individual genes. Patients (N=3) with no variation in disease activity showed no real changes in expression levels of these genes (Figure 385c).

Furthermore, major clinical disease activity changes were associated with major changes in the 13-gene composite signature score. Among the 80 patient-visits were 50 pairs of consecutive visits. Among these 50 were 14 major changes in disease activity (change in MITAX score of > 3; 10 improvements, 4 worsening events). All 14 changes were associated with major changes in both the 13-gene composite score and the expression of 4 individual type 1 IFN-inducible genes in the concordant direction (13-gene composite score, IFI44L and IFI27 provide the best correlation; Table 9).

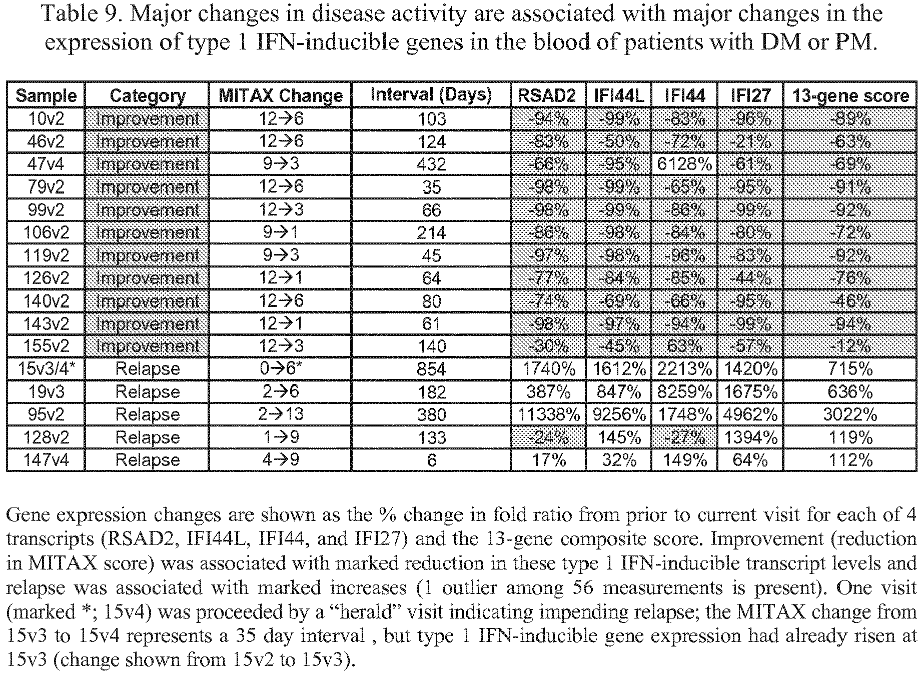

Seven patients had WB samples collected in at least 4 visits. All patients showed strong correlation between changes in disease activity and 13-gene composite scores (Figure 386a). For 6 of these patients, this was evident using the MITAX score as the disease activity measure; with one refractory patient (BGE92), a limitation of the MITAX score (it reaches a maximum at 13 and cannot increase with further increases in disease activity) limited its use, but the serum CK (increasing from 3,369 to 32,877 IU/L) reflected the changes in disease activity and correlated strongly with the changes in the 13-gene composite score (Figure 386b).

A general summary of association between clinical disease activity and type 1 IFN-inducible gene expression changes across all patients was performed using a linear mixed model. An advantage of linear mixed model analysis is the ability to improve estimates by pooling information across patients. The 13-gene composite score was found to be the most significantly correlated with disease clinical activity (p=0.0007), with IFI44L (p=0.001) and RSAD2 (p=0.002) the next well-correlated (still significant after adjustment) for the 12 patients with at least 3 follow-up measurements, after controlling for treatment status.

Furthermore, three patients appeared to have "herald" visits, or visits in which MITAX score disease activity was low but the 13-gene composite score was significantly increased relative to the prior visit (Figure 387). Shortly after these "herald" visits (7-35 days), each of the three patients relapsed into disease.

### Example 2a: IFNα/β-inducible gene signature scores predict myositis disease activity

DM and PM patients, from a study separate from Example 1b-d patients, were likewise monitored to determine whether disease progression and/or regression could be predicted by changes in IFNα/β-inducible gene signature.

*Total RNA extraction and microarray processing*: Total RNA was extracted from PAXgene blood using the PAXgene Blood RNA kit. RNA purity and concentration were determined spectrophotometrically (260/280 > 1.9). RNA quality was assessed on an Agilent 2100 Bioanalyzer using the RNA 6000 Nano LabChip^{®}.

Generation of biotin-labeled amplified cRNA, from 2ug of total RNA, was accomplished with the Affymetrix GeneChip^{®} One-Cycle cDNA Synthesis kit and the Affymetrix GeneChip^{®} IVT Labeling kit. Concentration and purity of the cRNA product were determined spectrophotometrically. Twenty micrograms of each biotin-labeled cRNA was fragmented for hybridization on Affymetrix Human Genome U133 Plus 2.0 GeneChip^{®} arrays. Fragmented cRNA was prepared for hybridization as outlined in the Affymetrix GeneChip^{®} manual. Hybridization was conducted overnight in a model 320 rotating hybridization oven set at 45°C. All GeneChip^{®} wash and staining procedures were performed on an Affymetrix Model 450 Fluidics station. Arrays were scanned on an Affymetrix GeneChip^{®} Scanner 3000. Data capture and initial array quality assessment were performed with the GeneChip Operating Software (GCOS) tool.

*Serum CK levels:* Serum CK levels were obtained from Steven Greenberg (Brigham and Women's Hospital, Inc.).

*Calculation of IFN gene signature using static list of 21 genes:* 21 genes were identified by their prevalence and magnitude of overexpression in Myositis patients as compared to those of normal controls. It includes a standard list of the same 21 genes (i.e. 21 probes that map to 21 unique genes) for each Myositis patient. These genes include DNA polymerase-transactivated protein 6 - DNAPTP6; epithelial stromal interaction 1 (breast) - EPSTI1; hect domain and RLD 5 - HERC5; interferon, alpha-inducible protein 27 - IFI27; Interferon-induced protein 44 - IFI44; interferon-induced protein 44-like - IFI44L; interferon, alpha-inducible protein 6 - IFI6; interferon-induced protein with tetratricopeptide repeats 1 - IFIT1; interferon-induced protein with tetratricopeptide repeats 3 - IFIT3; ISG15 ubiquitin-like modifier; lysosomal-associated membrane protein 3 - LAMP3; lymphocyte antigen 6 complex, locus E - LY6E; myxovirus (influenza virus) resistance 1, interferon-inducible protein p78 - MX1; 2',5'-oligoadenylate synthetase 1, 40/46kDa - OAS1; 2'-5'-oligoadenylate synthetase 2, 69/71kDa - OAS2; 2'-5'-oligoadenylate synthetase 3, 100kDa - OAS3; Phospholipid scramblase 1 - PLSCR1; radical S-adenosyl methionine domain containing 2 - RSAD2; receptor (chemosensory) transporter protein 4 - RTP4; sialic acid binding Ig-like lectin 1, sialoadhesin - SIGLEC1; ubiquitin specific peptidase 18 - USP18. The fold change is calculated between Myositis patients at day 0 (predose) and the average of 24 normal healthy controls. The median of these 21 fold change values is then computed for each Myositis patient and this value is used as the IFN-α/β-inducible gene signature score.

*TNFα*, *IL1β*, *IL4*, *IL10*, *and IL13 gene signatures*: Calculation of TNFα, IL1β, IL4, IL10, and IL13 gene signatures were calculated similarly to way that the IFN-α/β-inducible gene signature. However, because the initial number of cytokine-inducible genes that were identified differs for each cytokine, the percentage rather than absolute number of genes was used. For example, using the IFN-α/β-inducible gene signature as the standard, the top 25 of the 807 genes were selected for each patient (dynamic gene list). This represents approximately 3.5% of the IFN-α/β-inducible genes determined. This percentage of genes was maintained for each of the other cytokines to calculate the gene signature for each patient. The IL1β genes from which the IL1β signature was derived included: zinc finger CCCH-type containing 12A - ZC3H12A; ureidopropionase, beta - UPB1; SAM domain, SH3 domain and nuclear localisation signals, 1 - SAMSN1; nuclear factor of kappa light polypeptide gene enhancer in B-cells inhibitor, zeta - NFKBIZ; pentraxin-related gene, rapidly induced by IL-1 beta - PTX3; peptidase inhibitor 3, skin-derived (SKALP) - PI3; IBR domain containing 2 - IBRDC2; ATG7 autophagy related 7 homolog (S. cerevisiae) - ATG7; adenosine A2a receptor - ADORA2A; nuclear factor of kappa light polypeptide gene enhancer in B-cells inhibitor, zeta - NFKBIZ; Bcl2 modifying factor - BMF; peptidase inhibitor 3, skin-derived - (SKALP) ; pleiomorphic adenoma gene-like 2 - PLAGL2; mitogen-activated protein kinase kinase kinase 8 - MAP3K8; hypothetical protein LOC285286 - LOC285286; IBR domain containing 2 - IBRDC2; chemokine (C-C motif) ligand 4 - CCL4; EH-domain containing 1 EHD1; nuclear factor of kappa light polypeptide gene enhancer in B-cells 2 (p49/p100) - NFKB2; solute carrier family 39 (zinc transporter), member 8 - SLC39A8; reticulon 2 - RTN2; nibrin - NBN; orosomucoid 1 /// orosomucoid 2 - ORM1 /// ORM2; solute carrier family 11 (proton-coupled divalent metal ion transporters), member 2 - SLC11A2; RNA binding motif, single stranded interacting protein 1 - RBMS1; thrombospondin 1 - THBS1; nuclear factor of kappa light polypeptide gene enhancer in B-cells 2 (p49/p100) - NFKB2; hypothetical protein LOC253842 - LOC253842; interleukin-1 receptor-associated kinase 3 - IRAK3; immediate early response 5 - IER5; EH-domain containing 1 - EHD1; CDNA clone IMAGE:30408657; nuclear factor of kappa light polypeptide gene enhancer in B-cells inhibitor, alpha - NFKBIA; EH-domain containing 1 - EHD1; hypoxia-inducible protein 2 - HIG2; prostaglandin E synthase-PTGES; pleiomorphic adenoma gene-like 2 - PLAGL2; IBR domain containing 2 - IBRDC2; CDNA FLJ13601 fis, clone PLACE1010069; thrombospondin 1 - THBS1; antizyme inhibitor 1 - AZIN1; ATPase, H+ transporting, lysosomal 42kDa, V1 subunit C1 - ATP6V1C1; stannin - SNN; immediate early response 3 - IER3; ATG7 autophagy related 7 homolog (S. cerevisiae) - ATG7; v-rel reticuloendotheliosis viral oncogene homolog B, nuclear factor of kappa light polypeptide gene enhancer in B-cells 3 (avian)-RELB; G protein-coupled receptor 157 - GPR157; oligodendrocyte transcription factor 1 /// similar to oligodendrocyte transcription factor 1 /// similar to oligodendrocyte transcription factor 1 - OLIG1;/// LOC728598 ///; LOC732056 - 6355; Thrombospondin 1 - THBS1

The TNFα genes from which the TNFα signature was derived included: C-type lectin domain family 4, member D - CLEC4D; DENN/MADD domain containing 4A - DENND4A; phosphoinositide-3-kinase adaptor protein 1 - PIK3AP1; interferon induced with helicase C domain 1 - IFIH1; SAM domain, SH3 domain and nuclear localization signals 1 - SAMSN1; guanine nucleotide binding protein (G protein), gamma 2 - GNG2; Hypothetical protein LOC149478 - LOC149478; CDNA FLJ32866 fis, clone TESTI2003718; hypothetical protein LOC389072 - LOC389072; LIM domain kinase 2 - LIMK2; RNA binding motif, single stranded interacting protein 1 - RBMS1; hypothetical protein LOC731424 - LOC731424; cytoskeleton-associated protein 4 - CKAP4; acidic (leucine-rich) nuclear phosphoprotein 32 family, member A - ANP32A; solute carrier family 7 (cationic amino acid transporter, y+ system), member 5 - SLC7A5; influenza virus NS1A binding protein - IVNS1ABP; nuclear factor of kappa light polypeptide gene enhancer in B-cells inhibitor, alpha - NFKBIA; chloride intracellular channel 4 - CLIC4; fascin homolog 1, actin-bundling protein (Strongylocentrotus purpuratus) - FSCN1; interferon gamma receptor 2 (interferon gamma transducer 1) - IFNGR2; myristoylated alanine-rich protein kinase C substrate - MARCKS; early growth response 1 - EGR1; antizyme inhibitor 1 - AZIN1; LIM domain kinase 2 - LIMK2; guanylate binding protein 1, interferon-inducible, 67kDa /// guanylate binding protein 1, interferon-inducible, 67kDa - GBP1; tumor necrosis factor, alpha-induced protein 2 - TNFAIP2; intercellular adhesion molecule 1 (CD54), human rhinovirus receptor - ICAM1; tumor necrosis factor, alpha-induced protein 3 - TNFAIP3; signal-regulatory protein alpha - SIRPA; nibrin-NBN; EPM2A (laforin) interacting protein 1 - EPM2AIP1; pleiomorphic adenoma gene-like 2 - PLAGL2; NADH dehydrogenase (ubiquinone) flavoprotein 2, 24kDa - NDUFV2; ninjurin 1 - NINJ1; solute carrier family 11 (proton-coupled divalent metal ion transporters), member 2 - SLC11A2; presenilin 1 (Alzheimer disease 3) - PSEN1; pleckstrin - PLEK; fasciculation and elongation protein zeta 1 (zygin I) - FEZ1; peptidase inhibitor 3, skin-derived (SKALP); nuclear factor of kappa light polypeptide gene enhancer in B-cells inhibitor, epsilon - NFKBIE; purinergic receptor P2X, ligand-gated ion channel, 4 - P2RX4; chemokine (C-C motif) ligand 4 - CCL4; GTP cyclohydrolase 1 (dopa-responsive dystonia) - GCH1; CD83 molecule - CD83; human immunodeficiency virus type I enhancer binding protein 1 - HIVEP1; Nedd4 binding protein 1 - N4BP1; nuclear factor (erythroid-derived 2)-like 3 - NFE2L3; transcription factor-like 5 (basic helix-loop-helix) - TCFL5; prostaglandin E receptor 4 (subtype EP4) - PTGER4; polo-like kinase 3 (Drosophila) - PLK3; adenosine A2a receptor - ADORA2A; mitogen-activated protein kinase kinase kinase 8 - MAP3K8; orosomucoid 1 - ORM1; orosomucoid 1 /// orosomucoid 2 - ORM1 /// ORM2; chemokine (C-C motif) ligand 3 /// chemokine (C-C motif) ligand 3-like 1 /// chemokine; CCL3 /// CCL3L1 /// CCL3L3 /// LOC728830 /// LOC730422; CD40 molecule, TNF receptor superfamily member 5-CD40; v-rel reticuloendotheliosis viral oncogene homolog B, nuclear factor of kappa light polypeptide gene enhancer in B-cells 3 (avian) - RELB; butyrophilin, subfamily 2, member A2 - BTN2A2; metal-regulatory transcription factor 1 - MTF1; activin A receptor, type IIA -ACVR2A; plasminogen activator, urokinase - PLAU; TNF receptor-associated factor 1 - TRAF1; tumor necrosis factor, alpha-induced protein 6 - TNFAIP6; influenza virus NS1A binding protein - IVNS1ABP; platelet-activating factor receptor-PTAFR; immunoglobulin superfamily, member 6 - IGSF6; hypothetical protein FLJ23556 - FLJ23556; transcription factor EC - TFEC; potassium inwardly-rectifying channel, subfamily J, member 2 - KCNJ2; purinergic receptor P2X, ligand-gated ion channel, 7 P2RX7 - 6811; egf-like module containing, mucin-like, hormone receptor-like 1 - EMR1; tumor necrosis factor (TNF superfamily, member 2) - TNF; TNFAIP3 interacting protein 1 - TNIP1; LIM and senescent cell antigen-like domains 1 - LIMS1; acyl-CoA synthetase long-chain family member 1 - ACSL1; nuclear factor of kappa light polypeptide gene enhancer in B-cells 2 (p49/p100) - NFKB2; presenilin 1 (Alzheimer disease 3) - PSEN1; CASP8 and FADD-like apoptosis regulato - CFLAR; EH-domain containing 1 - EHD1; nuclear factor of kappa light polypeptide gene enhancer in B-cells 1 (p105) - NFKB1; solute carrier family 39 (zinc transporter), member 8 - SLC39A8; CASP8 and FADD-like apoptosis regulator /// CASP8 and FADD-like apoptosis regulator - CFLAR; receptor-interacting serine-threonine kinase 2 - RIPK2; nuclear factor of kappa light polypeptide gene enhancer in B-cells 2 (p49/p100) - NFKB2; serpin peptidase inhibitor, clade B (ovalbumin), member 9 - SERPINB9; complement component 3a receptor 1 - C3AR1; CASP8 and FADD-like apoptosis regulator-CFLAR; villin-like - VILL; G protein-coupled receptor 35 - GPR35; prostaglandin E synthase - PTGES; baculoviral IAP repeat-containing 3 - BIRC3; CASP8 and FADD-like apoptosis regulator - CFLAR; LIM domain kinase 2 - LIMK2; leukocyte-associated immunoglobulin-like receptor 1 - LAIR1; fascin homolog 1, actin-bundling protein (Strongylocentrotus purpuratus) - FSCN1; kynurenine 3-monooxygenase (kynurenine 3-hydroxylase) - KMO; phosphodiesterase 4B, cAMP-specific (phosphodiesterase E4 dunce homolog, Drosophila) - PDE4B; Fc fragment of IgA, receptor for - FCAR; CASP8 and FADD-like apoptosis regulator - CFLAR; chemokine (C-C motif) receptor-like 2 /// similar to chemokine (C-C motif) receptor-like 2 - CCRL2 /// LOC727811; platelet-activating factor receptor /// platelet-activating factor receptor - PTAFR; plasminogen activator, urokinase /// plasminogen activator, urokinase - PLAU; CASP8 and FADD-like apoptosis regulator - CFLAR; antizyme inhibitor 1 - AZIN1; RAB6 interacting protein 1 - RAB6IP1; interleukin 1 receptor antagonist - IL1RN; transcription factor 7-like 2 (T-cell specific, HMG-box) - TCF7L2; SEC24 related gene family, member A (S. cerevisiae) - SEC24A; Myristoylated alanine-rich protein kinase C substrate - MARCKS; IBR domain containing 3 - IBRDC3; membrane-associated ring finger (C3HC4); G0/G1switch 2 - G0S2; CDNA FLJ13601 fis, clone PLACE1010069; CDC42 effector protein (Rho GTPase binding) 2 - CDC42EP2; CASP8 and FADD-like apoptosis regulator - CFLAR; intercellular adhesion molecule 1 (CD54), human rhinovirus receptor - ICAM1; Dmx-like 2 - DMXL2; glycerol kinase - GK; NLR family, pyrin domain containing 3 - NLRP3; transcription factor 7-like 2 (T-cell specific, HMG-box) - TCF7L2; CD44 molecule (Indian blood group) - CD44; interleukin 1 receptor antagonist - IL1RN; superoxide dismutase 2, mitochondrial - SOD2; CD58 molecule - CD58; nibrin - NBN; kynureninase (L-kynurenine hydrolase) - KYNU; LIM domain kinase 2 - LIMK2; bromodomain adjacent to zinc finger domain, 1A - BAZ1A; stannin - SNN; damage-regulated autophagy modulator - DRAM; pleckstrin homology domain containing, family F (with FYVE domain) member 2 - PLEKHF2; SLAM family member 7 - SLAMF7; solute carrier family 15, member 3 - SLC15A3; C-type lectin domain family 4, member E - CLEC4E; solute carrier family 39 (zinc transporter), member 8 - SLC39A8; proline-serine-threonine phosphatase interacting protein 2 - PSTPIP2; interleukin-1 receptor-associated kinase 3 - IRAK3; solute carrier family 2 (facilitated glucose transporter), member 6 - SLC2A6; SAM domain, SH3 domain and nuclear localization signals 1 - SAMSN1; Jun dimerization protein p21SNFT - SNFT; ureidopropionase, beta - UPB1; apolipoprotein L, 3 - APOL3; FLJ12886; free fatty acid receptor 2 - FFAR2; free fatty acid receptor 3 /// G protein-coupled receptor 42 /// similar to Free fatty acid receptor 3 (G-protein coupled receptor 41) - FFAR3; UDP-Gal:betaGlcNAc beta 1,4- galactosyltransferase, polypeptide 5 - B4GALT5; adducin 3 (gamma) /// chloride ; EH-domain containing 1 - EHD1; pleckstrin homology domain containing, family F (with FYVE domain) member 2 - PLEKHF2; SLAM family member 7 - SLAMF7; C-type lectin domain family 4, member E - CLEC4E; chromosome 15 open reading frame 48 - C15orf48; G protein-coupled receptor 84 - GPR84; CD274 molecule - CD274 ureidopropionase, beta - UPB1; solute carrier family 35, member B - SLC35B2; GRAM domain containing 1A - GRAMD1A; tumor protein p53 inducible nuclear protein 2 - TP53INP2; tubulin tyrosine ligase - TTL; guanine nucleotide binding protein (G protein), gamma 2 - GNG2; major facilitator superfamily domain containing 2 - MFSD2; Homo sapiens, clone IMAGE:5547644; Cdc42 GTPase-activating protein - CDGAP; TRAF-interacting protein with a forkhead-associated domain - TIFA; dehydrogenase/reductase (SDR family) member 13 - DHRS13; Splicing factor 3a, subunit 2, 66kDa - SF3A2; ATPase, H+ transporting, lysosomal 42kDa, V1 subunit C1 - ATP6V1C1; Bcl2 modifying factor BMF; metal-regulatory transcription factor 1 - MTF1; Early growth response 1 - EGR1; EPM2A (laforin) interacting protein 1 - EPM2AIP1; hypothetical protein MGC7036 - MGC7036; IBR domain containing 2 - IBRDC2; oligodendrocyte transcription factor 1 /// similar to oligodendrocyte transcription factor 1 /// similar to oligodendrocyte transcription factor 1 - OLIG1; ureidopropionase, beta - UPB1; Mitogen-activated protein kinase 10 - MAPK10; Baculoviral IAP repeat-containing 3 - BIRC3; Full length insert cDNA clone YX74D05; Formin-like 3 - FMNL3; interleukin 4 induced 1 - IL4I1; guanylate binding protein 1, interferon-inducible, 67kDa - GBP1; interleukin-1 receptor-associated kinase 2 - IRAK2; zinc finger CCCH-type containing 12C - ZC3H12C; Homo sapiens, clone IMAGE:; Transcription factor 7-like 2 (T-cell specific, HMG-box) - TCF7L2; SLAM family member 7 - SLAMF7; TNF receptor-associated factor 1 - TRAF1; CDNA clone IMAGE:6254031; Mitogen-activated protein kinase kinase kinase 8 - MAP3K8; chromosome 8 open reading frame 38 - C8orf38; IBR domain containing 2 - IBRDC2; CDNA clone IMAGE:5270500; Hypothetical gene supported by BC008048 - LOC440934; IBR domain containing 2 - IBRDC2; dedicator of cytokinesis 4 - DOCK4; Nedd4 binding protein 1 - N4BP1; peptidase inhibitor 3, skin-derived (SKALP) - PI3

The IL4 genes from which the IL4 signature was derived included: the genes listed and annotated on figures 11-104

The IL10 genes from which the IL10 signature was derived included: the genes listed and annotated on figures 105-158.

The IL13 gene from which the IL13 signature was derived included: the genes listed and annotated on figures 159-234.

*Results*: In agreement with the results described in Example 1d, the IFNα/β-inducible gene signature score of DM and PM patients in this set of patients was able to predict disease stability, improvement, and relapse. Figure 2a shows that the IFNα/β-inducible gene signature of DM and PM patients is higher in those with active disease relative to those that are improving. Figure 2b provides the IFNα/β-inducible gene signature of individual DM patients in paired samples, one obtained during active disease and one obtained during disease improvement. The IFNα/β-inducible gene signature of DM patients decreased as the DM patients exhibited disease improvement. Figure 3 provides the IFNα/β-inducible gene signature in whole blood of three other DM patients that showed clinical improvement over the course of two visits with a physician. For each of the three patients ((a), (b), and (c)) the IFNα/β-inducible gene signature deceased as the patient clinically improved. This correlation of cytokine gene signature score with clinical improvement was not observed for other cytokine gene signatures (TNFα, IL1β, IL4, IL10, or IL13). Figure 4 provides changes in the IFNα/β-inducible gene signature of a DM patient. The IFNα/β-inducible gene signature increases as the patient's condition worsens clinically and decreases as the patient's condition improves clinically. Figure 5 furthers show correlation of IFNα/β-inducible gene signature and serum CK level in a PM patient. Figures 6 and 7 show correlation of clinical disease, serum CK activity, and IFNα/β-inducible gene signature in a DM patient. Figure 6 provides the tight correlation between the IFNα/β-inducible gene signature and serum CK activity. Figure 7 further correlates the IFNα/β-inducible gene signature and clinical disease course of the patient.

### Example 2b: TNF-α, IL-1β, GM-CSF, IL-10, and IL-13 signaling pathways are activated in DM and PM patients, but not correlated with disease activity

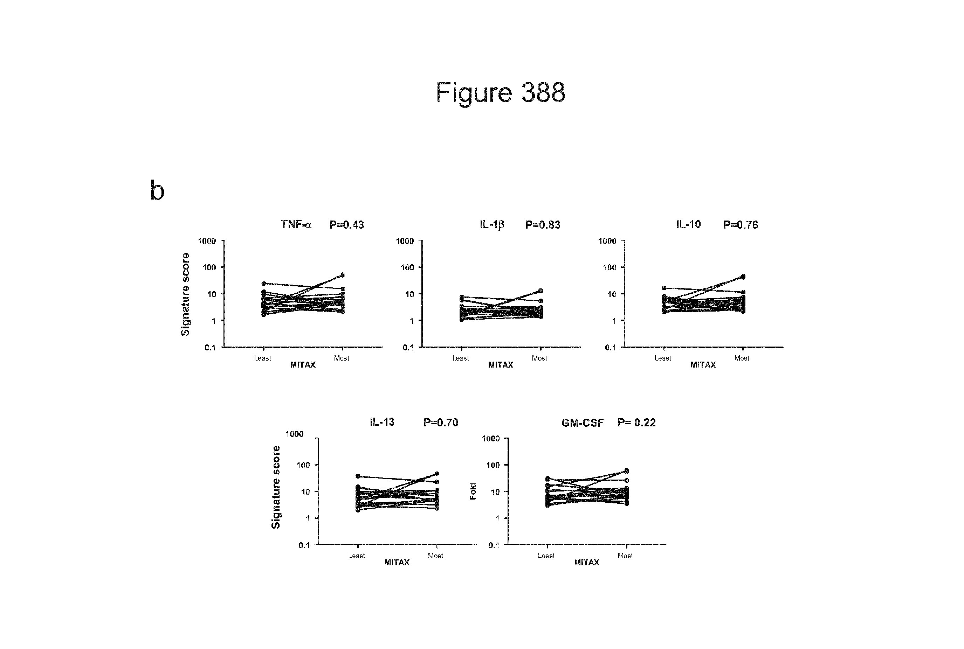

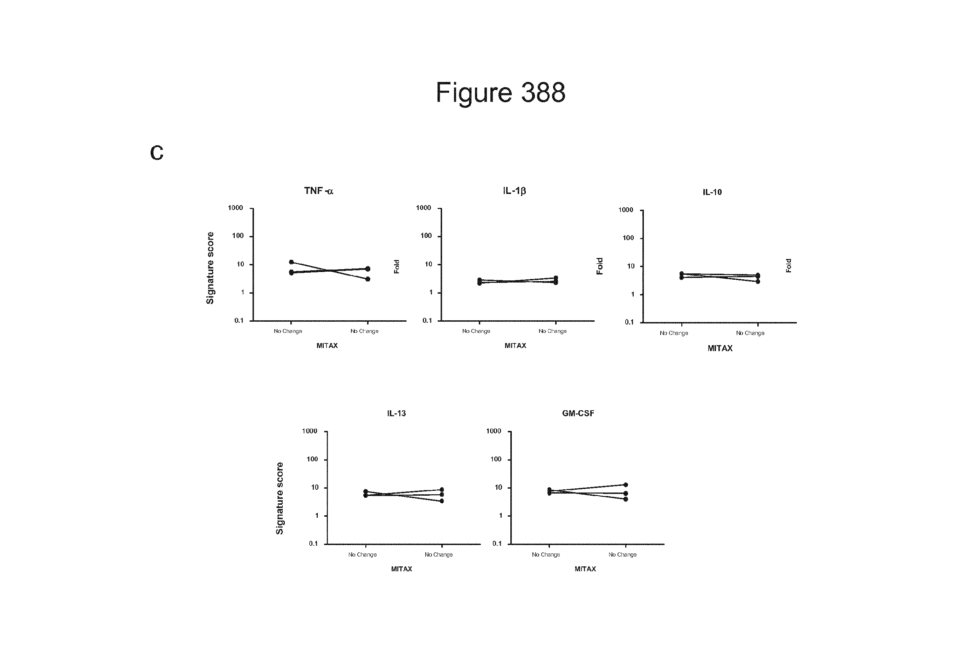

Similar to as described in Example 1d, the whole genome array approach was again used to evaluate the potential effects of cytokines, TNF-α, IL-1β, IL-10, IL-13 and GM-CSF, that might play a role in DM and PM pathogenesis. Figure 388a shows the composite cytokine-inducible gene signature scores for TNF-α, IL-1β, IL-10, IL-13 and GM-CSF, respectively, in WB of 36 healthy normal controls, and in DM and PM patients with low and high disease activity (patients discussed in Examples 1b - 1d). The cytokine-inducible genes signature scores of type 1 IFN, GM-CSF, IL-10, IL-13, IL-1β, and TNF-α were calculated as described (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008;Volume 2009, Article ID 374312, doi:10.4061/2009/374312; Yao, Y, Jallal J, et al. Neutralization of IFN-α/β-Inducible Genes and Downstream Effect in a Phase I Trial of an Anti-IFN-α Monoclonal Antibody in SLE. Accepted by Arthritis Rheum.). The DM and PM patients were classified into type I IFN-inducible gene signature weak, moderate, and high scores as described (Yao, Y, Jallal J, et al. Development of Potential Pharmacodynamic and Diagnostic Markers for Anti-IFN-α Monoclonal Antibody Trials in Systemic Lupus Erythematosus. Human Genomics and Proteomics. 2008; Volume 2009, Article ID 374312, doi:10.4061/2009/374312). Between the controls and the patients with either low or high disease activity, all differences between TNF-α, IL-1β, IL-10, IL-13 and GM-CSF inducible gene signature scores in WB were statistically significant, after adjustment for multiple comparisons (Table 8). This suggested that all of these cytokine signaling pathways were activated in the blood of these patients. TaqMan QRT-PCR also confirmed the overexpression of mRNAs of these cytokines in the muscle biopsies of PM, and to a lesser degree, DM patients (not shown). However, none of these cytokine-inducible gene signature scores showed any statistically significant difference between patients with low and high disease activity (Table 8). Furthermore, for 18 patients with changes in disease activity during the study, none of these cytokine-inducible gene signature scores changed in the concordant direction (Figure 388b). For the 3 patients for whom the level of disease activity never changed throughout their treatment, no significant changes in these cytokine-inducible gene signature scores were observed (Figure 388c).

Figure 389 shows the cytokine-inducible gene signature scores of all 24 patients stratified by high disease and low disease activity. Suppression of type 1 IFN-inducible genes was universal for patients who showed improvement in disease activity while it was not the case for the TNF-α, IL-1β, IL-10, IL-13 and GM-CSF inducible gene signatures.

### Example 3: IFNα/β-inducible gene signature is overexpressed in IBM patient muscle and whole blood samples

It was also determined that the IFNα/β-inducible gene signature is overexpressed in IBM patient muscle and whole blood samples.

*Total RNA extraction and microarray processing*: Total RNA was extracted from PAXgene blood and muscle biopsies using the PAXgene Blood RNA kit and the Qiagen RNeasy Fibrous Tissue Mini (Hilden, Germany), respectively. RNA purity and concentration were determined spectrophotometrically (260/280 > 1.9). RNA quality was assessed on an Agilent 2100 Bioanalyzer using the RNA 6000 Nano LabChip^{®}.

Generation of biotin-labeled amplified cRNA, from 2ug of total RNA, was accomplished with the Affymetrix GeneChip^{®} One-Cycle cDNA Synthesis kit and the Affymetrix GeneChip^{®} IVT Labeling kit. Concentration and purity of the cRNA product were determined spectrophotometrically. Twenty micrograms of each biotin-labeled cRNA was fragmented for hybridization on Affymetrix Human Genome U133 Plus 2.0 GeneChip^{®} arrays. Fragmented cRNA was prepared for hybridization as outlined in the Affymetrix GeneChip^{®} manual. Hybridization was conducted overnight in a model 320 rotating hybridization oven set at 45°C. All GeneChip^{®} wash and staining procedures were performed on an Affymetrix Model 450 Fluidics station. Arrays were scanned on an Affymetrix GeneChip^{®} Scanner 3000. Data capture and initial array quality assessment were performed with the GeneChip Operating Software (GCOS) tool.

*Calculation of gene signature using static list of 21 genes:* The 21 genes within this list were identified by their prevalence and magnitude of overexpression in Myositis patients as compared to those of normal controls. It includes a standard list of the same 21 genes (i.e. 21 probes that map to 21 unique genes) for each Myositis patient. These 21 genes were DNAPTP6; EPSTI1; HERC5; IFI27; IFI44; IFI44L; IFI6; IFIT1; IFIT3; ISG15; LAMP3; LY6E; MX1; OAS1; OAS2; OAS3; PLSCR1; RSAD2; RTP4; SIGLEC1; and USP18. The fold change is calculated between Myositis patients at day 0 (predose) and the average of 24 normal healthy controls. The median of these 21 fold change values is then computed for each Myositis patient and this value is used as the IFN-α/β-inducible gene signature score.

*Calculation of gene signature using the dynamic list of 25 genes:* For each patient, a dynamic list of the top 25 genes (determined by fold change value) is selected from a set of 807 probes previously found to be IFN-α/β-inducible and neutralized by MEDI-545. See Figures 235-381 These 25 genes are chosen by their fold change magnitude and all represent a unique known gene (for genes that are represented by multiple probes, the one that showed the highest fold change was used for the purpose). The fold change is calculated between Myositis patients at day 0 (predose) and the average of 24 normal healthy controls. The median of these 25 fold change values is then computed for each Myositis patient and this value is used as the IFN-α/β-inducible gene signature score.

*Results*: Table 1 shows that the IFNα/β-inducible gene signature is overexpressed in IBM patient muscle whether calculated using a static list of 21 genes or a dynamic list of 25 genes. Figure 8 provides a scatter plot which illustrates the overexpression of the IFNα/β-inducible gene signature in muscle tissue of the individual IBM patients shown in Table 1.

**Table 1: Overexpression of IFN-α/β-inducible gene signature in IBM patient muscle specimens**

| **Sample** | **ID** | **IFNab.med.fc (25 genes)** | **IFNab.med.fc (21 genes)** |
|---|---|---|---|
| IBM | MA45_GEIM18 | 29.49 | 4.48 |
| IBM | MA45_GEIM127 | 16.56 | 3.37 |
| IBM vs PM | MA45_GEIM323 | 26.09 | 7.07 |
| IBM | MA45_GEIM335 | 15.24 | 3.57 |
| IBM | MA45_GEIM354 | 27.11 | 5.49 |
| IBM-HIV | MA45_GEIM358 | 6.74 | 3.13 |
| IBM | MA45_GEIM363 | 58.73 | 7.14 |
| IBM | MA45_GEIM366 | 8.14 | 2.86 |
| IBM | MA45_GEIM372 | 53.50 | 11.16 |
| IBM | MA45_GEIM373 | 21.65 | 4.85 |
| IBM | MA45_GEIM374 | 33.71 | 5.47 |
| IBM | MA45_GEIM377 | 48.42 | 6.20 |
| IBM | MA45_GEIM380 | 25.23 | 4.24 |
| IBM | MA45_GEIM397 | 10.00 | 3.01 |

Whole blood of IBM patients was also examined to determine whether overexpression of the IFNα/β-inducible gene signature could be detected. Using a dynamic list of 25 genes to detect the IFNα/β-inducible gene signature in the samples, overexpression of the IFNα/β-inducible gene signature could be detected in IBM whole blood. See Figure 9a for the IFNα/β-inducible gene signature scores of 9 IBM patient whole blood samples and Figure 9b for a scatter plot illustrating the IFNα/β-inducible gene signature score of the individual IBM patients.

### Example 4: The IFNα/β-inducible gene signature is more elevated in muscle of Jo1⁻ than Jo1⁺ DM patients

Overexpression of the IFNα/β-inducible gene signature is greater in muscle of Jo1⁻ than Jo1⁺ DM patients.

*Total RNA extraction and microarray processing*: Total RNA was extracted from PAXgene muscle biopsies using the the Qiagen RNeasy Fibrous Tissue Mini (Hilden, Germany). RNA purity and concentration were determined spectrophotometrically (260/280 > 1.9). RNA quality was assessed on an Agilent 2100 Bioanalyzer using the RNA 6000 Nano LabChip^{®}.

Generation of biotin-labeled amplified cRNA, from 2ug of total RNA, was accomplished with the Affymetrix GeneChip^{®} One-Cycle cDNA Synthesis kit and the Affymetrix GeneChip^{®} IVT Labeling kit. Concentration and purity of the cRNA product were determined spectrophotometrically. Twenty micrograms of each biotin-labeled cRNA was fragmented for hybridization on Affymetrix Human Genome U133 Plus 2.0 GeneChip^{®} arrays. Fragmented cRNA was prepared for hybridization as outlined in the Affymetrix GeneChip^{®} manual. Hybridization was conducted overnight in a model 320 rotating hybridization oven set at 45°C. All GeneChip^{®} wash and staining procedures were performed on an Affymetrix Model 450 Fluidics station. Arrays were scanned on an Affymetrix GeneChip^{®} Scanner 3000. Data capture and initial array quality assessment were performed with the GeneChip Operating Software (GCOS) tool.

*Calculation of gene signature using static list of 19 genes*: The 19 genes within this list were identified by their prevalence and magnitude of overexpression in Myositis patients as compared to those of normal controls. The same 19 genes (i.e. 19 probes that map to 19 unique genes) were used for each myositis patient. The 19 genes included EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, and USP18. The fold change is calculated between myositis patients at day 0 (predose) and the average of 24 normal healthy controls. The median of these 19 fold change values is then computed for each

*Results*: Figure 10a provides the median-fold overexpression of the each of the 19 genes included in the IFNα/β-inducible gene signature and the overall median-fold change in IFNα/β-inducible gene signature for each of 10 IBM patient muscle samples. Figure 10b provides a scatter plot which illustrates the overexpression of the IFNα/β-inducible gene signature in muscle tissue of each individual IBM patient shown in Figure 10a. While the IFNα/β-inducible gene signature was over-expressed in muscle samples of both the Jo1⁻ and Jo1⁺ patients, overexpression was greater in muscle samples of Jo1⁻ patients.

### Example 5: miRNAs are differentially regulated in myositis patient muscle tissue relative to healthy donor muscle tissue

miRNA levels in muscle tissue were measured in samples obtained from 8 healthy donors, 11 DM patients, and 10 IBM patients.

*Sample processing*: Muscle tissue samples were processed with the *mir*Vana miRNA Isolation Kit (Ambion, Inc., Austin, TX) following the manufacturers suggested protocol. Concentration and purity (260/280 nm) of each sample was determined spectrophotometrically. Sample quality was assessed by Agilent 2100 Bioanalyzer (Agilent Technologies, Inc., Santa Clara, CA) analysis.

*Multiplex RT for TaqMan Array Human MicroRNA Panel (Applied Biosystems, Foster City, CA)*: Processed RNA samples were normalized to a concentration of 25 ng/µL. 4 µL of each normalized RNA sample was transferred to individual wells of an eight-tube microtube strip (4 µL x 8) that had been placed on ice. 5 µL ice cold RT master mix was added to well containing the 4 µL of normalized RNA. Each 5 µL RT master mix contained 0.2 µL 100 mM dNTPs, 2.0 µL MultiScribe Reverse Transcriptase (50 U/mL), 1.0 µL 10X Reverse Transcription Buffer, 0.125 µL RNase Inhibitor (20 U/µL), and 1.675 µL Nuclease-free water that had been prepared on ice. A 1 µL volume of a Multiplex RT human primer pool (one of each of primer pools #1-8) was added to a corresponding microtube (microtubes 1-8)), resulting in a total of eight independent RT reactions per RNA sample. The microtubes were capped, mixed gently and centrifuged briefly. All reaction samples were then incubated on ice for at least 5 minutes prior to conducting the cDNA synthesis protocol.

The cDNA synthesis procedure was conducted in a Bio-Rad (Hercules, CA) Tetrad thermal cycler using the following profile: 16°C for 30 minutes, 42°C for 30 minutes, 85°C for 5 minutes, 4°C-hold. After completion of the cDNA synthesis reaction, all sample volumes (10 µL) were transferred to individual 1.5 mL microcentrifuge tubes and diluted 62.5-fold by adding 615 µL nuclease-free water. All samples were kept on ice until further processing.

*TaqMan Low-Density Array*: In preparation for analysis on the Human MicroRNA Panel TaqMan Low-Density Array (TLDA) (Applied Biosystems, Foster City, CA), 50 µL diluted RT reaction and 50 µL TaqMan Universal PCR Master Mix (2X) (Applied Biosystems, Foster City, CA) were combined in individual 1.5 mL microcentrifuge tubes. The sample tubes were capped, mixed gently and centrifuged briefly. Standard procedures for loading the array were followed and the array was run on a 7900HT Fast Real-Time PCR System (Applied Biosystems, Foster City, CA). Data analysis of the resulting Ct values was conducted with SDSv2.2.2 software (Applied Biosystems, Foster City, CA).

*Results*: miRNAs were differentially regulated in muscle of both DM and IBM patients. See Tables 2-5. Unique detector Ids, miRNA ids, and sequences are provided in Figure sheets

**Table 2: Differentially up-regulated miRNAs in DM patient muscle specimens**

| **Detector** | **p value** | **fold change** |
|---|---|---|
| hsa-miR-21-4373090 | 0.042 | 2.141 |
| hsa-miR-34b-4373037 | 0.046 | 34.091 |
| hsa-miR-382-4373019 | 0.024 | 2.07 |

**Table 3: Differentially down-regulated miRNAs in DM patient muscle specimens**

| **Detector** | **p value** | **fold change** |
|---|---|---|
| hsa-miR-1-4373161 | 0.022 | 0.413 |
| hsa-miR-100-4373160 | 0.023 | 0.47 |
| hsa-miR-101-4373159 | 0 | 0.381 |
| hsa-miR-128b-4373170 | 0.012 | 0.287 |
| hsa-miR-133a-4373142 | 0 | 0.225 |
| hsa-miR-133b-4373172 | 0.006 | 0.471 |
| hsa-miR-181 c-4373115 | 0.019 | 0.407 |
| hsa-miR-186-4373112 | 0.012 | 0.468 |
| hsa-miR-193b-4373185 | 0.004 | 0.294 |
| hsa-miR-22-4373079 | 0.007 | 0.445 |
| hsa-miR-23a-4373074 | 0.008 | 0.494 |
| hsa-miR-23b-4373073 | 0.016 | 0.374 |
| hsa-miR-26b-4373069 | 0.038 | 0.491 |
| hsa-miR-27a-4373287 | 0.008 | 0.384 |
| hsa-miR-27b-4373068 | 0.013 | 0.253 |
| hsa-miR-296-4373066 | 0.002 | 0.327 |
| hsa-miR-29a-4373065 | 0.002 | 0.435 |
| hsa-miR-29c-4373289 | 0 | 0.399 |
| hsa-miR-30a-3p-4373062 | 0.029 | 0.402 |
| hsa-miR-30a-5p-4373061 | 0.01 | 0.398 |
| hsa-miR-30b-4373290 | 0.005 | 0.407 |
| hsa-miR-30c-4373060 | 0.006 | 0.344 |
| hsa-miR-30d-4373059 | 0.004 | 0.395 |
| hsa-miR-30e-3p-4373057 | 0.011 | 0.331 |
| hsa-miR-30e-5p-4373058 | 0.032 | 0.36 |
| hsa-miR-324-5p-4373052 | 0.002 | 0.446 |
| hsa-miR-328-4373049 | 0.032 | 0.436 |
| hsa-miR-331-4373046 | 0.025 | 0.471 |
| hsa-miR-340-4373041 | 0 | 0.445 |
| hsa-miR-361-4373035 | 0.001 | 0.371 |
| hsa-miR-365-4373194 | 0 | 0.304 |
| hsa-miR-378-4373024 | 0.004 | 0.227 |
| hsa-miR-422a-4373200 | 0.004 | 0.306 |
| hsa-miR-423-4373015 | 0.031 | 0.416 |
| hsa-miR-489-4373214 | 0.015 | 0.307 |
| hsa-miR-491-4373216 | 0 | 0.384 |
| hsa-miR-504-4373229 | 0.031 | 0.293 |
| hsa-miR-518e-4373265 | 0.033 | 0.236 |
| hsa-miR-594-4380958 | 0.047 | 0.442 |
| hsa-miR-99a-4373008 | 0.016 | 0.445 |

**Table 4: Differentially up-regulated miRNAs in IBM patient muscle specimens**

| **Detector** | **p value** | **fold change** |
|---|---|---|
| hsa-miR-127-4373147 | 0.001343696 | 2.383908732 |
| hsa-miR-132-4373143 | 0.016749924 | 2.309908878 |
| hsa-miR-146a-4373132 | 4.77E-05 | 6.948265587 |
| hsa-miR-155-4373124 | 0.018655052 | 12.97376751 |
| hsa-miR-21-4373090 | 0.000174951 | 2.970268559 |
| hsa-miR-214-4373085 | 0.012834402 | 2.089563254 |
| hsa-miR-221-4373077 | 0.000213444 | 4.590059735 |
| hsa-miR-222-4373076 | 5.61E-05 | 2.156256092 |
| hsa-miR-299-5p-4373188 | 0.002824631 | 3.814890525 |
| hsa-miR-31-4373190 | 0.011811928 | 4.216344588 |
| hsa-miR-323-4373054 | 0.005094215 | 4.026315243 |
| hsa-miR-34a-4373278 | 0.026043587 | 3.074758212 |
| hsa-miR-34b-4373037 | 0.006396276 | 47.2379796 |
| hsa-miR-376a-4378104 | 0.027686874 | 2.592096707 |
| hsa-miR-382-4373019 | 6.05E-05 | 3.002877696 |
| hsa-miR-411-4381013 | 0.002347682 | 2.735004685 |
| hsa-miR-432-4373280 | 0.009111595 | 4.007329367 |
| hsa-miR-433-4373205 | 0.001574922 | 2.996897264 |
| hsa-miR-511-4373236 | 0.000664051 | 4.444594072 |

**Table 5: Differentially down-regulated miRNAs in IBM patient muscle specimens**

| **Detector** | **p value** | **fold change** |
|---|---|---|
| hsa-let-7a-4373169 | 0.000261495 | 0.461940063 |
| hsa-let-7d-4373166 | 0.000519994 | 0.449301892 |
| hsa-let-7f-4373164 | 0.004942999 | 0.275220876 |
| hsa-let-7g-4373163 | 0.003108841 | 0.479827561 |
| hsa-miR-1-4373161 | 0.0089026 | 0.151102573 |
| hsa-miR-101-4373159 | 4.99E-06 | 0.242569835 |
| hsa-miR-126-4373269 | 0.042276687 | 0.45132497 |
| hsa-miR-128b-4373170 | 0.014270087 | 0.160708669 |
| hsa-miR-133a-4373142 | 5.99E-05 | 0.200538744 |
| hsa-miR-133b-4373172 | 2.25E-08 | 0.238646208 |
| hsa-miR-181 c-4373115 | 0.019419918 | 0.420494392 |
| hsa-miR-186-4373112 | 0.016570765 | 0.488720432 |
| hsa-miR-190-4373110 | 0.011508084 | 0.366253183 |
| hsa-miR-193b-4373185 | 0.005790023 | 0.19249679 |
| hsa-miR-196b-4373103 | 0.024105127 | 0.375328485 |
| hsa-miR-197-4373102 | 0.022613224 | 0.479233993 |
| hsa-miR-19a-4373099 | 0.042678905 | 0.45412976 |
| hsa-miR-20a-4373286 | 0.009776077 | 0.4466972 |
| hsa-miR-22-4373079 | 0.004224752 | 0.263087054 |
| hsa-miR-23b-4373073 | 0.0146826 | 0.359600816 |
| hsa-miR-26a-4373070 | 0.005930571 | 0.39550998 |
| hsa-miR-26b-4373069 | 0.033096249 | 0.443882937 |
| hsa-miR-27b-4373068 | 0.015743098 | 0.297783418 |
| hsa-miR-296-4373066 | 0.001194699 | 0.298589928 |
| hsa-miR-29a-4373065 | 0.001349097 | 0.336664818 |
| hsa-miR-29c-4373289 | 2.52E-09 | 0.238818173 |
| hsa-miR-30a-3p-4373062 | 0.017730482 | 0.229143827 |
| hsa-miR-30a-5p-4373061 | 0.006268296 | 0.257975023 |
| hsa-miR-30b-4373290 | 0.004246722 | 0.274619234 |
| hsa-miR-30c-4373060 | 0.005198547 | 0.281971024 |
| hsa-miR-30d-4373059 | 0.002285223 | 0.241803139 |
| hsa-miR-30e-3p-4373057 | 0.010367212 | 0.2350075 |
| hsa-miR-30e-5p-4373058 | 0.020271038 | 0.244720048 |
| hsa-miR-32-4373056 | 0.019282796 | 0.385941654 |
| hsa-miR-328-4373049 | 0.022423378 | 0.370315868 |
| hsa-miR-331-4373046 | 0.016552922 | 0.372139854 |
| hsa-miR-340-4373041 | 5.05E-05 | 0.441742765 |
| hsa-miR-345-4373039 | 0.04317298 | 0.422795116 |
| hsa-miR-361-4373035 | 0.002496089 | 0.377110989 |
| hsa-miR-365-4373194 | 0.000215304 | 0.21743854 |
| hsa-miR-374-4373028 | 0.042460027 | 0.4973205 |
| hsa-miR-378-4373024 | 0.003719421 | 0.233224009 |
| hsa-miR-422a-4373200 | 0.003828686 | 0.171239493 |
| hsa-miR-423-4373015 | 0.022144073 | 0.35633136 |
| hsa-miR-486-4378096 | 0.01445536 | 0.345595073 |
| hsa-miR-491-4373216 | 6.63E-06 | 0.34787378 |
| hsa-miR-504-4373229 | 0.027141237 | 0.237911164 |
| hsa-miR-518e-4373265 | 0.030022387 | 0.147803021 |
| hsa-miR-594-4380958 | 0.022205513 | 0.250995551 |
| hsa-miR-616-4380992 | 0.043443863 | 0.235726158 |
| hsa-miR-92-4373013 | 0.041324118 | 0.347364092 |
| hsa-miR-95-4373011 | 0.00098202 | 0.187541196 |
| hsa-miR-98-4373009 | 0.004874227 | 0.322319097 |
| hsa-miR-99a-4373008 | 0.026616112 | 0.499299362 |

Owing to this differential regulation, miRNAs may be useful in the diagnosis, prognosis, treatment, and/or stratification of myositis patients.

## Claims

1. A method of monitoring myositis disease progression of a patient receiving treatment with a therapeutic agent comprising:
(i) obtaining a first IFNα-inducible PD marker expression profile in a first sample from the patient;
(ii) obtaining a second IFNα-inducible PD marker expression profile in a second sample from the patient after a therapeutic agent has been administered; and
(iii) comparing the first and the second IFNα-inducible PD marker expression profiles,
wherein the IFNα-inducible PD marker expression profile is based upon up-regulation of the expression of the following genes EPSTI1, HERC5, IFI27, IFI44, IFI44L, IFI6, IFIT1, IFIT3, ISG15, LAMP3, LY6E, MX1, OAS1, OAS2, OAS3, RSAD2, RTP4, SIGLEC1, and USP18; and
wherein a variance in the first and the second IFNα-inducible PD marker expression profiles indicates a level of efficacy of the therapeutic agent, wherein the variance is a decrease in up-regulated expression of the gene.

2. The method of claim 1, wherein the therapeutic agent is an antibody that binds to and modulates IFNα activity.

3. The method of claim 1, wherein the first IFNα-inducible PD marker expression profile is obtained prior to administration of the therapeutic agent.

4. The method of claim 1, wherein the first IFNα-inducible PD marker expression profile is obtained at the time of administration of the therapeutic agent.

5. The method of claim 1, wherein the first and the second sample are whole blood, muscle, or serum.

6. The method of claim 1, wherein the IFNα-inducible PD marker expression profile comprises a moderate type I IFN or IFNα-inducible gene signature score.

7. The method of claim 1, wherein the moderate score is a score greater than or equal to 4 but less than 10.
